# EUROPEAN PATENT SPECIFICATION

(11) **EP 3 728 516 B1**
(45) Date of publication and mention of the grant of the patent: **03.07.2024**
(21) Application number: 18892070.6
(22) Date of filing: 21.12.2018
(51) Int. Cl.: C10C 3/00, C04B 35/83, C10C 3/02, C10C 3/06, C10C 3/08, D01F 9/145, C01B 32/158, C01B 32/182, C01B 32/20, C01B 32/30, C10B 53/04, C10B 57/08, C10G 1/00, C10G 1/02, C10G 1/04, C10G 7/02, C10G 27/12, D01F 9/15, G01N 33/22, C10G 17/02

(54) **METHODS FOR PRODUCING CARBON FIBERS, RESINS, GRAPHENE, AND OTHER ADVANCED CARBON MATERIALS FROM COAL**
VERFAHREN ZUR HERSTELLUNG VON KOHLENSTOFFASERN, HARZEN, GRAPHEN UND ANDEREN KOHLENSTOFFMATERIALIEN AUS KOHLE
PROCÉDÉS DE PRODUCTION DE FIBRES DE CARBONE, DE RÉSINES, DE GRAPHÈNE ET D'AUTRES MATÉRIAUX CARBONÉS AVANCÉS À PARTIR DE CHARBON

(30) Priority: 22.12.2017 US 201762610037 P
(43) Date of publication of application: 28.10.2020
(73) Proprietor: Carbon Holdings Intellectual Properties, LLC, Sheridan, WY 82801 (US)
(72) Inventor: ATKINS, Charles, Sheridan, Wyoming 82801 (US); LINDEMANN, Garrett, Buffalo, Wyoming 82834 (US); TARGETT, Matthew, Sheridan, Wyoming 82801 (US)
(74) Representative: McKinnon, Alistair James
(86) International application number: PCT/US2018/067341
(87) International publication number: WO 2019/126782

(56) References cited:
- WO-A2-2016/118214
- US-A- 4 439 304
- US-A1- 2009 061 193
- US-A1- 2011 011 719
- US-A1- 2014 223 882
- US-A1- 2016 060 122
- US-A1- 2017 198 221
- KIM et al.: "Pitch-based carbon fibers from coal tar or petroleum residue under the same processing condition", Carbon Letters, vol. 19, 2016, pages 72-78, XP055620575,

## Description

### CROSS-REFERENCE TO RELATED APPLICATIONS

### BACKGROUND

Coal is a highly varied heterogeneous material that has been mined and principally used for three purposes over thousands of years: 1) the generation of thermal heat and power generation through incineration, 2) the production of steel and other metals by coking, and 3) the production of what are now widely known as "petrochemicals" through pyrolysis or liquefaction. Despite the fact that coal has been extensively used for thousands of years, more than 99% of it has been incinerated to produce heat and power. This process is now widely known to produce a host of adverse environmental and economic effects.

Additional uses of coal has been the topic of research for many years. The basic chemistry of coal was well understood by at least the early twentieth century. Significant research was conducted with the aim of deriving liquid transportation fuels from coal in order to supplant petroleum, One notable breakthrough was the development of the Fischer-Tropsch process in Germany, around 1925, which converted gasified coal into liquid hydrocarbons. Additionally, Sasol, a major South African company, focused on the conversion of solid coal to liquid transportation fuels via catalytic cracking. Similarly, the United States Department of Energy sought to develop coal-based transportation fuels as an alternative to petroleum-based fuels. However, due to research driven petroleum technology and the decreasing costs of petroleum, the use of coal to produce liquid transportation fuels at large scales never became economically feasible.

Although significant research has been conducted on coal liquefaction and the use of coal to form other products for more than a century, the ability to produce high-value, high-performance carbon based products, such as carbon fibers, from coal remains an open question. In recent years, carbon-based technologies have come to the forefront, with rapid developments being made in in the commercialization of advanced carbon materials such as carbon fibers, resins, graphene, and carbon nanotubes. As these advanced materials are increasingly used in mass produced, high volume applications, there is a need to quickly and economically supply large quantities of advanced carbon materials to manufacturers. Thus, while improvements in the derivation of fuels and other products from coal are being explored, there remains significant work to be done in developing processes to convert coal into the advanced carbon materials that will be instrumental in the economy of the future.

### SUMMARY

The present invention is directed to a method of producing graphene oxide from coal according to claim 1. Preferred embodiments are covered by the dependent claims.

The method of producing an advanced carbon material can include beneficiating the coal to produce a beneficiated amount of coal having less than about 5 wt. % of water.

The method of producing an advanced carbon material can include removing volatile matter from the coal. Removal of at least 50% of the volatile matter can be removed from the coal.

The method of producing an advanced carbon material can include processing the beneficiated amount of coal at the processing facility to produce an amount of pitch from at least some of the amount of coal, including subjecting the beneficiated amount of coal to a pyrolysis process.

The method of producing an advanced carbon material can include processing the beneficiated amount of coal at the processing facility to produce an amount of pitch from at least some of the amount of coal, including subjecting the beneficiated amount of coal to a direct liquefaction process.

The method of producing an advanced carbon material can include processing the beneficiated amount of coal at the processing facility includes subjecting the beneficiated amount of coal to an indirect liquefaction process.

The method of producing an advanced carbon material can include processing the beneficiated amount of coal at the processing facility includes producing an amount of solid char.

The method of producing an advanced carbon material can include treating at least some solid char to produce an amount of activated carbon.

The method of producing an advanced carbon material can include processing the beneficiated amount of coal at the processing facility includes producing an amount of coal liquid extract.

The method of producing an advanced carbon material can include treating at least some of the coal liquid extract to produce an amount of benzene.

The method of producing an advanced carbon material can include treating at least some of the coal liquid extract to produce an amount of paraxylene.

The method of producing an advanced carbon material can include using pitch that comprises one of mesophase pitch, isotropic pitch, or mesophase pitch.

The method of producing an advanced carbon material can include spinning at least some of the amount of pitch to produce the advanced carbon material.

The method of producing an advanced carbon material can include forming advanced carbon materials including one or more of carbon fibers, carbon nanotubes, graphite, graphene, graphite nano-platelets, fullerenes, pyrolytic carbon, carbon foams, and resins.

The method of producing an advanced carbon material can include treating at least some of the amount of pitch at the processing facility including treating a first amount of pitch to form a first advanced carbon material and treating a second amount of pitch to form a second advanced carbon material.

The method of producing an advanced carbon material wherein the first advanced carbon material includes carbon fibers and the second advanced carbon material includes a polymer.

The method of producing an advanced carbon material can include combining the carbon fibers and the polymer to form a carbon fiber reinforced polymer.

According to some embodiments, a method of producing synthetic graphite from coal at a processing facility, can comprise providing coal to the processing facility, beneficiating the coal to remove a desired amount of impurities therefrom, and processing the beneficiated coal to produce synthetic graphite.

The synthetic graphite includes a desired amount of impurities. The impurities can include one or more of cadmium, selenium, or other metals. The method can further comprise processing the synthetic graphite to produce synthetic graphene. Processing the synthetic graphite to produce synthetic graphene can comprise exfoliation. The synthetic graphene can include a desired amount of impurities.

According to some embodiments, a synthetic graphite formed from pitch derived from coal is described herein. The synthetic graphite can further comprise a desired amount of one or more impurities found in coal.

According to some embodiments, a synthetic graphene formed from pitch derived from coal is described herein. The synthetic graphene can further comprise a desired amount of one or more impurities found in coal.

In an embodiment, a method of producing at least one or more resins is disclosed. The method includes providing an amount of raw coal. The raw coal includes one or more impurities therein. The method also includes beneficiating the amount of raw coal to selectively removing at least a portion of some of the one or more impurities in the raw coal to form beneficiated coal. Additionally, the method includes processing the beneficiated coal to produce an amount of pitch. The method further includes modifying at least some of the pitch to produce the one or more resins. The one or more resins include a selected amount of a remainder of the one or more impurities that were not removed while beneficiating the amount of the raw coal, processing the beneficiated coal, and modifying at least some of the pitch.

In an embodiment, a method of producing an advanced carbon material at a single processing facility. The method includes providing an amount of raw coal to the single processing facility. The raw coal includes one or more impurities therein. The method also includes beneficiating the amount of raw coal at the single processing facility to selectively removing at least a portion of some of the one or more impurities in the raw coal to form beneficiated coal. Additionally, the method further includes processing the beneficiated coal at the single processing facility to produce an amount of pitch. The method further includes modifying at least some of the pitch at the single processing facility to produce the one or more resins. The one or more resins include a selected amount of a remainder of the one or more impurities that were not removed while beneficiating the amount of the raw coal, processing the beneficiated coal, and modifying at least some of the pitch.

In an embodiment, one or more resins formed from coal are disclosed. The one or more resins includes a plurality of mer units. Each of the plurality of mer units includes carbon and hydrogen. The one or more resins also include a selected amount of a remainder of one or more impurities that were initially present in raw coal.

A method of producing graphene from coal includes thermally processing coal at a temperature of at least about 300° F, and after the coal has been at least partially cooled from thermal processing, forming reduced graphene oxide from the coal.

In some cases, forming reduced graphene oxide from the coal can include oxidizing the coal to form a coal oxide, centrifuging the coal oxide, collecting precipitate from the coal oxide after centrifuging, the precipitate comprising graphene oxide, and reducing the graphene oxide to form reduced graphene oxide. Oxidizing the coal to form a coal oxide includes mixing the coal with at least one of sulfuric acid, nitric acid, or potassium permanganate, or hydrogen peroxide to form the coal oxide. Mixing the coal with at least one of sulfuric acid, nitric acid, potassium permanganate, or hydrogen peroxide to form the coal oxide can include mixing the coal with at least one of sulfuric acid and nitric acid, stirring the coal mixed with at least one of the sulfuric acid and the nitric acid, mixing potassium permanganate to the coal mixed with at least one of the sulfuric acid and the nitric acid, stirring the coal mixed with the potassium permanganate and at least one of the sulfuric acid and the nitric acid, diluting, with water, the coal mixed with the potassium permanganate and at least one of the sulfuric acid and the nitric acid to form a solution, mixing the solution with hydrogen peroxide, performing a first centrifugation of the solution mixed with the hydrogen peroxide, after performing the first centrifugation, separating a supernatant of the solution mixed with the hydrogen peroxide from precipitate of the solution mixed with the hydrogen peroxide, the supernatant including the coal oxide.

The method can further include diluting, with water, the coal oxide before centrifuging the coal oxide. In some cases, reducing the graphene oxide to form reduced graphene oxide includes sonicating the graphene oxide, and hydrothermally treating the graphene oxide in a par reactor after sonicating the graphene oxide. In some cases, thermally processing coal at a temperature of at least about 300°F includes heating the coal to a first temperature not to exceed 350°F, transferring the coal to a mercury removal reactor, heating the coal in the mercury removal reactor to a second temperature of at least 500°F, and contacting the coal with an inert gas to remove at least a portion of mercury present in the coal. In some cases, forming reduced graphene oxide from the coal includes forming the reduced graphene oxide from the coal at a reduced graphene oxide yield rate of between approximately 10 weight percent and approximately 20 weight percent of the coal. In some cases, forming reduced graphene oxide from the coal includes retaining a predetermined amount of one or more impurity atoms present in the amount of coal in the reduced graphene oxide. In some cases, the impurity atoms include one or more of boron, nitrogen, and silicon.

A method of producing synthetic graphene can include beneficiating an amount of coal including one or more impurity atoms to remove a predetermined amount of the one or more impurity atoms therefrom, processing the beneficiated amount of coal to produce an amount of pitch from at least some of the amount of coal, and treating at least some of the amount of pitch to produce the synthetic graphene, wherein the synthetic graphene includes a desired amount of the one or more impurity atoms. In some cases, the amount of pitch includes mesophase pitch. In some cases, the impurity atoms include one or more of silicon, nitrogen, and boron. In some cases, the impurity atoms result in a predetermined amount of point defects in the synthetic graphene.

A method of producing synthetic graphene can include thermally processing an amount of coal to achieve a predetermined concentration of one or more impurity atoms in the amount of coal, oxidizing at least some of the amount of coal to form a coal oxide including a predetermined concentration of one or more impurity atoms, and processing the coal oxide to form reduced graphene oxide including a predetermined concentration of one or more impurity atoms.

In some cases, the one or more impurity atoms occur naturally in the amount of coal. In some cases, the impurity atoms include one or more of cadmium, selenium, boron,

### BRIEF DESCRIPTION OF THE DRAWINGS

The accompanying drawings illustrate various embodiments of the present apparatus and are a part of the specification. The illustrated embodiments are merely examples of the present apparatus and do not limit the scope thereof.
FIG. 1 is a flow chart of an example method 100 to form carbon fibers, according to an embodiment.
FIG. 2 is a flow chart of an example method 200 to form carbon fibers using a direct liquefaction process, according to an embodiment.
FIG. 3 is a flow chart of an example method 300 to form carbon fibers using an indirect liquefaction process, according to an embodiment.
FIG. 4 is a flow chart of an example method 400 to form carbon fibers using one or more membranes, according to an embodiment.
FIG. 5 is a flow chart of an example method 100 to form one or more resins, according to an embodiment.
FIG. 6 is a flow chart of an example method 200 to form one or more resins using a direct liquefaction process, according to an embodiment.
FIG. 7 is a flow chart of an example method 300 to form one or more resins using an indirect liquefaction process, according to an embodiment.
FIG. 8 is a flow chart of an example method 400 to form one or more resins using one or more membranes, according to an embodiment.
FIG. 9 illustrates a process flow diagram of an example of a method of producing advanced carbon materials from coal in accordance with the present disclosure
FIG. 10 illustrates a process flow diagram of an example of a method of producing advanced carbon material from coal including a pyrolysis process in accordance with the present disclosure
FIG. 11 illustrates a process flow diagram of an example of a method of producing advanced carbon material from coal including a direct liquefaction process in accordance with the present disclosure.
FIG. 12 illustrates a process flow diagram of an example of a method of producing advanced carbon material from coal including an indirect liquefaction process in accordance with the present disclosure.
FIG. 13 illustrates a material flow diagram of an example of a method 500 of producing carbon fiber and, optionally, one or more advanced carbon materials from coal in accordance with the present disclosure, according to an embodiment.
FIG. 14 illustrates a process flow diagram of an example of a method of producing graphene from coal in accordance with the present disclosure.
FIG. 15 illustrates a process flow diagram of an example of a method of producing reducing graphene oxide from coal in accordance with the present disclosure.
FIG. 16A is a Raman spectroscopy graph of a sample of Monarch seam coal in accordance with the present disclosure.
FIG. 16B is a Raman spectroscopy graph of a sample of thermally-treated Monarch seam coal in accordance with the present disclosure.
FIG. 16C is a Raman spectroscopy graph comparing the sample of Monarch seam coal of FIG. 8A to the sample of thermally-treated Monarch seam coal of FIG. 8B in accordance with the present disclosure.
FIG. 16D is a Raman spectroscopy graph of a sample of graphene from thermally-treated Monarch seam coal, in accordance with this disclosure.
FIG. 17 is a diagram illustrating the flow of energy and coal in a processing facility for the production of one or more advanced carbon materials as described herein and according to some embodiments.
FIG. 18 is a diagram illustrating the process flow of raw coal, for example from a high wall coal mine, as it is processed according to the embodiments described herein to form various advanced carbon materials, such as activated carbon, graphene, materials for use in batteries, and building and construction materials, according to an embodiment.
FIG. 19 is a diagram illustrating the process flow of raw coal to various advanced carbon materials according to the processes described herein, according to an embodiment.

Throughout the drawings, identical reference numbers designate similar, but not necessarily identical, elements.

### DETAILED DESCRIPTION

As described below, carbon fibers can be produced from raw, mined coal. In an embodiment, raw coal can be transported to a processing facility. The coal can then be beneficiated in order to remove a desired amount of impurities. The impurities (e.g., any compound or element other than carbon or hydrogen) removed from the coal can include at least one of mercury, arsenic, cadmium, other heavy metals, water, or volatile compounds. In some cases, beneficiation can include heating the coal to remove these impurities. The beneficiated coal can then be processed to produce pitch and, optionally, one or more additional advanced carbon materials (*i.e.*, non-carbon fiber advanced carbon materials). The processing can include subjecting the beneficiated coal to a liquid extraction process (*e*.*g*., pyrolysis process, direct liquefaction process, indirect liquefaction process, or processing involving one or more membranes). In some cases, these processes disclosed herein can produce one or more byproducts (*e.g.*, at least one of gases, solid char, or coal liquid extract) in addition to pitch which themselves can be processed to form useful materials, such as carbon fiber and additional advanced carbon materials. For example, solid char can be processed to form activated carbon, and coal liquid extracted can be processed to form aromatic compounds such as benzene and paraxylene.

In some embodiments, the pitch produced by the processes described herein can be an isotropic pitch, and can be converted to a mesophase pitch by processing as needed or desired. The pitch can then be treated and/or modified to produce the carbon fiber *(e.g.,* the pitch can be spun to form carbon fibers), and, optionally, one or more additional advanced carbon materials (e.g., processed to form synthetic graphite, etc.). The carbon fiber and, optionally, the additional advanced carbon materials can be subjected to further processing, or can be delivered to third parties for use, for example in manufacturing. In some cases, the carbon fiber can be produced and combined to form secondary material, such as a resin or polymer to form a carbon fiber reinforced composite.

In some embodiments, one or more of the processes or process steps described herein can utilize or be carried out in the presence of one or more catalysts. For example, one or more process can include a hydrogenation catalyst. In some embodiments, the catalyst can comprise a metal *(e.g.,* platinum). In some cases, the catalyst can be a multi-part catalyst *(e.g.,* a catalyst comprising two or more metals). In some cases, a catalyst can include a ceramic or mineral material *(e.g.,* a silicate material, such as an aluminosilicate material). In some cases, a catalyst can include any catalytic material now known or as can yet be discovered for use in processing coal.

In some embodiments, all of the beneficiation, processing, and treatment steps described herein can be performed at a single processing facility, for example a single processing plant or compound. However, in other embodiments, one or more steps can be performed at separate facilities and the products of each step can be stored and transported between each facility. As used herein, the term processing facility can refer to one or more laboratories, buildings, process flows, or other apparatuses at about the same geographic location. For example, a processing facility can comprise a single building or factory complex at a single geographic location which comprises such equipment to perform the processes and methods described herein.

In an embodiment, carbon fiber is the only advanced carbon material produced during the processes disclosed herein. In an embodiment, as previously discussed, the processes disclosed herein can form carbon fiber and one or more additional advanced carbon materials. In an example, the one or more additional advanced carbon materials can include, but are not limited to, resins (e.g., polyacrylonitrile, polyurethane resins, cyanate ester resins, epoxy resins, methacrylate resins, polyester resins, and other suitable resins), carbon foams, single-walled carbon nanotubes, multi-walled carbon nanotubes, carbon megatubes, graphite, graphene, graphite nano-platelets, nanoribbons, nanobuds, fullerenes *(e.g.,* buckminsterfullerene and multi-cored fullerenes), quantum dots, activated carbon, and pyrolyzed carbon. In an example, the additional advanced carbon materials produced by the processes described herein can also include, but are not limited to polymers. In an example, the additional advanced carbon materials produced by the processes described herein can also include one or more materials that can be used as precursors in the formation of additional advanced carbon materials. Examples of the precursors can include at least one of alkanes, alkenes, or alkynes. In an example, the additional advanced carbon materials can comprise biologically useful materials or biopolymers *(e.g.,* at least one of proteins, amino acids, nucleic acids, collagen, chitosan, or sugars).

Producing the carbon fiber and, optionally, the one or more additional advanced carbon materials from coal has several advantages over producing the carbon fiber and, optionally, the one or more additional advanced carbon materials from other carbon sources *(e.g.,* oil). For example, the supply and price of oil is highly volatile which can affect the ability of manufacturers to obtain oil for the production of the carbon fibers and the additional advanced carbon materials. This, in turn, can cause shortages of the carbon fibers and the additional advanced carbon materials which can hamper manufacturer's ability to make devices, parts, etc. that include the carbon fiber and the additional advanced carbon materials. Additionally, producing the resin from coal produces at least one of resin, pitch, or one or more byproducts exhibiting a low hydrogen to carbon ratio *(e.g.,* a hydrogen to carbon ratio of less than about 0.5, less than about 0.2, or less than about 0.1). The low hydrogen to carbon ratio can at least one improve the yield of resin formed from the coal, eliminate the need for an external source of hydrogen, or reduce the amount of carbon dioxide produced during the processes disclosed herein. Further, coal can include one or more impurities therein. The presence of the one or more impurities can affect the properties of the carbon fiber and the additional advanced carbon material. For instance, at least some of at least one of the impurities can intentionally and selectively not be removed from the coal during the beneficiation process or during another process. The impurity or impurities that are not removed from the coal can act as dopants in the carbon fiber and the one or more additional advance carbon materials which, in turn, can affect the properties of the carbon fiber and the one or more additional advanced carbon materials. As such, the impurities that are present in the coal and the ability to selectively remove the impurities from the coal can allow for a high degree of control over the composition and properties of the carbon fiber and the additional advanced carbon materials that are manufactured from coal. Further, the presence of the impurities in the coal result in less processing being required to form the carbon fiber and the additional advanced carbon materials. For example, the maintaining the impurity or impurities in the coal can at least one of simplify the beneficiation processes or other purification processes, facilitate the operation of one or more other non-beneficiation processes, or preclude the need for actively doping the coal, carbon fiber, or additional advanced carbon materials. Additionally, the carbon fibers formed from coal can exhibit higher graphene levels, are more elastic, and exhibit higher tensile strengths than carbon fiber formed from oil.

### METHODS OF FORMING THE CARBON FIBER AND OTHER BYPRODUCTS FROM RAW COAL

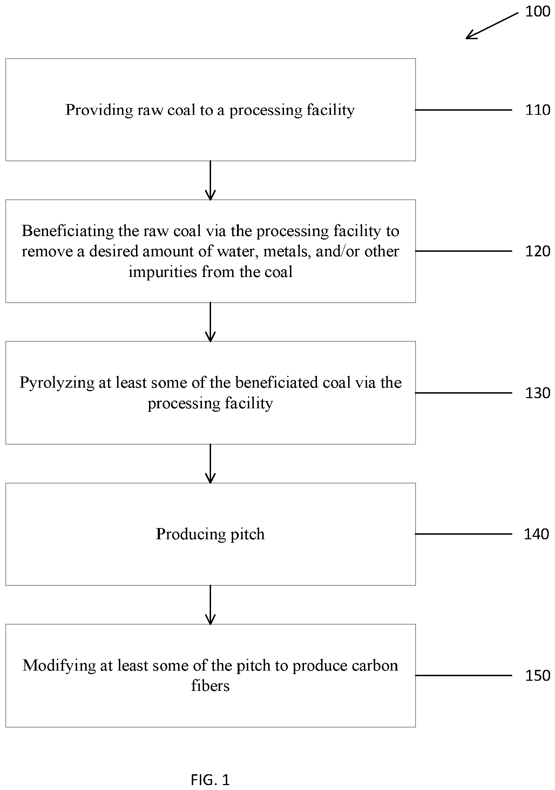

FIG. 1 is a flow chart of an example method 100 to form carbon fibers, according to an embodiment. The method 100 can include one or more of providing raw coal to a processing facility at block 110; beneficiating the raw coal via the processing facility at block 120 to remove a desired amount of water, metals, and/or other impurities from the coal; pyrolyzing at least some of the beneficiated coal via the processing facility at block 130; producing pitch at block 140; and modifying at least some of the pitch to produce carbon fibers at block 150. The carbon fibers can include a selected amount of a remainder of one or more impurities that were not removed during the method 100. The method 100 is only an example. As such, one or more blocks of the method 100 can be omitted, supplemented, combined, or divided. Further, the method 100 can include one or more additional acts, such as producing at least one byproduct from the coal *(e.g.,* at least one of char, one or more gases, or one or more coal liquid extracts) or treating the pitch to produce one or more additional advanced carbon materials.

In some embodiments, the raw coal can be provided to a processing facility at block 110 by any method that is now known or that can be developed in the future. For example, coal is generally extracted from naturally occurring layers or veins, known as coal beds or coal seams, by mining. Coal can be extracted by surface mining, underground mining, or various other forms of mining. Typically, coal that has been extracted via mining, but has not been otherwise processed is referred to as raw coal. In some cases, the raw coal can be extracted via a surface mining process, such as a high wall mining process, strip mining process, or contour mining process. In some cases, the raw coal can be extracted via an underground mining process, such as by a longwall mining process, continuous mining process, blast mining process, retreat mining process, or room and pillar mining process.

The raw coal can be mined or extracted from a location relatively near to the processing facility. For example, the processing facility can be located at, or near a coal extraction area. However, in other cases coal can be extracted from any location and transported to the processing facility. In some cases raw coal can be provided to the processing facility as needed to produce a desired amount of advanced carbon materials. However, in some other cases, raw coal can be provided and stored at the processing facility until it is processed.

The coal provided in block 110 can be ranked or graded based on its contents and properties. Although a variety of coal classification schemes exist, a general metamorphic grade is used herein to generally describe raw coal. These grades are used generally to aid in the understanding of the present disclosure and are not intended to limit to types of coal which can be used to produce the carbon fiber and, optionally, the one or more additional advanced carbon materials as described herein. While certain classifications of coal can be preferable for use in the processes described herein, such processes are not strictly limited to the discussed classifications of coal, if any. In some embodiments, the coal utilized by the processes described herein can be lignite coal, and can have a volatile content of greater than about 45 wt. %. In some embodiments, the coal can be sub-bituminous coal, bituminous coal, and/or anthracite coal. In some embodiments, the coal can be coal extracted from the Brook Mine near Sheridan, Wyoming. It is currently believed by the inventors that the composition of the coal extracted from the Brook Mine includes several impurities at beneficial concentrations that can facilitate the formation of carbon fiber exhibiting certain mechanical, chemical, and/or electrical properties, as discussed in more detail below. In some cases, the preferred coal for use in the processes described herein can be selected by the skilled artisan. For example, the preferred coal can be selected based one at least one of one or more destructive or nondestructive chemical analyzation techniques (e.g., Raman spectroscopy, energy dispersive x-ray spectroscopy, etc.) or one or more computing techniques. In some embodiments, the coal can exhibit an initial hydrogen to carbon ratio that is greater than about 0.7, such as in ranges 0.7 to about 1.0, about 0.7 to about 0.75, about 0.725 to about 0.0775, about 0.75 to about 0.8, about 0.0775 to about 0.85, about 0.8 to about 0.9, about 0.85 to about 0.95, or about 0.9 to about 1.0.

As previously discussed, the raw coal can be provided to a processing facility at block 110 for use in the method 100. The processing facility can have the capacity to store raw coal for use as needed, or can receive raw coal as needed to produce a desired amount of the carbon fiber. As is well known in the art, coal can be provided via truck, train, or any other form of transportation. Further, the processing facility can be situated at a coal extraction site, such that coal extraction site can be considered as part of the processing facility.

As previously discussed, the raw coal can include one or more impurities therein. The one or more impurities can include, but are not limited to volatile heavy metals *(e.g.,* mercury, selenium, arsenic, and cadmium), alkali metals (e.g., sodium metals, potassium metals), heteroatoms (e.g., sulfur, oxygen, and halogens), silicon, aluminum, titanium, calcium, iron, magnesium, sodium, potassium, sulfur, strontium, barium, manganese, phosphorus, antimony, arsenic, barium, beryllium, boron, bromine, cadmium, chlorine, chromium, cobalt, copper, fluorine, lead, lithium, manganese, mercury, molybdenum, nickel, selenium, silver, strontium, thallium, tin, vanadium, zinc, and/or zirconium or oxides thereof. For example, depending on the impurity and the source of the raw coal, any of the impurities disclosed herein can form 0 weight percent ("wt. %") to about 25 wt. % of the raw coal, such as in ranges of about greater than 0 wt. % to about 0.001 wt. %, about 0.0005 wt. % to about 0.002 wt. %, about 0.001 wt. % to about 0.003 wt. %, about 0.002 wt. % to about 0.004 wt. %, about 0.003 wt. % to about 0.005 wt. %, about 0.004 wt. % to about 0.006 wt. %, about 0.005 wt. % to about 0.008 wt. %, about 0.007 wt. % to about 0.01 wt. %, about 0.009 wt. % to about 0.02 wt. %, about 0.01 wt. % to about 0.03 wt. %, about 0.02 wt. % to about 0.04 wt. %, about 0.03 wt. % to about 0.05 wt. %, about 0.04 wt. % to about 0.06 wt. %, about 0.05 wt. % to about 0.08 wt. %, about 0.07 wt. % to about 0.1 wt. %, about 0.09 wt. % to about 0.2 wt. %, about 0.1 wt. % to about 0.3 wt. %, about 0.2 wt. % to about 0.4 wt. %, about 0.3 wt. % to about 0.5 wt. %, about 0.4 wt. % to about 0.6 wt. %, about 0.5 wt. % to about 0.8 wt. %, about 0.7 wt. % to about 1 wt. %, about 0.9 wt. % to about 2 wt. %, about 1 wt. % to about 4 wt. %, about 3 wt. % to about 6 wt. %, about 5 wt. % to about 8 wt. %, about 7 wt. % to about 10 wt. %, about 9 wt. % to about 15 wt. %, or about 10 wt. % to about 25 wt. %.

At block 120, the raw coal can be beneficiated to remove at least some of at least one of the impurities that are present in the raw coal to form beneficiated coal (also known as upgraded coal), according to an embodiment. For example, the raw coal can be beneficiated to remove at least one water, heavy metals, volatile compounds, alkali metals, or heteroatoms from the raw coal, thereby producing the beneficiated coal. In an embodiment, the raw coal can be beneficiated to remove a significant portion of at least one of the impurities.

The beneficiation process can include heating the raw coal to one or more desired temperatures. The one or more desired temperature can be about 100 °C to about 500 °C, such as in ranges of about 100 °C to about 290 °C, 100 °C to about 150 °C, about 125 °C to about 200 °C, or about 150 °C to about 290 °C. The temperature that the raw coal is heated to can be selected to selectively remove at least some of at least one of the impurities that are present in the raw coal. For example, the raw coal can be heated to a temperature of about 100 °C to about 150 °C to remove moisture from the raw coal and about 150 °C to about 290 °C to remove volatile metals from the raw coal. In some cases, the beneficiation process can comprise heating the raw coal to a first desired temperature. Heating the raw coal to the first desired temperature can remove one or more first impurities. In some embodiments, beneficiation can then include heating the raw coal to a second, higher desired temperature. Heating the raw coal to the second desired temperature can remove one or more second impurities.

The beneficiation process can include heating the raw coal to the desired temperature for a desired duration. The desired duration can be about 1 second to several days, such as in ranges of about 1 second to about 1 minute, about 30 seconds to about 30 minutes, about 1 minute to about 1 hour, about 30 minutes to about 3 hours, about 1 hours to about 5 hours, about 3 hours to about 10 hours, about 7 hours to about 18 hours, about 12 hours to about 1 day, or about 18 hours to about 3 days. Typically, increasing the duration that the raw coal is heated to the desired temperature can increase the amount of the one or more impurities are removed from the raw coal. However, the raw coal can exhibit a maximum duration where heating the raw coal for periods of time longer than the maximum duration will have little or no effect on the amount of the one or more impurities that are removed from the raw coal. In some cases, the beneficiation process can comprise heating the raw coal to a first desired temperature for a first duration followed by heating the raw coal to a second, higher desired temperature for a second duration. The first and second durations can be the same or different.

In an embodiment, the beneficiation process can include heating the raw coal in an atmosphere comprising a halogen gas which can facilitate removal of one or more of the impurities from the raw coal and prevent oxidation or other reactions with the raw coal. In some embodiments, the coal can be beneficiated by heating the raw coal in an atmosphere including hydrogen which can increase the hydrogen to carbon ratio. In such an embodiment, the hydrogen can be provided from an outside hydrogen source or from hydrogen that was collected in another stage of the method 100. In some embodiments, the coal can be beneficiated by heating the raw coal in an atmosphere that is substantially hydrogen free. In such an embodiment, the substantially hydrogen free atmosphere can decrease the hydrogen to carbon ratio.

In some embodiments, the coal can be beneficiated by heating the raw coal in an atmosphere including hydrogen which can increase the hydrogen to carbon ratio. In such an embodiment, the hydrogen can be provided from an outside hydrogen source or from hydrogen that was collected in another stage of the method 100. In some embodiments, the coal can be beneficiated by heating the raw coal in an atmosphere that is substantially hydrogen free. In such an embodiment, the substantially hydrogen free atmosphere can decrease the hydrogen to carbon ratio.

In some other embodiments, the coal can be beneficiated by heating the coal to a desired temperature in the presence of one or more catalyst compounds. In some cases, beneficiating the coal can comprise pyrolyzing the coal, for example in the presence of a catalyst. In some embodiments, the raw coal can be beneficiated at or near atmospheric pressure *(e.g.,* 0.8 to about 1.2 atmospheres), though the raw coal can be beneficiated at higher or lower pressures.

In some embodiments, beneficiation can include subjecting the raw coal to a WRITECoal beneficiation process, as described, for example, in U.S. Patent No. 9,181,509 which is hereby incorporate by reference in its entirety. In some other embodiments, the coal can be beneficiated by heating the coal to a desired temperature in the presence of one or more catalyst compounds. In some cases, beneficiating the coal can comprise pyrolyzing the coal, for example in the presence of a catalyst. In some cases, the coal can be beneficiated by the BenePlus System, as developed and licensed by LP Amina and as described, for example, in U.S. Patent Publication No. 2017/0198221.

The beneficiated coal can comprise a significantly reduced amount (e.g., at least 25 wt. %, at least 50 wt. %, at least 75 wt. %, at least 90 wt. %, at least 95 wt. %, or at least 99 wt. %) of at least one of mercury, cadmium, other heavy metals, water, any of the other impurities disclosed herein, or any other impurity that can be present in the raw coal. The amount of the impurities that are left in the beneficiated coal can depend on the temperature that the raw coal was heated, the duration that the raw coal was heated, the atmosphere that the raw coal was exposed to during the beneficiation process, the presence of catalysts, etc. For example, beneficiating the coal can reduce the amount of mercury in the coal by about at least about 70 wt. %, 75 wt. %, 80 wt. %, 85 wt. %, 90 wt. %, or 92 wt. % or more. In some cases, beneficiating the coal can reduce the water or moisture content of the coal to less than about 5 wt. %, 4 wt. %, 3 wt. %, 2 wt. %, or 1.5 wt. % or lower. In some cases, beneficiating the coal can remove one or more of hydrogen, sulfur, oxygen, arsenic, selenium, cadmium, or volatile matter from the coal. The amount of one or more of these elements in the coal can be reduced by about 25 wt. % to about 90 wt. %.

However, as previously discussed, it can be desirable for a desired amount of one or more impurities to remain in the beneficiated coal after being subjected to a beneficiation process. For example, the beneficiation process can remove a desired amount of impurities such that a predetermined amount of mercury, cadmium, selenium, alkali metals, heterogeneous elements, and/or another element can selectively remain in the beneficiated coal after processing. In some cases, the desired amount of impurity that can remain in the beneficiated coal can be useful in the subsequent formation of the carbon fiber or, optionally, the one or more additional advanced carbon materials. In some cases, the desired amount of the impurity that can remain in the beneficiated coal can be incorporated into the carbon fiber and, optionally, the one or more advanced carbon materials. For example, where the advanced carbon material comprises synthetic graphene, a desired amount of cadmium can remain in the beneficiated coal and can be incorporated into the synthetic graphene to thereby improve the electrical, mechanical, or chemical properties thereof

In an embodiment, the beneficiation process can be configured to maintain at least some of any of the impurities disclosed herein at any of the concentrations disclosed herein in the beneficiated coal after block 120 (but before block 130).

In an embodiment, the beneficiation process can be configured to decrease the hydrogen to carbon ratio of the coal. For example, after the beneficiation process, the hydrogen to carbon ratio of beneficiated coal can less than about 0.8, such as in ranges of about 0.6 to about 0.7, about 0.65 to about 0.75, or about 0.7 to about 0.8.

In some embodiments, beneficiating the coal during act 120 can produce one or more byproducts, such as one or more byproducts that can be captured and used in later processing steps, that can be valuable in and of themselves, or that can be subjected to further processing or use in the method 100. For example, beneficiating the coal can produce or separate gases or coal liquid extracts from the raw coal. These gases and/or coal liquid extracts can be captured or separated during processing. For example, beneficiating the coal at block 120 can produce at least one of H₂, CO₂, CO, CH₄, C₂H₄, C₃H₆, or other hydrocarbon gases, which can be captured and subsequently utilized in block 130 or in other process steps. In some cases, beneficiating the coal can result in coal liquid extracts (e.g., toluene or benzene) which can be captured for subsequent use or processing. In some cases, the impurities removed from the coal by the beneficiation process at block 120 can be captured for subsequent use. For example, water removed from the coal by the beneficiation process can be capture and utilized in subsequent process steps. In some embodiments, beneficiating the coal can also produce a solid material known as ash or char. In some cases, this char can be subjected to further processing to form activated carbon.

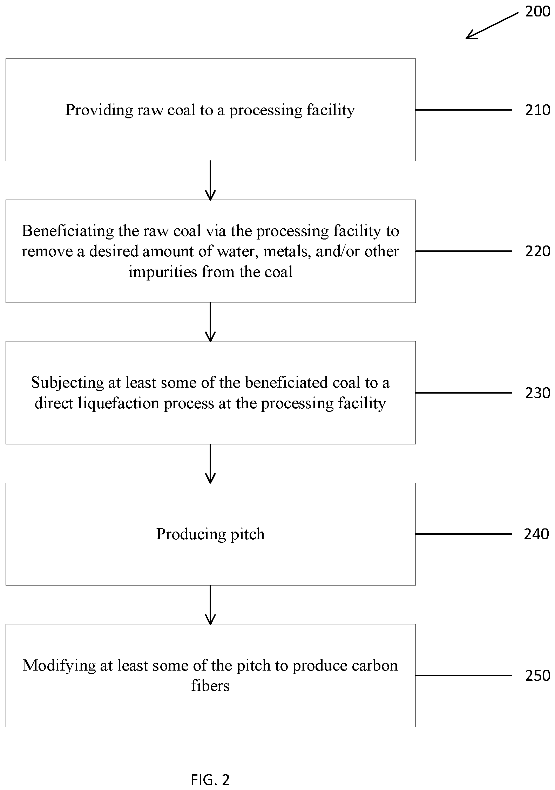

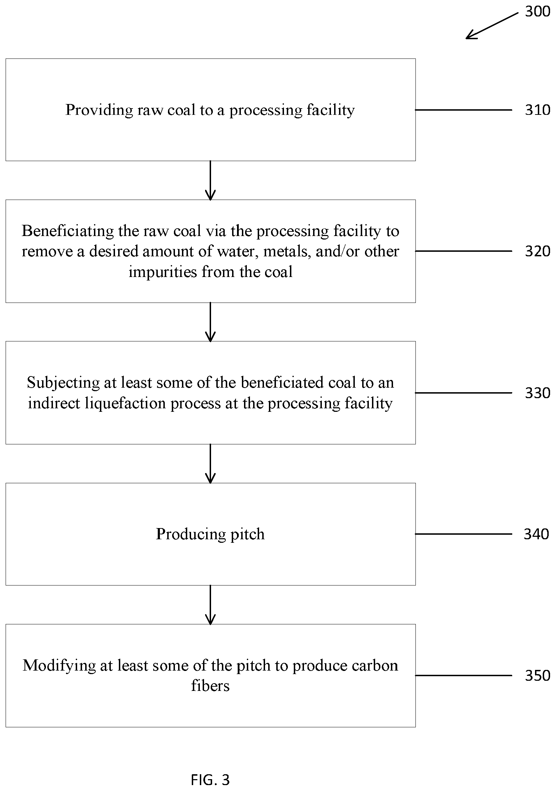

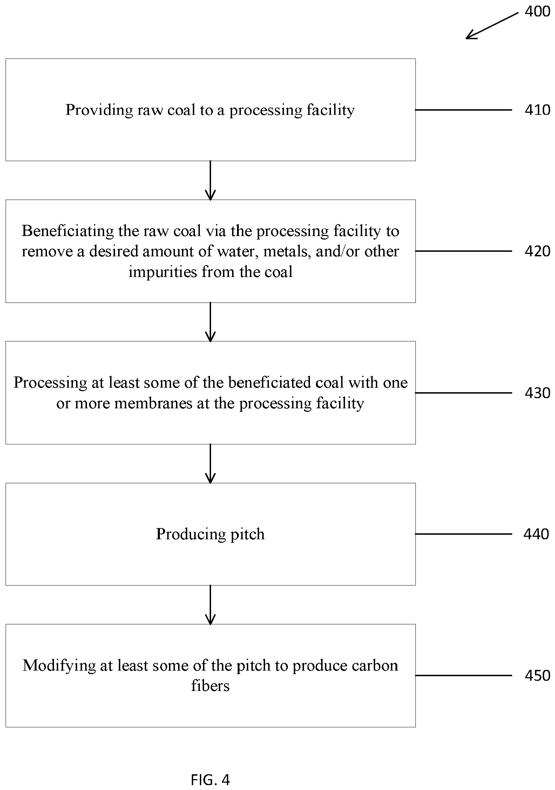

At block 130 the beneficiated coal can be processed via the processing facility. In some embodiments, processing the beneficiated coal can include subjecting the beneficiated coal to a liquid extraction process, such as a pyrolysis process *(e.g.,* a high temperature pyrolysis process or a mild temperature pyrolysis process). It is noted that other liquefaction processes can be used instead of or in conjunction with the pyrolysis process, such as using a direct liquefaction process (discussed in more detail with regard to FIG. 2), an indirect liquefaction process (discussed in more detail with regard to FIG. 3), membranes (e.g., discussed in more detail with regard to FIG. 4), an electric arc process, a super critical solvent extraction process, or an electromagnetic heating process. The liquid extraction process can convert the beneficiated coal into at least one of pitch, one or more gases, one or more coal liquid extracts, or char.

In an embodiment, the liquid extraction processes of block 130 can comprise pyrolyzing the beneficiated coal via the processing facility. Pyrolyzing the beneficiated coal to form carbon fibers can include heating the beneficiated coal to a desired temperature for a desired duration, with or without elevating the pressure applied to the beneficiated coal. At the elevated temperatures, some of the organic structures within the beneficiated coal begin to breakdown forming lower molecular weight pyrolytic fragments. Some of the lower molecular weight pyrolytic fragments can escape the beneficiated coal as light gases *(e.g.,* hydrogen, methane, carbon dioxide, etc.). These light gases can be captured and/or reused as discussed in more detail below. However, some of the lower molecular weight pyrolytic fragments can recombine or depolymerize to form small ring aromatic structures (e.g., single ring aromatic structure). The small ring aromatic structure can undergo condensation reactions wherein the small aromatic ring structure join together to form larger ring aromatic structures (e.g., polyaromatic hydrocarbons). Examples of the condensation reactions includes at least one of ring condensation, ring fusion, dehydrogenation, or other condensation reactions that grow the small ring aromatic structure. The larger ring aromatic structures can comprise isotropic pitches, isotropic resins, other products that include aligned polyaromatic layers, liquid crystalline structure (e.g. anisotropic pitches or anisotropic resins), mesophase pitches, or mesophase resins.

In an example, pyrolyzing the beneficiated coal can comprise a high temperature pyrolysis process that includes heating the beneficiated coal to a temperature greater than about 1000 °C at atmospheric process. The high temperature pyrolysis process can form benzene compounds, phenol compounds, high value oil, or other compounds that are useful in the formation of carbon fibers. In an example, pyrolyzing the beneficiated coal can comprise a mild temperature pyrolysis process that includes heating the beneficiated coal to a temperature of about 400 °C to about 650 °C at atmospheric pressure. The mild temperature pyrolysis process is likely to form coke than the high temperature pyrolysis process which can facilitate the formation of the resins disclosed herein. In some cases, the coal can be heated at high pressure *(e.g.,* a pressure greater than about 1 atmosphere) and in the presence of a solvent. For example, the beneficiated coal can be pyrolyzed in the presence of a CO₂ solvent which can be held in a supercritical state. In some cases, the beneficiated coal can be pyrolyzed in a hydrogen atmosphere (e.g., hydrogen provided from an outside hydrogen source or hydrogen collected during the method 100) to increase the hydrogen to carbon ratio of the beneficiated coal or pyrolyzed in a hydrogen free atmosphere to decrease the hydrogen to carbon ratio of the beneficiated coal. In an embodiment, the beneficiated coal can be pyrolyzed in a hydrogen atmosphere (e.g., hydrogen provided from an outside hydrogen source or hydrogen collected from another stop of the method 100) to increase the hydrogen to carbon ratio of the coal or pyrolyzed in a hydrogen free atmosphere to decrease the hydrogen to carbon ratio of the coal. In some cases, the beneficiated coal can be pyrolyzed by the MuSCL System developed by TerraPower described, for example, in U.S. Patent No. 10,144,874.

Additional examples of pyrolysis processes that can be used to process the beneficiated coal are described in U.S. Patent Application Publication No. 2017/0198221, U.S. Patent Application Publication No. 2018/0311657, and The ENCOALmild gasification project, a DOEAssessment, DOE/NETL-2002/1171 (2002)

In some embodiments, the pyrolysis process can comprise exposing the beneficiated coal to electromagnetic radiation at a desired intensity and for a desired duration. For example, block 130 can comprise exposing the beneficiated coal to microwave and/or radiofrequency (RF) radiation for a desired duration as part of the pyrolysis process. In some cases, this pyrolysis process can result in the bulk of the beneficiated coal remaining below pyrolytic temperatures, while individual particles of coal can be subjected to temperatures greater than about 1200 F. In some cases, this pyrolysis process can also comprise methane activation and/or methylation of at least some of the carbon comprising the beneficiated coal. In some embodiments, the beneficiated coal can be pyrolyzed by the Wave Liquefaction process developed by H Quest Vanguard, Inc. as described, for example, in U.S. Patent Publication No. 2017/0080399.

Additional examples of processes for processing coal by exposing the beneficiated coal to electromagnetic radiation are disclosed in U.S. Patent No. 6,512,216 and U.S. Patent Application Publication No. 2017/0101584. Further examples of processes for processing coal by exposing the beneficiated coal to electromagnetic radiation are disclosed in U.S. Patent Application Publication No. 2018/0311657.

Block 130 can be configured to prevent or selectively maintain selected quantities of the one or more impurities in the pyrolyzed coal. For example, after block 130, the pitch can include any of the impurities disclosed herein at any of the concentrations disclosed herein.

In an embodiment, after block 130, the pyrolyzed can exhibit a hydrogen to carbon ratio of less than about 0.8, such as in ranges as less than about 0.1, less than about 0.2, about 0.1 to about 0.2, about 0.15 to about 0.25, about 0.2 to about 0.4, about 0.3 to about 0.5, about 0.4 to about 0.6, or about 0.5 to about 0.7.

At block 140. nitch and. optionally. one or more byproducts (*e.g*.. at least one of one or more gases, one or more coal liquid extracts, or char) are processed (e.g., extracted to the pyrolyzed coal) via the processing facility. In an embodiment, block 140 can represent the result of at least one of block 120 or block 130, rather than a separate action or process step. In an embodiment, block 140 can be performed substantially simultaneously with at least one of block 120 or block 130. In an embodiment, block 140 can be performed after at least one of block 120 or block 130.

In an embodiment, block 140 can include adding one or more additives to the beneficiated coal. For example, one or more other gases or liquids can be used during block 140 to add one or more additives to the beneficiated coal. Examples of gases or liquids that can be used during block 104 include hydrogen containing gases, natural gases, CO₂, petroleum products, one or more materials or compounds that are produced during at least one of block 120 or block 130, or one or more materials or compounds that can be produced by or captured during previous iterations of method 100.

As previously discussed, the raw coal can include one or more impurities therein and blocks 120 and 130 can be configured to not remove at least some of the impurities such that the beneficiated or pyrolyzed coal includes at least some of the impurities. The presence of the one or more impurities in the beneficiated or pyrolyzed coal can make adding one or more additives to the beneficiated or pyrolyzed coal unnecessary or can reduce the amount of additives that are added to the beneficiated or pyrolyzed coal. As such, the presence of the one or more impurities in the beneficiated or pyrolyzed coal can make the method 100 more efficient than methods of forming carbon fibers from a non-coal source since the beneficiated or pyrolyzed coal can have less additives added thereto than the non-coal source.

In some embodiments, pitch can be produced via the processing facility at block 140. As used herein, pitch, also known as coal pitch, coal tar, or coal tar pitch, can refer to a mixture of one or more typically viscoelastic polymers as will be well understood by the skilled artisan. In some embodiments, the pitch produced at block 140 can be a direct result of processing the beneficiated coal at step 130. The pitch produced at block 140 can comprise one or more high molecular weight polymers. In some embodiments, the pitch can have a melting point of greater than about 650 °F. In some embodiments, the pitch can have a melting point that is high enough that some of the pitch (e.g., portions of the pitch not used to form the carbon fiber) can be used in a carbon fiber spinning process without the need for a plasticizer.

In an embodiment, the pitch can comprise aromatic hydrocarbons, for example polycyclic aromatic hydrocarbons. In some cases, the pitch can comprise at least about 50 wt. % polycyclic aromatic hydrocarbons, at least about 60 wt. %, 70 wt. %, 80 wt. %, 90 wt. %, 95 wt. %, or 99 wt. % or greater of polycyclic aromatic hydrocarbons. In an embodiment, the pitch can comprise less than about 0.1 wt. % ash or other solid material, less than about 0.05 wt. % ash or solid material, or less than about 0.01 wt. % ash or solid material. In some cases, the pitch can have a flash point greater than about 230 °F, greater than about 250 °F, greater than about 300 F, or in ranges of about 230 °F to about 250 °F, about 240 °F to about 275 °F, about 250 °F to about 300 °F, about 275 °F to about 350 °F, or about 300 °F to about 400 °F. In some cases, the pitch can have an API gravity of less than about 4, less than about 3, or less than about 2, or less than about 1.5. In some embodiments, the pitch produced by the method 100 is not coke pitch. That is, in some cases, the pitch produced at block 140 is not produced from coke or a coke-based material. In some embodiments, coke is not produced at any point during the method 100.

In some embodiments, the pitch can include any of the impurities disclosed herein at any of the concentrations disclosed herein.

In some embodiments, the pitch can have a hydrogen to carbon ratio of about of less than about 0.8, such as in ranges as less than about 0.1, less than about 0.2, about 0.1 to about 0.2, about 0.15 to about 0.25, about 0.2 to about 0.4, about 0.3 to about 0.5, about 0.4 to about 0.6, or about 0.5 to about 0.7.

In an embodiment, the pitch can be relatively free of one or more selected impurities, such as water, non-carbon atoms including sulfur or nitrogen, or material such as coal ash or char, one or more non-carbon atoms (e.g., one or more of mercury, selenium, cadmium, arsenic, alkali metals, oxygen, halogens, sulfur or nitrogen), or material such as coal ash or char. For example, the pitch can comprise less than about 0.2 wt. % water, less than about 0.1 wt.%, less than about 0.05 wt.%, or less than about 0.01 wt. % water or lower. In an embodiment, the pitch can include one or more selected impurities in the concentrations disclosed above.

In an embodiment, the method 100 can also include producing one or more byproducts during block 140. Examples of the byproducts that can be formed during the block 140 are disclosed in [cited the other related applications]. For example, the byproducts produced during block 140 can include at least one of one or more gases, one or more coal liquid extract, or char. It is noted that the one or more byproducts can also be produced during at least one of block 120 or block 130 instead of or in addition to block 140.

The coal liquid extracts can refer to any material that is extracted or produced from raw coal or beneficiated coal that is liquid at or near normal temperature and pressure (about 68 F and 1 atmosphere of pressure). The one or more coal liquid extracts can comprise one or more liquid hydrocarbons. For example, coal liquid extracts can comprise one or more of benzene, toluene, alkanes or paraffins, alkenes, C2 compounds, C3 compounds, C4 compounds, T compounds, halogen compounds, phenols, or other saturated or unsaturated hydrocarbons. In some embodiments, the coal liquid extracts can include any of the impurities disclosed herein at any of the concentrations disclosed herein.

Char, also known as ash, can refer to any solid material which remains after gases, coal extract liquids, and/or pitch have been removed from raw coal. Char can be produced during at least one of block 120, block 130, or block 140. In an example, the char can comprise a solid high surface area carbonaceous material. In an example, the char can have a relatively low hydrogen to carbon ratio, such as a hydrogen to carbon ratio that is lower than the hydrogen to carbon ratio of pitch produced at block 140. In some cases, char can have a hydrogen to carbon ratio of from about 0.05 to about 0.65. In an example, char can additionally comprise at least some pitch material, which can be referred to herein as intrinsic binder impregnation. In some cases, any residual pitch or other gaseous or liquid materials can be removed from the char prior to any subsequent processing of the char.

In some embodiments, the char can include any of the impurities disclosed herein at any of the concentrations disclosed herein. In some embodiments, the char can have a hydrogen to carbon ratio of less than about 0.7, such as in ranges as less than about 0.1, less than about 0.2, about 0.1 to about 0.2, about 0.15 to about 0.25, about 0.2 to about 0.4, about 0.3 to about 0.5, about 0.4 to about 0.6, or about 0.5 to about 0.7.

The gases can comprise hydrogen and/or carbon. For example, the gases can include H₂, CO₂, CO, CH₄, C₂H₄, C₃H₆, and/or other hydrocarbon gases. The gases can also include sulfur. In some cases, these gases can be at least one of captured, otherwise contained, or used during the processes described herein. In some embodiments at least 50%, at least 75%, at least 90%, 95%, or 99% of any gaseous or volatile byproducts of the method 100 can be captured. The gases captured during certain process steps can be used in subsequent process steps as described herein. In some cases, gases produced by and a captured as part of the processes described herein can be utilized by these same or subsequent processes in order to increase the efficiency and/or cost effectiveness of said processes. In some cases, the capture and reuse of byproducts can improve the efficiency and/or lower the cost of the method 100.

In an embodiment, the method 100 can include reacting captured hydrogen gas (H₂) with captured carbon dioxide (CO₂) (*i*.*e.*, syngas) to form methane or another suitable gas or liquid thereby reducing the carbon footprint of the method 100. The captured hydrogen and carbon dioxide gas can be processed chemically (e.g., catalytically) to form monomeric compounds *(e.g.,* olefins such as ethylene and/or propylene) that can then be polymerized into higher molecular weight resin compounds. In an example, reacting the hydrogen with the carbon dioxide can reduce the amount of carbon dioxide produced by the method 100 by about 1% to about 99%, such as by about 1% to about 25%, about 20% to about 40%, or about 25% to about 50%.

In an embodiment, the pitch and the byproducts are all produced at block 140. In an embodiment, at least one of the pitch and at least one of the byproducts can be produced at separate times or separate processing steps from one another. In an embodiment, the pitch and at least one of the byproducts are produced together by the process 100 and, in particular, during block 140. In such an embodiment, the method 100 can include separating these products before any further processing of each individual product can occur. For example, pitch and coal liquid extracts can be simultaneously produced as a result of block 130 and can need to be separated from one another, by any process now know or which can be developed in the future, before further processing of either pitch or coal liquid extracts occurs.

In an embodiment, the pitch produced during block 140 are not subjected to further processing or refinement to alter the chemical composition of the pitch before block 150. In an embodiment, the pitch produced during block 140 can be subjected to one or more processes which can alter the chemical composition thereof prior to block 150. In an example, undesired impurities that remain in the pitch after block 140 can be removed therefrom prior to block 150. In such an example, the undesirable impurities can include impurities that could not be removed during block 120 and block 130 or excessive amounts of at least one impurity that is selected to be present in the carbon fiber. In an example, the pitch can be subjected to one or more processes to increase or decrease the hydrogen to carbon ratio of the pitch. In an example, the pitch can be subjected to one or more processes to produce mesophase pitch or otherwise alter the composition or properties of the pitch. In an embodiment, the one or more byproducts can also be used to form the carbon fiber during block 150. In such an embodiment, the one or more byproducts can or may not be subjected to further processing or refinement to alter the chemical composition thereof before block 150 using any of the processes disclosed herein or any other suitable process.

At block 150, at least the pitch produced at block 140 can be treated via the processing facility to produce at least one of the carbon fibers disclosed herein. In an embodiment, the pitch can be treated to product at least one of the carbon fibers disclosed herein by spinning the pitch to form carbon fibers. In some cases, spinning the pitch to form carbon fibers can include any process known in the art or developed in the future to convert pitch to carbon fibers, or carbon filament. In some cases, the pitch can be heated to a desired temperature during the spinning process, such as to about 650 °F. In some cases, forming the carbon fibers can comprise drawing, spinning, and heating the pitch to produce the carbon fibers. In some cases, forming the carbon fibers can comprise spinning filaments of the pitch, heating the pitch in air to a first temperature, and then heating the spun pitch in an inert atmosphere to a second, higher temperature to form carbon filament. In some cases, a plasticizer can be added to the pitch to aid in spinning the pitch, however in some other embodiments, plasticizer may not be added before spinning the pitch. In some embodiments, forming the carbon fibers can comprise treating the pitch to produce one or more of any of the advanced carbon materials described herein.

In an embodiment, block 150 can include adding one or more additives to the pitch or the carbon fiber if the pitch has already been treated to form the carbon fiber.

The carbon fibers formed during block 150 can include one or more impurities therein. The properties of the carbon fibers can depend, at least in part, on the one or more impurities that are present in the carbon fiber. In other words, the properties of the carbon fibers can be tunable via at least one of the addition, control, or removal of the impurities from the coal. For example, the properties of the carbon fibers that can depend on the presences of the one or more impurities can include at least one of elasticity (Young's modulus), tensile strength, failure mechanism, and the like. In an embodiment, the one or more impurities can include at least one impurity that was initially present in the raw coal thereby negating the need to add the at least one impurity into at least one of the raw coal, the beneficiated coal, the pitch, or the byproducts. In an embodiment, the one or more impurities can include at least one impurity that was added to at least one of the raw coal, the beneficiated coal, the pitch, or the byproducts.

In some embodiments, where the additional advanced carbon materials can comprise carbon fibers, the carbon fibers can have different or improved physical properties as compared to carbon fibers formed by conventional processes (e.g., carbon fibers produced by spinning polyacrylonitrile ("PAN")). The different or improved physical properties can be caused by the impurities that are present in the raw coal and that remain in the carbon fibers. In some cases, carbon fibers produced by the processes described herein can have a higher degree of molecular orientation along the fiber axis than carbon fibers produced from PAN. In some cases, carbon fibers produced by the processes described herein can have a higher elastic modulus than carbon fibers produced from PAN. In some cases, carbon fibers produced by the processes described herein can have a higher thermal and electrical conductivity than carbon fibers produced from PAN. However, in some embodiments, the additional advanced carbon materials can comprise PAN, and thus carbon fibers can be produced from PAN that is formed from coal according to the processes described herein.

In an embodiment, the carbon fibers can include any of the impurities disclosed herein at any of the concentrations disclosed herein.

In an embodiment, the hydrogen to carbon ratio of the resin can be about 0.7 to about 1.0, such as in ranges of about 0.7 to about 0.75, about 0.725 to about 0.0775, about 0.75 to about 0.8, about 0.0775 to about 0.85, about 0.8 to about 0.9, about 0.85 to about 0.95, or about 0.9 to about 1.0.

In an embodiment, the method 100 can include recycling carbon material (e.g., resin, pitch, etc.) exhibiting a hydrogen to carbon ratio that is greater than the desired hydrogen to carbon ratio, such as greater than about 0.2, greater than about 0.3, greater than about 0.4, greater than about 0.5, greater than about 0.6, or greater than about 0.7. For example, recycling the carbon material exhibiting a high hydrogen to carbon ratio can include adding the carbon material to the raw coal, the beneficiated coal, the pyrolyzed coal, the pitch before the pitch is processed, etc. Recycling the carbon material can increase the amount of resin or other advanced carbon material that is produced during the method 100.

As previously discussed, the beneficiated coal can be processed using a direct liquefaction process other than a pyrolysis process. The direct liquefaction process can include producing resins from coal under hydrogenation conditions to produce a naphtha which can further be "steam cracked" *(e.g.,* converted into so-called "naphtha cracker") to produce light olefin products (e.g., ethylene and/or) propylene. One example of a direct liquefaction process includes the H-Coal process developed in the Catlettsburg refinery and by Shenhua of China to produce liquid transportation fuels, chemical intermediates, and naphtha.

FIG. 2 is a flow chart of an example method 200 to form carbon fibers using a direct liquefaction process, according to an embodiment. Except as otherwise disclosed herein, the method 200 is the same or substantially similar to the method 100. The method 200 can include providing coal to a processing facility at block 210. The method 200 can also include beneficiating the coal via the processing facility at block 220 to remove a desired amount of water, metals, and/or other impurities from the coal. The method 200 can further includes subjecting at least some of the beneficiated coal to a direct liquefaction process at the processing facility at block 230. Additionally, the method 200 can include producing pitch at block 240. Also, the method 200 can include modifying the pitch to produce carbon fibers at block 250. The method 200 is only an example. As such, one or more blocks of the method 200 can be omitted, supplemented, combined, or divided. Further, the method 200 can include one or more additional acts.

Block 230 includes processing the beneficiated coal using a direct liquefaction process. The direct liquefaction process can include heating the beneficiated coal above a desired temperature (e.g., about 400 °C to about 500 °C) for a duration thereby converting the beneficiated coal into a liquid. In some cases, the beneficiated coal can be heated in the presence of one or more catalysts and/or at an elevated pressure. In some cases, the beneficiated coal can be heated in an atmosphere comprising H. In some cases, the direct liquefaction process can be a hydrogenation or hydro-cracking process. In such cases, the direct liquefaction process can be performed in a hydrogen atmosphere. In some cases, a solvent can be added to the beneficiated coal during the direct liquefaction process. In some cases, the beneficiated coal can be subjected to a direct liquefaction process developed by Axens as described, for example, in U.S. Patent Publication No. 2017/0313886.

As previously discussed, the beneficiated coal can be processed using an indirect liquefaction process other than a pyrolysis process, The indirect liquefaction process can include producing resins from the raw coal in a high temperature gasification process to form so-called syngas (e.g., a mixture of hydrogen gas and at least one of carbon monoxide or carbon dioxide). The syngas can be processed chemically to form monomeric compounds that can then be polymerized into higher molecular weight resin compounds. For example, syngas can be converted into methanol which can be catalytically converted into light olefins (e.g., ethylene and/or propylene). Ethylene and/or propylene can be converted into carbon fibers. The light olefins formed during the indirect liquefaction process and/or the other process disclosed herein can also be precursors to other polymeric compounds. For example, the ammoxidation of propylene results in the formation of acrylonitrile monomers which can then be converted into polyacrylonitrile.

FIG. 3 is a flow chart of an example method 300 to form carbon fibers using an indirect liquefaction process, according to an embodiment. Except as otherwise disclosed herein, the method 300 is the same or substantially similar to the method 100 and/or method 200. The method 300 can include providing coal to a processing facility at block 310. The method 300 can also include beneficiating the coal via the processing facility at block 320 to remove a desired amount of water, metals, and/or other impurities from the coal. The method 300 can further includes subjecting at least some of the beneficiated coal to an indirect liquefaction process at the processing facility at block 330. Additionally, the method 300 can include producing syngas or another coa-derived element (e.g., char, pitch, etc.) at block 340. Also, the method 300 can include modifying the pitch to produce carbon fibers at block 350. The method 300 is only an example. As such, one or more blocks of the method 300 can be omitted, supplemented, combined, or divided. Further, the method 300 can include one or more additional acts.

Block 330 includes processing the beneficiated coal using an indirect liquefaction process. The indirect liquefaction process can include converting the beneficiated coal to a gas or gases and then converting the gas or gases into one or more liquids. The one or more gases can include, but are not limited to, syngas (e.g., a mixture of H and CO gas). In an example, the indirect liquefaction process can include heating the beneficiated coal to a desired temperature (e.g., about 1400 °C to about 1600 °C) for a desired duration at a desire pressure (e.g., about 40 bars to about 60 bars). In an example, the gases formed during the indirect liquefaction process can then be converted to liquids or other materials, such as ammonia or methanol. The gases formed during the indirect liquefaction process can be converted into liquids or other materials at a desired temperature (e.g., about 200 °C to about 350 °C) and at a desired pressure *(e.g*., about 10 bars to about 40 bars). The liquids or other material can then be subjected to further processing to produce hydrocarbons that can be formed into the carbon fibers, polymers that can be formed into the carbon fibers, other precursors of the carbon fibers, or the carbon fibers themselves. The hydrocarbon can include olefins (e.g., ethylene and propylene), aromatic hydrocarbons, toluene, benzene, paraxylene, or other hydrocarbons. In a particular example, the gases can be converted to olefins by a process developed by Honeywell UOP as described, for example, in U.S. Patent Publication No. 2015/0141726. In some cases, the beneficiated coal can be subjected to an indirect liquefaction process.

As previously discussed, the beneficiated coal can be processed using membranes instead of a pyrolysis process. Examples of using membranes to process coal include separating hydrogen from coal in gasification reactors using membranes. The membranes can include specialty ceramic materials, such as one or more advanced carbon-based materials *(e.g.,* one or more graphene-based materials). The membranes can facilitate the separation of hydrogen from coal at relative low temperature and/or processing of slurry-based coal, both of which can decrease costs and improve the efficiency of the process of forming the one or more resins. Examples of using membranes to process the beneficiated coal are disclosed in V. Kyriakou, et al., A protonic ceramic membrane reactor for the production of hydrogen from coal steam gasification, 553 J. Membrane Sci. 163 (2018) and Francis C. Arrillaga, et al., Coal to Hydrogen: A Novel Membrane Reactor for Direct Extraction, GCEP Energy Workshops (2004).

FIG. 4 is a flow chart of an example method 400 to form carbon fibers using one or more membranes, according to an embodiment. Except as otherwise disclosed herein, the method 400 is the same as or substantially similar to the method 100, method 200, and/or the method 300. The method 400 can include providing coal to a processing facility at block 410. The method 400 can also include beneficiating the coal via the processing facility at block 420 to remove a desired amount of water, metals, and/or other impurities from the coal. The method 400 can further includes processing at least some of the beneficiated coal with one or more membranes at the processing facility at block 430. Additionally, the method 400 can include producing pitch at block 440. Also, the method 400 can include modifying the pitch to produce carbon fibers at block 450. The method 400 is only an example. As such, one or more blocks of the method 400 can be omitted, supplemented, combined, or divided. Further, the method 400 can include one or more additional acts.

Block 430 includes processing the beneficiated coal in the presence of one or more membranes. In some cases, these membranes can serve to at least one of physically separate, chemically separate, or crack the beneficiated coal to produce products therefrom. Examples of the membranes includes advanced carbon-based materials. In an embodiment, these products can be substantially similar to the products produced by the other liquefaction processes disclosed herein, but may not use the amount of heat or pressure that the other liquefaction process can require. Thus, processing beneficiated coal with one or more membranes can produce substantially similar products to a liquefaction process but can require substantially less energy to do so. In an embodiment, the one or more membranes can comprise various pore sizes, chemical properties, physical properties, or electrical properties to isolate desirable compounds and/or produce desirable compounds from the beneficiated coal.

The beneficiated coal can be processed using other process other than or in addition to the pyrolysis processes disclosed herein, the direct liquefaction processes disclosed herein, the indirect liquefaction processes disclosed herein, or the process disclosed herein that use membranes. In an example, the beneficiated coal can be processed using an electric arc process. In an example, the beneficiate coal can be processed using a super critical solvent extraction process. Examples of super critical solvent extraction processes that can be used to process the beneficiate coal are disclosed in Jonathan J. Kolak, A Procedure for the Supercritical Fluid Extraction of Coal Samples, with Subsequent Analysis of Extracted Hydrocarbon, USGS (2006) and Ye Sun et al., Evaluation of Coal Extraction with Supercritical Carbon Dioxide/1-Methyl-2-pyrrolidone Mixed Solvent*.*

### METHODS OF FORMING THE RESIN AND OTHER BYPRODUCTS FROM RAW COAL

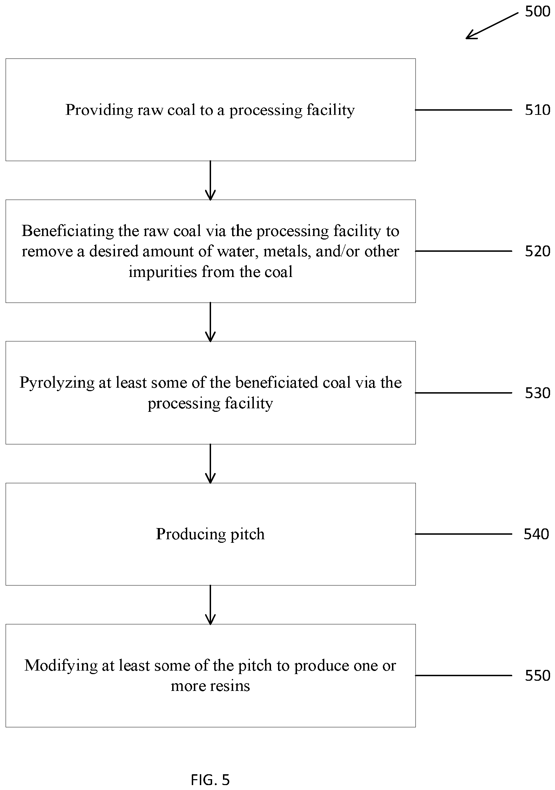

FIG. 5 is a flow chart of an example method 500 to form one or more resins, according to an embodiment. The method 500 can include one or more of providing raw coal to a processing facility at block 510; beneficiating the raw coal via the processing facility at block 520 to remove a desired amount of water, metals, and/or other impurities from the coal; pyrolyzing at least some of the beneficiated coal via the processing facility at block 530; producing pitch at block 540; and modifying at least some of the pitch to produce one or more resins at block 550. The one or more resins can include a selected amount of a remainder of one or more impurities that were not removed during the method 500. The method 500 is only an example. As such, one or more blocks of the method 500 can be omitted, supplemented, combined, or divided. Further, the method 500 can include one or more additional acts, such as producing at least one byproduct from the coal *(e.g.,* at least one of char, one or more gases, or one or more coal liquid extracts) or treating the pitch to produce one or more additional advanced carbon materials.

In some embodiments, the raw coal can be provided to a processing facility at block 510 by any method that is now known or that can be developed in the future. For example, coal is generally extracted from naturally occurring layers or veins, known as coal beds or coal seams, by mining. Coal can be extracted by surface mining, underground mining, or various other forms of mining. Typically, coal that has been extracted via mining, but has not been otherwise processed is referred to as raw coal. In some cases, the raw coal can be extracted via a surface mining process, such as a high wall mining process, strip mining process, or contour mining process. In some cases, the raw coal can be extracted via an underground mining process, such as by a longwall mining process, continuous mining process, blast mining process, retreat mining process, or room and pillar mining process.

The raw coal can be mined or extracted from a location relatively near to the processing facility. For example, the processing facility can be located at, or near a coal extraction area. However, in other cases coal can be extracted from any location and transported to the processing facility. In some cases raw coal can be provided to the processing facility as needed to produce a desired amount of advanced carbon materials. However, in some other cases, raw coal can be provided and stored at the processing facility until it is processed.

The coal provided in block 510 can be ranked or graded based on its contents and properties. Although a variety of coal classification schemes exist, a general metamorphic grade is used herein to generally describe raw coal. These grades are used generally to aid in the understanding of the present disclosure and are not intended to limit to types of coal which can be used to produce the resin and, optionally, the one or more additional advanced carbon materials as described herein. While certain classifications of coal can be preferable for use in the processes described herein, such processes are not strictly limited to the discussed classifications of coal, if any. In some embodiments, the coal utilized by the processes described herein can be lignite coal, and can have a volatile content of greater than about 45 wt. %. In some embodiments, the coal can be sub-bituminous coal, bituminous coal, and/or anthracite coal. In some embodiments, the coal can be coal extracted from the Brook Mine near Sheridan, Wyoming. It is currently believed by the inventors that the composition of the coal extracted from the Brook Mine includes several impurities at beneficial concentrations that can facilitate the formation of resin exhibiting certain mechanical, chemical, and/or electrical properties, as discussed in more detail below. In some cases, the preferred coal for use in the processes described herein can be selected by the skilled artisan. For example, the preferred coal can be selected based one at least one of one or more destructive or nondestructive chemical analyzation techniques (e.g., Raman spectroscopy, energy dispersive x-ray spectroscopy, etc.) or one or more computing techniques. In some embodiments, the coal can exhibit an initial hydrogen to carbon ratio that is greater than about 0.7, such as in ranges 0.7 to about 5.0, about 0.7 to about 0.75, about 0.725 to about 0.0775, about 0.75 to about 0.8, about 0.0775 to about 0.85, about 0.8 to about 0.9, about 0.85 to about 0.95, or about 0.9 to about 1.0.

As previously discussed, the raw coal can be provided to a processing facility at block 510 for use in the method 500. The processing facility can have the capacity to store raw coal for use as needed, or can receive raw coal as needed to produce a desired amount of the resin. As is well known in the art, coal can be provided via truck, train, or any other form of transportation. Further, the processing facility can be situated at a coal extraction site, such that coal extraction site can be considered as part of the processing facility.

As previously discussed, the raw coal can include one or more impurities therein. The one or more impurities can include, but are not limited to volatile heavy metals *(e.g.,* mercury, selenium, arsenic, and cadmium), alkali metals (e.g., sodium metals, potassium metals), heteroatoms (e.g., sulfur, oxygen, and halogens), silicon, aluminum, titanium, calcium, iron, magnesium, sodium, potassium, sulfur, strontium, barium, manganese, phosphorus, antimony, arsenic, barium, beryllium, boron, bromine, cadmium, chlorine, chromium, cobalt, copper, fluorine, lead, lithium, manganese, mercury, molybdenum, nickel, selenium, silver, strontium, thallium, tin, vanadium, zinc, and/or zirconium or oxides thereof. For example, depending on the impurity and the source of the raw coal, any of the impurities disclosed herein can form 0 weight percent ("wt. %") to about 25 wt. % of the raw coal, such as in ranges of about greater than 0 wt. % to about 0.001 wt. %, about 0.0005 wt. % to about 0.002 wt. %, about 0.001 wt. % to about 0.003 wt. %, about 0.002 wt. % to about 0.004 wt. %, about 0.003 wt. % to about 0.005 wt. %, about 0.004 wt. % to about 0.006 wt. %, about 0.005 wt. % to about 0.008 wt. %, about 0.007 wt. % to about 0.01 wt. %, about 0.009 wt. % to about 0.02 wt. %, about 0.01 wt. % to about 0.03 wt. %, about 0.02 wt. % to about 0.04 wt. %, about 0.03 wt. % to about 0.05 wt. %, about 0.04 wt. % to about 0.06 wt. %, about 0.05 wt. % to about 0.08 wt. %, about 0.07 wt. % to about 0.1 wt. %, about 0.09 wt. % to about 0.2 wt. %, about 0.1 wt. % to about 0.3 wt. %, about 0.2 wt. % to about 0.4 wt. %, about 0.3 wt. % to about 0.5 wt. %, about 0.4 wt. % to about 0.6 wt. %, about 0.5 wt. % to about 0.8 wt. %, about 0.7 wt. % to about 1 wt. %, about 0.9 wt. % to about 2 wt. %, about 1 wt. % to about 4 wt. %, about 3 wt. % to about 6 wt. %, about 5 wt. % to about 8 wt. %, about 7 wt. % to about 10 wt. %, about 9 wt. % to about 15 wt. %, or about 10 wt. % to about 25 wt. %.

At block 520, the raw coal can be beneficiated to remove at least some of at least one of the impurities that are present in the raw coal to form beneficiated coal (also known as upgraded coal), according to an embodiment. For example, the raw coal can be beneficiated to remove at least one water, heavy metals, volatile compounds, alkali metals, or heteroatoms from the raw coal, thereby producing the beneficiated coal. In an embodiment, the raw coal can be beneficiated to remove a significant portion of at least one of the impurities.

The beneficiation process can include heating the raw coal to one or more desired temperatures. The one or more desired temperature can be about 100 °C to about 500 °C, such as in ranges of about 100 °C to about 290 °C, 100 °C to about 150 °C, about 125 °C to about 200 °C, or about 150 °C to about 290 °C. The temperature that the raw coal is heated to can be selected to selectively remove at least some of at least one of the impurities that are present in the raw coal. For example, the raw coal can be heated to a temperature of about 100 °C to about 150 °C to remove moisture from the raw coal and about 150 °C to about 290 °C to remove volatile metals from the raw coal. In some cases, the beneficiation process can comprise heating the raw coal to a first desired temperature. Heating the raw coal to the first desired temperature can remove one or more first impurities. In some embodiments, beneficiation can then include heating the raw coal to a second, higher desired temperature. Heating the raw coal to the second desired temperature can remove one or more second impurities.

The beneficiation process can include heating the raw coal to the desired temperature for a desired duration. The desired duration can be about 1 second to several days, such as in ranges of about 1 second to about 1 minute, about 30 seconds to about 30 minutes, about 1 minute to about 1 hour, about 30 minutes to about 3 hours, about 1 hours to about 5 hours, about 3 hours to about 10 hours, about 7 hours to about 18 hours, about 12 hours to about 1 day, or about 18 hours to about 3 days. Typically, increasing the duration that the raw coal is heated to the desired temperature can increase the amount of the one or more impurities are removed from the raw coal. However, the raw coal can exhibit a maximum duration where heating the raw coal for periods of time longer than the maximum duration will have little or no effect on the amount of the one or more impurities that are removed from the raw coal. In some cases, the beneficiation process can comprise heating the raw coal to a first desired temperature for a first duration followed by heating the raw coal to a second, higher desired temperature for a second duration. The first and second durations can be the same or different.

In an embodiment, the beneficiation process can include heating the raw coal in an atmosphere comprising a halogen gas which can facilitate removal of one or more of the impurities from the raw coal and prevent oxidation or other reactions with the raw coal. In some embodiments, the coal can be beneficiated by heating the raw coal in an atmosphere including hydrogen which can increase the hydrogen to carbon ratio. In such an embodiment, the hydrogen can be provided from an outside hydrogen source or from hydrogen that was collected in another stage of the method 500. In some embodiments, the coal can be beneficiated by heating the raw coal in an atmosphere that is substantially hydrogen free. In such an embodiment, the substantially hydrogen free atmosphere can decrease the hydrogen to carbon ratio.

In some other embodiments, the coal can be beneficiated by heating the coal to a desired temperature in the presence of one or more catalyst compounds. In some cases, beneficiating the coal can comprise pyrolyzing the coal, for example in the presence of a catalyst. In some embodiments, the raw coal can be beneficiated at or near atmospheric pressure *(e.g.,* 0.8 to about 1.2 atmospheres), though the raw coal can be beneficiated at higher or lower pressures.

In some embodiments, beneficiation can include subjecting the raw coal to a WRITECoal beneficiation process. The WRITECoal beneficiation process is discussed in U.S. Pat. No. 9,181,509. In some cases, the coal can be beneficiated by the BenePlus System, as developed and licensed by LP Amina, and as described, for example, in U.S. Patent Publication No. 2017/0198221.

The beneficiated coal can comprise a significantly reduced amount (e.g., at least 25 wt. %e,at least 50 wt. %, at least 75 wt. %, at least 90 wt. %, at least 95 wt. %, or at least 99 wt. %) of at least one of mercury, cadmium, other heavy metals, water, any of the other impurities disclosed herein, or any other impurity that can be present in the raw coal. The amount of the impurities that are left in the beneficiated coal can depend on the temperature that the raw coal was heated, the duration that the raw coal was heated, the atmosphere that the raw coal was exposed to during the beneficiation process, the presence of catalysts, etc. For example, beneficiating the coal can reduce the amount of mercury in the coal by about at least about 70 wt. %, 75 wt. %, 80 wt. %, 85 wt. %, 90 wt. %, or 92 wt. % or more. In some cases, beneficiating the coal can reduce the water or moisture content of the coal to less than about 5 wt. %, 4 wt. %, 3 wt. %, 2 wt. %, or 1.5 wt. % or lower. In some cases, beneficiating the coal can remove one or more of hydrogen, sulfur, oxygen, arsenic, selenium, cadmium, or volatile matter from the coal. The amount of one or more of these elements in the coal can be reduced by about 25 wt. % to about 90 wt. %.

However, as previously discussed, it can be desirable for a desired amount of one or more impurities to remain in the beneficiated coal after being subjected to a beneficiation process. For example, the beneficiation process can remove a desired amount of impurities such that a predetermined amount of mercury, cadmium, selenium, alkali metals, heterogeneous elements, and/or another element can selectively remain in the beneficiated coal after processing. In some cases, the desired amount of impurity that can remain in the beneficiated coal can be useful in the subsequent formation of the resin or, optionally, the one or more additional advanced carbon materials. In some cases, the desired amount of the impurity that can remain in the beneficiated coal can be incorporated into the resin and, optionally, the one or more advanced carbon materials. For example, where the advanced carbon material comprises synthetic graphene, a desired amount of cadmium can remain in the beneficiated coal and can be incorporated into the synthetic graphene to thereby improve the electrical, mechanical, or chemical properties thereof.

In an embodiment, the beneficiation process can be configured to maintain at least some of any of the impurities disclosed herein at any of the concentrations disclosed herein in the beneficiated coal after block 520 (but before block 530).

In an embodiment, the beneficiation process can be configured to decrease the hydrogen to carbon ratio of the coal. For example, after the beneficiation process, the hydrogen to carbon ratio of beneficiated coal can less than about 0.8, such as in ranges of about 0.6 to about 0.7, about 0.65 to about 0.75, or about 0.7 to about 0.8.

In some embodiments, beneficiating the coal during act 520 can produce one or more byproducts, such as one or more byproducts that can be captured and used in later processing steps, that can be valuable in and of themselves, or that can be subjected to further processing or use in the method 500. For example, beneficiating the coal can produce or separate gases or coal liquid extracts from the raw coal. These gases and/or coal liquid extracts can be captured or separated during processing. For example, beneficiating the coal at block 520 can produce at least one of H₂, CO₂, CO, CH₄, C₂H₄, C₃H₆, or other hydrocarbon gases, which can be captured and subsequently utilized in block 530 or in other process steps. In some cases, beneficiating the coal can result in coal liquid extracts (e.g., toluene or benzene) which can be captured for subsequent use or processing. In some cases, the impurities removed from the coal by the beneficiation process at block 520 can be captured for subsequent use. For example, water removed from the coal by the beneficiation process can be capture and utilized in subsequent process steps. In some embodiments, beneficiating the coal can also produce a solid material known as ash or char. In some cases, this char can be subjected to further processing to form activated carbon.

At block 530 the beneficiated coal can be processed via the processing facility. In some embodiments, processing the beneficiated coal can include subjecting the beneficiated coal to a liquid extraction process, such as a pyrolysis process *(e.g.,* a high temperature pyrolysis process or a mild temperature pyrolysis process). It is noted that other liquefaction processes can be used instead of or in conjunction with the pyrolysis process, such as using a direct liquefaction process (discussed in more detail with regard to FIG. 2), an indirect liquefaction process (discussed in more detail with regard to FIG. 3), membranes (e.g., discussed in more detail with regard to FIG. 4), an electric arc process, a super critical solvent extraction process, or an electromagnetic heating process. The liquid extraction process can convert the beneficiated coal into at least one of pitch, one or more gases, one or more coal liquid extracts, or char.

In an embodiment, the liquid extraction processes of block 530 can comprise pyrolyzing the beneficiated coal via the processing facility. Pyrolyzing the beneficiated coal to form one or more resins can include heating the beneficiated coal to a desired temperature for a desired duration, with or without elevating the pressure applied to the beneficiated coal. At the elevated temperatures, some of the organic structures within the beneficiated coal begin to breakdown forming lower molecular weight pyrolytic fragments. Some of the lower molecular weight pyrolytic fragments can escape the beneficiated coal as light gases *(e.g.,* hydrogen, methane, carbon dioxide, etc.). These light gases can be captured and/or reused as discussed in more detail below. However, some of the lower molecular weight pyrolytic fragments can recombine or depolymerize to form small ring aromatic structures (e.g., single ring aromatic structure). The small ring aromatic structure can undergo condensation reactions wherein the small aromatic ring structure join together to form larger ring aromatic structures (e.g., polyaromatic hydrocarbons). Examples of the condensation reactions includes at least one of ring condensation, ring fusion, dehydrogenation, or other condensation reactions that grow the small ring aromatic structure. The larger ring aromatic structures can comprise isotropic pitches, isotropic resins, other products that include aligned polyaromatic layers, liquid crystalline structure (e.g. anisotropic pitches or anisotropic resins), mesophase pitches, or mesophase resins.

In an example, pyrolyzing the beneficiated coal can comprise a high temperature pyrolysis process that includes heating the beneficiated coal to a temperature greater than about 1000 °C at atmospheric process. The high temperature pyrolysis process can form benzene compounds, phenol compounds, high value oil, or other compounds that are useful in the formation of resins. In an example, pyrolyzing the beneficiated coal can comprise a mild temperature pyrolysis process that includes heating the beneficiated coal to a temperature of about 400 °C to about 650 °C at atmospheric pressure. The mild temperature pyrolysis process is likely to form coke than the high temperature pyrolysis process which can facilitate the formation of the resins disclosed herein. In some cases, the coal can be heated at high pressure *(e.g.,* a pressure greater than about 1 atmosphere) and/or in the presence of a solvent. For example, the beneficiated coal can be pyrolyzed in the presence of a CO₂ solvent which can be held in a supercritical state. In some cases, the beneficiated coal can be pyrolyzed in a hydrogen atmosphere (e.g., hydrogen provided from an outside hydrogen source or hydrogen collected during the method 500) to increase the hydrogen to carbon ratio of the beneficiated coal or pyrolyzed in a hydrogen free atmosphere to decrease the hydrogen to carbon ratio of the beneficiated coal. In an embodiment, the beneficiated coal can be pyrolyzed by the MuSCL System developed by TerraPower as described, for example, in U.S. Patent No. 10,144, 874.

In some embodiments, the pyrolysis process can comprise exposing the beneficiated coal to electromagnetic radiation at a desired intensity and for a desired duration. For example, block 530 can comprise exposing the beneficiated coal to microwave and/or radiofrequency (RF) radiation for a desired duration as part of the pyrolysis process. In some cases, this pyrolysis process can result in the bulk of the beneficiated coal remaining below pyrolytic temperatures, while individual particles of coal can be subjected to temperatures greater than about 1200 F. In some cases, this pyrolysis process can also comprise methane activation and/or methylation of at least some of the carbon comprising the beneficiated coal. In some embodiments, the beneficiated coal can be pyrolyzed by the Wave Liquefaction process developed by H Quest Vanguard, Inc. Additional examples of processes for processing coal by exposing the beneficiated coal to electromagnetic radiation are disclosed in U.S. Patent No. 6,512,216 and U.S. Patent Application Publication No. 2017/0080399.

Block 530 can be configured to prevent or selectively maintain selected quantities of the one or more impurities in the pyrolyzed coal. For example, after block 530, the pitch can include any of the impurities disclosed herein at any of the concentrations disclosed herein.

In an embodiment, after block 530, the pyrolyzed can exhibit a hydrogen to carbon ratio of less than about 0.8, such as in ranges as less than about 0.1, less than about 0.2, about 0.1 to about 0.2, about 0.15 to about 0.25, about 0.2 to about 0.4, about 0.3 to about 0.5, about 0.4 to about 0.6, or about 0.5 to about 0.7.

At block 540, pitch and, optionally, one or more byproducts (e.g., at least one of one or more gases, one or more coal liquid extracts, or char) are processed (e.g., extracted from the pyrolyzed coal) via the processing facility. In an embodiment, block 540 can represent the result of at least one of block 520 or block 530, rather than a separate action or process step. In an embodiment, block 540 can be performed substantially simultaneously with at least one of block 520 or block 530. In an embodiment, block 540 can be performed after at least one of block 520 or block 530.

In an embodiment, block 540 can include adding one or more additives to the beneficiated coal. For example, one or more other gases or liquids can be used during block 540 to add one or more additives to the beneficiated coal. Examples of gases or liquids that can be used during block 540 include hydrogen containing gases, natural gases, CO, petroleum products, one or more materials or compounds that are produced during at least one of block 520 or block 530, or one or more materials or compounds that can be produced by or captured during previous iterations of method 500.

As previously discussed, the raw coal can include one or more impurities therein and blocks 520 and 530 can be configured to not remove at least some of the impurities such that the beneficiated or pyrolyzed coal includes at least some of the impurities. The presence of the one or more impurities in the beneficiated or pyrolyzed coal can make adding one or more additives to the beneficiated or pyrolyzed coal unnecessary or can reduce the amount of additives that are added to the beneficiated or pyrolyzed coal. As such, the presence of the one or more impurities in the beneficiated or pyrolyzed coal can make the method 500 more efficient than methods of forming resins from a non-coal source since the beneficiated or pyrolyzed coal can have less additives added thereto than the non-coal source.

In some embodiments, pitch can be produced via the processing facility at block 540. As used herein, pitch, also known as coal pitch, coal tar, or coal tar pitch, can refer to a mixture of one or more typically viscoelastic polymers as will be well understood by the skilled artisan. In some embodiments, the pitch produced at block 540 can be a direct result of processing the beneficiated coal at step 530. The pitch produced at block 540 can comprise one or more high molecular weight polymers. In some embodiments, the pitch can have a melting point of greater than about 650 °F. In some embodiments, the pitch can have a melting point that is high enough that some of the pitch (e.g., portions of the pitch not used to form the resin) can be used in a carbon fiber spinning process without the need for a plasticizer.

In an embodiment, the pitch can comprise aromatic hydrocarbons, for example polycyclic aromatic hydrocarbons. In some cases, the pitch can comprise at least about 50 wt. % polycyclic aromatic hydrocarbons, at least about 60 wt. %, 70 wt. %, 80 wt. %, 90 wt. %, 95 wt. %, or 99 wt. % or greater of polycyclic aromatic hydrocarbons. In an embodiment, the pitch can comprise less than about 0.1 wt. % ash or other solid material, less than about 0.05 wt. % ash or solid material, or less than about 0.01 wt. % ash or solid material. In some cases, the pitch can have a flash point greater than about 230 °F, greater than about 250 °F, greater than about 300 °F, or in ranges of about 230 °F to about 250 °F, about 240 °F to about 275 °F, about 250 °F to about 300 °F, about 275 °F to about 350 °F, or about 300 °F to about 400 °F. In some cases, the pitch can have an API gravity of less than about 4, less than about 3, or less than about 2, or less than about 1.5. In some embodiments, the pitch produced by the method 500 is not coke pitch. That is, in some cases, the pitch produced at block 540 is not produced from coke or a coke-based material. In some embodiments, coke is not produced at any point during the method 500.

In some embodiments, the pitch can include any of the impurities disclosed herein at any of the concentrations disclosed herein.

In some embodiments, the pitch can have a hydrogen to carbon ratio of about of less than about 0.8, such as in ranges as less than about 0.1, less than about 0.2, about 0.1 to about 0.2, about 0.15 to about 0.25, about 0.2 to about 0.4, about 0.3 to about 0.5, about 0.4 to about 0.6, or about 0.5 to about 0.7.

In an embodiment, the pitch can be relatively free of one or more selected impurities, such as water, one or more non-carbon atoms (e.g., one or more of mercury, selenium, cadmium, arsenic, alkali metals, oxygen, halogens, sulfur or nitrogen) or material such as coal ash or char. For example, the pitch can comprise less than about 0.2 wt. % water, less than about 0.1 wt. %, less than about 0.05 wt. %, or less than about 0.01 wt. % water or lower. In an embodiment, the pitch can include one or more selected impurities in the concentrations disclosed above.

In an embodiment, the method 500 can also include producing one or more byproducts during block 540. Examples of the byproducts that can be formed during the block 540 are disclosed in [cited the other related applications]. For example, the byproducts produced during block 540 can include at least one of one or more gases, one or more coal liquid extract, or char. It is noted that the one or more byproducts can also be produced during at least one of block 520 or block 530 instead of or in addition to block 540.

The coal liquid extracts can refer to any material that is extracted or produced from raw coal or beneficiated coal that is liquid at or near normal temperature and pressure (about 68 F and 1 atmosphere of pressure). The one or more coal liquid extracts can comprise one or more liquid hydrocarbons. For example, coal liquid extracts can comprise one or more of benzene, toluene, alkanes or paraffins, alkenes, C2 compounds, C3 compounds, C4 compounds, T compounds, halogen compounds, phenols, or other saturated or unsaturated hydrocarbons.

In some embodiments, the coal liquid extracts can include any of the impurities disclosed herein at any of the concentrations disclosed herein.

Char, also known as ash, can refer to any solid material which remains after gases, coal extract liquids, and/or pitch have been removed from raw coal. Char can be produced during at least one of block 520, block 530, or block 540. In an example, the char can comprise a solid high surface area carbonaceous material. In an example, the char can have a relatively low hydrogen to carbon ratio, such as a hydrogen to carbon ratio that is lower than the hydrogen to carbon ratio of pitch produced at block 540. In some cases, char can have a hydrogen to carbon ratio of from about 0.05 to about 0.65. In an example, char can additionally comprise at least some pitch material, which can be referred to herein as intrinsic binder impregnation. In some cases, any residual pitch or other gaseous or liquid materials can be removed from the char prior to any subsequent processing of the char.

In some embodiments, the char can include any of the impurities disclosed herein at any of the concentrations disclosed herein. In some embodiments, the char can have a hydrogen to carbon ratio of less than about 0.7, such as in ranges as less than about 0.1, less than about 0.2, about 0.1 to about 0.2, about 0.15 to about 0.25, about 0.2 to about 0.4, about 0.3 to about 0.5, about 0.4 to about 0.6, or about 0.5 to about 0.7.

The gases can comprise hydrogen and/or carbon. For example, the gases can include H₂, CO₂, CO, CH₄, C₂H₄, C₃H₆, and/or other hydrocarbon gases. The gases can also include sulfur. In some cases, these gases can be at least one of captured, otherwise contained, or used during the processes described herein. In some embodiments at least 50%, at least 75%, at least 90%, 95%, or 99% of any gaseous or volatile byproducts of the method 500 can be captured. The gases captured during certain process steps can be used in subsequent process steps as described herein. In some cases, gases produced by and a captured as part of the processes described herein can be utilized by these same or subsequent processes in order to increase the efficiency and/or cost effectiveness of said processes. In some cases, the capture and reuse of byproducts can improve the efficiency and/or lower the cost of the method 500.

In an embodiment, the method 500 can include reacting captured hydrogen gas (H₂) with captured carbon dioxide (CO₂) (*i*.*e.*, syngas) to form methane or another suitable gas or liquid thereby reducing the carbon footprint of the method 500. The captured hydrogen and carbon dioxide gas can be processed chemically (*e*.*g*., catalytically) to form monomeric compounds *(e.g.,* olefins such as ethylene and/or propylene) that can then be polymerized into higher molecular weight resin compounds. In an example, reacting the hydrogen with the carbon dioxide can reduce the amount of carbon dioxide produced by the method 500 by about 1% to about 99%, such as by about 1% to about 25%, about 20% to about 40%, or about 25% to about 50%.

In an embodiment, the pitch and the byproducts are all produced at block 540. In an embodiment, at least one of the pitch and at least one of the byproducts can be produced at separate times or separate processing steps from one another. In an embodiment, the pitch and at least one of the byproducts are produced together by the process 500 and, in particular, during block 540. In such an embodiment, the method 500 can include separating these products before any further processing of each individual product can occur. For example, pitch and coal liquid extracts can be simultaneously produced as a result of block 530 and can need to be separated from one another, by any process now know or which can be developed in the future, before further processing of either pitch or coal liquid extracts occurs.

In an embodiment, the pitch produced during block 540 are not subjected to further processing or refinement to alter the chemical composition of the pitch before block 550. In an embodiment, the pitch produced during block 540 can be subjected to one or more processes which can alter the chemical composition thereof prior to block 550. In an example, undesired impurities that remain in the pitch after block 540 can be removed therefrom prior to block 550. In such an example, the undesirable impurities can include impurities that could not be removed during block 520 and block 530 or excessive amounts of at least one impurity that is selected to be present in the resin. In an example, the pitch can be subjected to one or more processes to increase or decrease the hydrogen to carbon ratio of the pitch. In an example, the pitch can be subjected to one or more processes to produce mesophase pitch or otherwise alter the composition or properties of the pitch. In an embodiment, the one or more byproducts can also be used to form the resin during block 550. In such an embodiment, the one or more byproducts can or may not be subjected to further processing or refinement to alter the chemical composition thereof before block 550 using any of the processes disclosed herein or any other suitable process.

At block 550, at least the pitch produced at block 540 can be treated via the processing facility to produce at least one of the resins disclosed herein. In an embodiment, block 550 can include adding one or more additives to the pitch or the resin if the pitch has already been treated to form the resin. In an embodiment, the additives added to the pitch or the resin can include one or more photo activators, such as one or more ultraviolet light activates. In an embodiment, the additives added to the pitch or the resin can include one or more thermal activators. In an embodiment, the additives can include any suitable catalyst.

In an embodiment, block 550 can include treating the coal liquid extracts via the processing facility to form at least one of the resins disclosed herein.

The resin formed during block 550 can include polyurethane resins, cyanate ester resins, epoxy resins, methacrylate resins, polyester resins, polyacrylonitrile (e.g., formed via the Sohio Acrylonitrile Process), or any other suitable resin.

The resins formed during block 550 can include one or more impurities therein. The impurities that are present in the resins can include any of the impurities disclosed herein at any of the concentrations disclosed herein. For example, the concentration of the one or more impurities that are present in the resin can be selected to be substantially the same as *(e.g.,* + 10 wt. %), less than, or greater than *(e.g.,* the additives include the impurity) the concentration of the one or more impurities that were present in the raw coal. The properties of the resins can depend, at least in part, on the one or more impurities that are present in the resin. For example, the impurities that are present in the one or more resins can affect at least one of the elasticity (Young's modulus), tensile strength, or failure mechanism of the resin. In an embodiment, the one or more impurities can include at least one impurity that was initially present in the raw coal thereby negating the need to add the at least one impurity into at least one of the raw coal, the beneficiated coal, the pitch, or the byproducts. In an embodiment, the one or more impurities can include at least one impurity that was added to at least one of the raw coal, the beneficiated coal, the pitch, or the byproducts.

In an embodiment, the hydrogen to carbon ratio of the resin can be about 0.7 to about 1.0, such as in ranges of about 0.7 to about 0.75, about 0.725 to about 0.0775, about 0.75 to about 0.8, about 0.0775 to about 0.85, about 0.8 to about 0.9, about 0.85 to about 0.95, or about 0.9 to about 1.0.

In an embodiment, the method 500 can include recycling carbon material (e.g., resin, pitch, etc.) exhibiting a hydrogen to carbon ratio that is greater than the desired hydrogen to carbon ratio, such as greater than about 0.2, greater than about 0.3, greater than about 0.4, greater than about 0.5, greater than about 0.6, or greater than about 0.7. For example, recycling the carbon material exhibiting a high hydrogen to carbon ratio can include adding the carbon material to the raw coal, the beneficiated coal, the pyrolyzed coal, the pitch before the pitch is processed, etc. Recycling the carbon material can increase the amount of resin or other advanced carbon material that is produced during the method 500.

As previously discussed, the beneficiated coal can be processed using a direct liquefaction process other than a pyrolysis process. The direct liquefaction process can include producing resins from coal under hydrogenation conditions to produce a naphtha which can further be "steam cracked" *(e.g.,* converted into so-called "naphtha cracker") to produce light olefin products (e.g., ethylene and/or) propylene. One example of a direct liquefaction process includes the H-Coal process developed in the Catlettsburg refinery and by Shenhua of China to produce liquid transportation fuels, chemical intermediates, and naphtha.

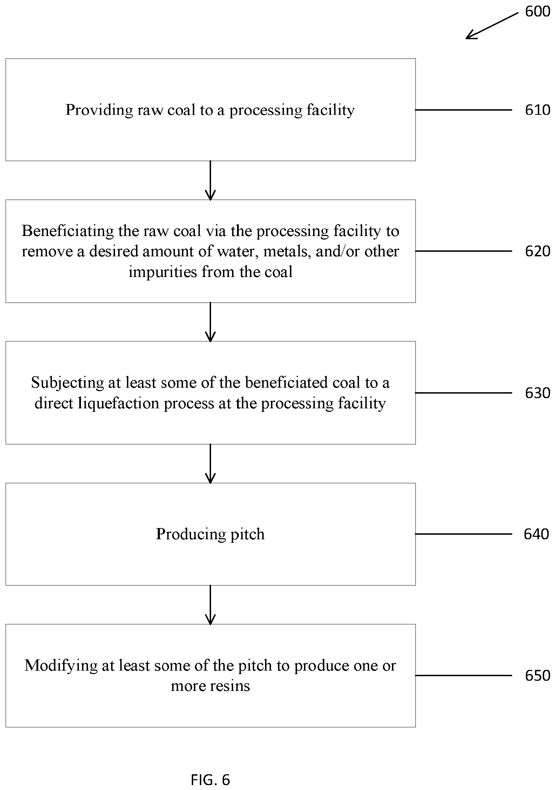

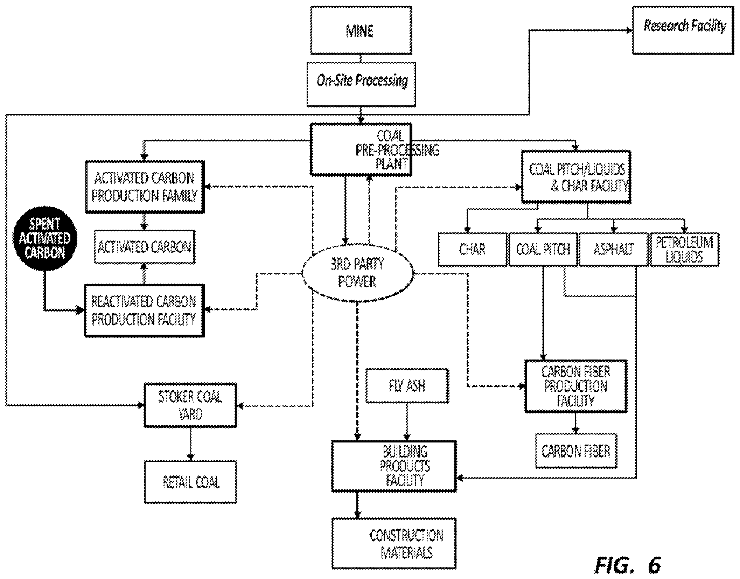

FIG. 6 is a flow chart of an example method 600 to form one or more resins using a direct liquefaction process, according to an embodiment. Except as otherwise disclosed herein, the method 600 is the same or substantially similar to the method 500. The method 600 can include providing coal to a processing facility at block 610. The method 600 can also include beneficiating the coal via the processing facility at block 620 to remove a desired amount of water, metals, and/or other impurities from the coal. The method 600 can further includes subjecting at least some of the beneficiated coal to a direct liquefaction process at the processing facility at block 630. Additionally, the method 600 can include producing pitch at block 640. Also, the method 600 can include modifying the pitch to produce one or more resins at block 650. The method 600 is only an example, As such, one or more blocks of the method 600 can be omitted, supplemented, combined, or divided. Further, the method 600 can include one or more additional acts.

Block 630 includes processing the beneficiated coal using a direct liquefaction process. The direct liquefaction process can include heating the beneficiated coal above a desired temperature for a duration thereby converting the beneficiated coal into a liquid. For example, the desired temperature can be about 400 °C to about 500 °C. In some cases, the beneficiated coal can be heated in the presence of one or more catalysts and/or at an elevated pressure (e.g., about 10 bars to about 1000 bars). In some cases, the beneficiated coal can be heated in an atmosphere comprising H. In some cases, the direct liquefaction process can be a hydrogenation or hydro-cracking process. In such cases, the direct liquefaction process can be performed in a hydrogen atmosphere. In some cases, a solvent can be added to the beneficiated coal during the direct liquefaction process. In some cases, the beneficiated coal can be subjected to a direct liquefaction process developed by Axens, as described for example, in U.S. Patent Publication No. 2017/0313886.

As previously discussed, the beneficiated coal can be processed using an indirect liquefaction process other than a pyrolysis process, The indirect liquefaction process can include producing resins from the raw coal in a high temperature gasification process to form so-called syngas (e.g., a mixture of hydrogen gas and at least one of carbon monoxide or carbon dioxide). The syngas can be processed chemically to form monomeric compounds that can then be polymerized into higher molecular weight resin compounds. For example, syngas can be converted into methanol which can be catalytically converted into light olefins (e.g., ethylene and/or propylene). Ethylene and/or propylene can be further polymerized into polyethylene and polypropylene resins, respectively. The light olefins formed during the indirect liquefaction process and/or the other process disclosed herein can also be precursors to other polymeric compounds. For example, the ammoxidation of propylene results in the formation of acrylonitrile monomers which can then be converted into polyacrylonitrile.

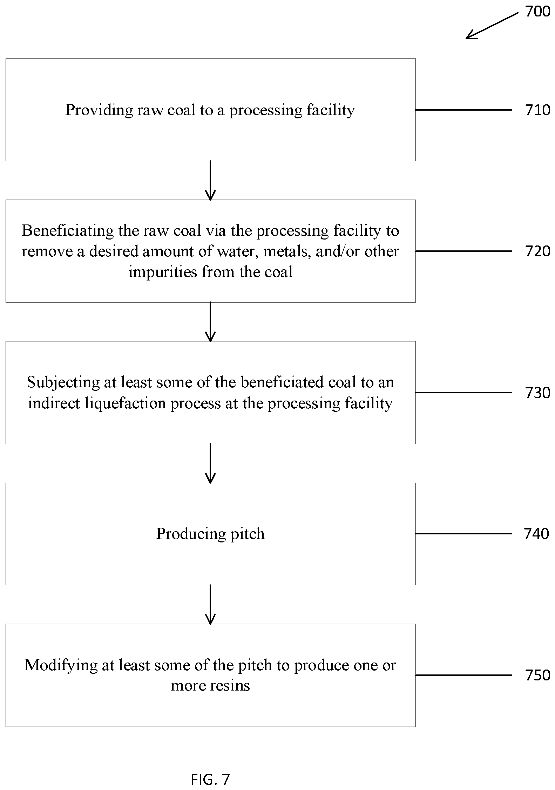

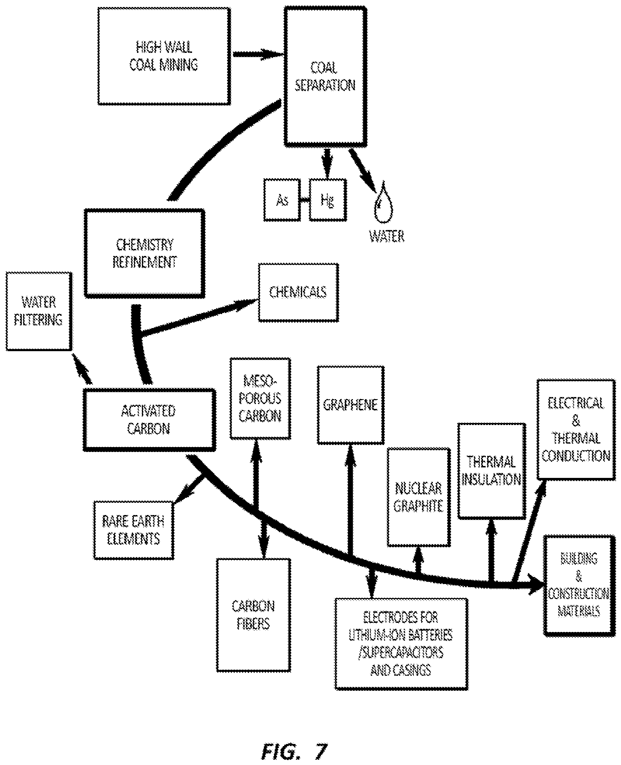

FIG. 7 is a flow chart of an example method 700 to form one or more resins using an indirect liquefaction process, according to an embodiment. Except as otherwise disclosed herein, the method 700 is the same or substantially similar to the method 500 and/or method 600. The method 700 can include providing coal to a processing facility at block 710. The method 700 can also include beneficiating the coal via the processing facility at block 720 to remove a desired amount of water, metals, and/or other impurities from the coal. The method 700 can further includes subjecting at least some of the beneficiated coal to an indirect liquefaction process at the processing facility at block 730. Additionally, the method 700 can include producing syngas or another coal-derived element *(e.g*., char, pitch, etc.) at block 740. Also, the method 700 can include modifying the pitch to produce one or more resins at block 750. The method 700 is only an example, As such, one or more blocks of the method 700 can be omitted, supplemented, combined, or divided. Further, the method 700 can include one or more additional acts.

Block 730 includes processing the beneficiated coal using an indirect liquefaction process. The indirect liquefaction process can include converting the beneficiated coal to a gas or gases and then converting the gas or gases into one or more liquids. The one or more gases can include, but are not limited to, syngas (e.g., a mixture of H and CO gas). In an example, the indirect liquefaction process can include heating the beneficiated coal to a desired temperature *(e*.*g*., 1400 °C to about 1600 °C) for a desired duration at a desire pressure *(e*.*g*., about 40 bars to about 60 bars. In an example, the gases formed during the indirect liquefaction process can then be converted to liquids or other materials, such as ammonia or methanol. The gases formed during the indirect liquefaction process can be converted into liquids or other materials at a desired temperature (e.g., about 200 °C to about 350 °C) and at a desired pressure (e.g., about 10 bars to about 40 bars). The liquids or other material can then be subjected to further processing to produce hydrocarbons that can be formed into the resins, polymers that can be formed into the resins, other precursors of the resins, or the resins themselves. The hydrocarbon can include olefins (e.g., ethylene and propylene), aromatic hydrocarbons, toluene, benzene, paraxylene, or other hydrocarbons. In a particular example, the gases can be converted to olefins by a process developed by Honeywell UOP, as described, for example, in U.S. Patent Publication No. 2015/0141726.

As previously discussed, the beneficiated coal can be processed using membranes instead of a pyrolysis process. Examples of using membranes to process coal include separating hydrogen from coal in gasification reactors using membranes. The membranes can include specialty ceramic materials, such as one or more advanced carbon-based materials (e.g., one or more graphene-based materials). The membranes can facilitate the separation of hydrogen from coal at relative low temperature and/or processing of slurry-based coal, both of which can decrease costs and improve the efficiency of the process of forming the one or more resins. Examples of using membranes to process the beneficiated coal are disclosed in V. Kyriakou, et al., A protonic ceramic membrane reactor for the production of hydrogen from coal steam gasification, 553 J. Membrane Sci. 163 (2018) and Francis C. Arrillaga, et al., Coal to Hydrogen: A Novel Membrane Reactor for Direct Extraction, GCEP Energy Workshops (2004).

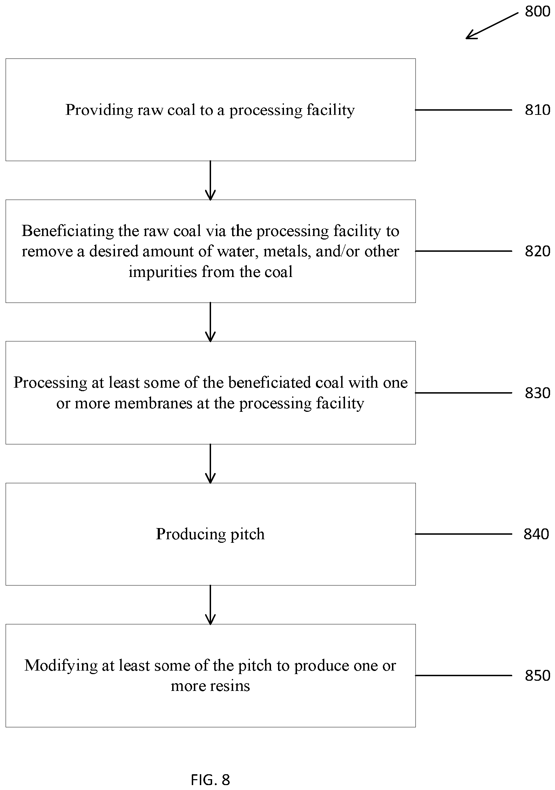

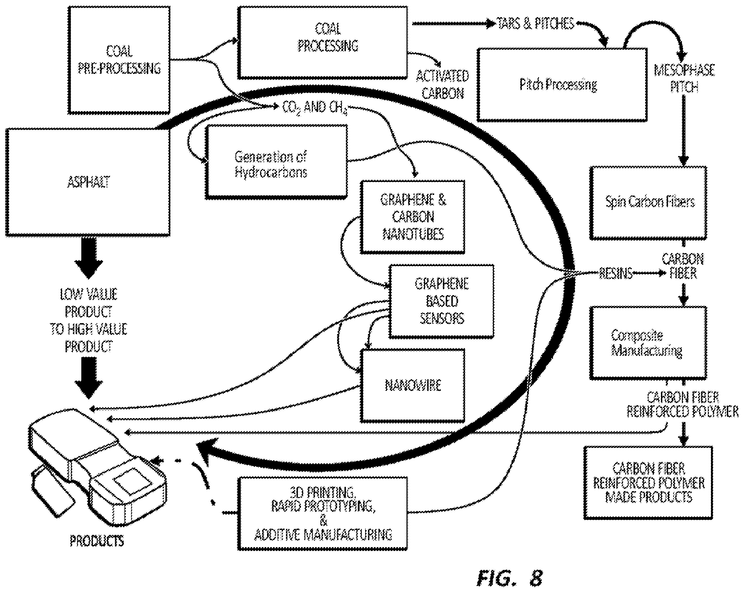

In an FIG. 8 is a flow chart of an example method 800 to form one or more resins using one or more membranes, according to an embodiment. Except as otherwise disclosed herein, the method 800 is the same as or substantially similar to the method 500, method 600, and/or the method 700. The method 800 can include providing coal to a processing facility at block 810. The method 800 can also include beneficiating the coal via the processing facility at block 820 to remove a desired amount of water, metals, and/or other impurities from the coal. The method 800 can further includes processing at least some of the beneficiated coal with one or more membranes at the processing facility at block 830. Additionally, the method 800 can include producing pitch at block 840. Also, the method 800 can include modifying any other carbon-based material formed during the method 800 to produce one or more resins at block 850. The method 800 is only an example. As such, one or more blocks of the method 800 can be omitted, supplemented, combined, or divided. Further, the method 800 can include one or more additional acts.

Block 830 includes processing the beneficiated coal in the presence of one or more membranes. In some cases, these membranes can serve to at least one of physically separate, chemically separate, or crack the beneficiated coal to produce products therefrom, Examples of the membranes includes advanced carbon-based materials. In an embodiment, these products can be substantially similar to the products produced by the other liquefaction processes disclosed herein, but may not use the amount of heat or pressure that the other liquefaction process can require. Thus, processing beneficiated coal with one or more membranes can produce substantially similar products to a liquefaction process but can require substantially less energy to do so. In an embodiment, the one or more membranes can comprise various pore sizes, chemical properties, physical properties, or electrical properties to isolate desirable compounds and/or produce desirable compounds from the beneficiated coal.

The beneficiated coal can be processed using other process other than or in addition to the pyrolysis processes disclosed herein, the direct liquefaction processes disclosed herein, the indirect liquefaction processes disclosed herein, or the process disclosed herein that use membranes. In an example, the beneficiated coal can be processed using an electric arc process. In an example, the beneficiate coal can be processed using a super critical solvent extraction process. Examples of super critical solvent extraction processes that can be used to process the beneficiate coal are disclosed in Jonathan J. Kolak, A Procedure for the Supercritical Fluid Extraction of Coal Samples, with Subsequent Analysis of Extracted Hydrocarbon, USGS (2006) and Ye Sun et al., Evaluation of Coal Extraction with Supercritical Carbon Dioxide/1-Methyl-2-pyrrolidone Mixed Solvent*.*

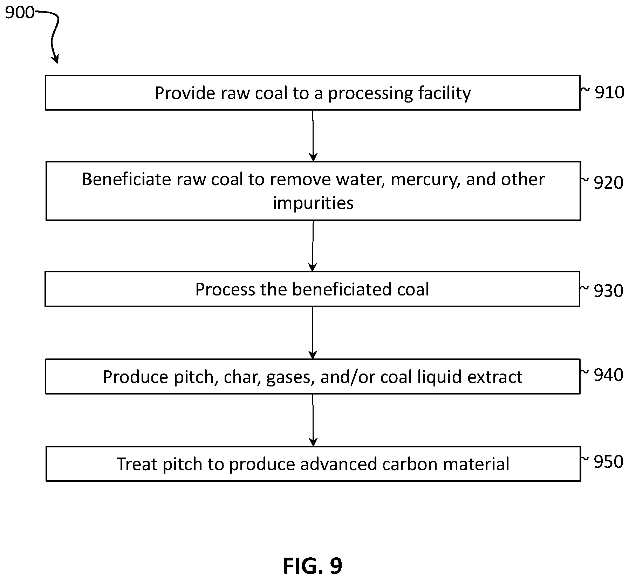

Coal can be ranked or graded based on its contents and properties. Although a variety of coal classification schemes exist, a general metamorphic grade is used herein to generally describe raw coal. These grades are used generally to aid in the understanding of the present disclosure and are not intended to limit to types of coal which can be used to produce advanced carbon materials as described herein. While certain classifications of coal can be preferable for use in the processes described herein, such processes are not strictly limited to the discussed classifications of coal, if any. In some embodiments, the coal utilized by the processes described herein can be lignite coal, and can have a volatile content of greater than about 45 wt. %. In some embodiments, the coal can be sub-bituminous coal, bituminous coal, and/or anthracite coal. In some embodiments, the coal can be coal extracted from the Brook Mine near Sheridan, Wyoming. In some cases, the preferred coal for use in the processes described herein can be selected by the skilled artisan. According to some embodiments, and as illustrated in FIG. 9, graphene or another advanced carbon material can be produced from coal by a method or process 900 including:
providing coal to a processing facility at block 910;
beneficiating the coal via the processing facility at block 920 to remove a desired amount of water, metals, and/or other impurities from the coal;
processing at least some of the beneficiated coal via the processing facility at block 930;
producing pitch, char, gases, and/or coal liquid extract at block 940; and
treating the pitch via the processing facility at block 950 to produce the graphene or other advanced carbon material.

Although the method 900 describes a process flow for producing a single type of advanced carbon material via a processing facility, the method 900 can be used to produce more than one type of advanced carbon material via the processing facility. For example, the method 900 can be utilized to produce an amount of a first advanced carbon material and subsequently used to produce an amount of a second, different advanced carbon material. In some cases however, the processing facility can be able to produce two or more different types of advanced carbon materials via parallel processing utilizing the method 900. Further, while producing pitch, char, and/or coal liquid extract is described as a separate block 940, the pitch, char, and/or coal liquid extract can be produced as a result of blocks 920 and/or 930 and may not be a separate process step in and of itself.

As described herein, raw coal can be provided to a processing facility at block 910 for use in the method 900. The processing facility can have the capacity to store raw coal for use as needed, or can receive raw coal as needed to produce a desired amount of advanced carbon material. As is well known in the art, coal can be provided via truck, train, or any other form of transportation. Further, the processing facility can be situated at a coal extraction site, such that coal extraction site can be considered as part of the processing facility.

At block 920, the raw coal can be beneficiated to remove contaminants or impurities such as water, heavy metals, and/or volatile compounds from the raw coal, thereby producing beneficiated or upgraded coal. In some cases, the beneficiation process can comprise heating the raw coal to a desired temperature for a first duration. In some embodiments, beneficiation can also include heating the raw coal to a second, higher desired temperature of a second duration. In some embodiments, the coal can be heated in an atmosphere comprising a halogen gas. In some embodiments, beneficiation can include subjecting the raw coal to a WRITECoal beneficiation process, as described, for example, in U.S. Patent No. 9,181,509. In some other embodiments, the coal can be beneficiated by heating the coal to a desired temperature in the presence of one or more catalyst compounds. In some cases, beneficiating the coal can comprise pyrolyzing the coal, for example in the presence of a catalyst. In some cases, the coal can be beneficiated by the BenePlus System, as developed and licensed by LP Amina and as described, for example, in U.S. Patent Publication No. 2017/0198221.

. The beneficiated coal can comprise a significantly reduced amount of mercury, cadmium, other heavy metals, water, and/or other impurities. As used herein, an impurity can be considered any element or compound other than carbon or hydrogen. For example, beneficiating the coal can reduce the amount of mercury in the coal by about at least about 70%, 75%, 80%, 85%, 90%, or 92% or more. In some cases, beneficiating the coal can reduce the water or moisture content of the coal to less than about 5 wt. %, 4 wt. %, 3 wt. %, 2 wt. %, or 1.5 wt. % or lower. In some cases, beneficiating the coal can remove one or more of hydrogen, sulfur, oxygen, arsenic, selenium, cadmium, or volatile matter from the coal. The amount of one or more of these elements in the coal can be reduced by from about 25% to about 90%.

However, in some cases it can be desirable for a desired amount of one or more impurities to remain in the beneficiated coal after being subjected to a beneficiation process. For example, in some embodiments a beneficiation process can remove a desired amount of impurities such that a predetermined amount of cadmium, selenium, or another element can remain in the beneficiated coal after processing. In some cases, the desired amount of impurity that can remain in the coal after beneficiation can be useful in the subsequent formation of advanced carbon materials, and/or can be incorporated into the advanced carbon materials. For example, in some embodiments where the advanced carbon material comprises synthetic graphene, a desired amount of cadmium can remain in the beneficiated coal and can be incorporated into the synthetic graphene to thereby improve the electrical, mechanical, or chemical properties thereof.

In some embodiments, beneficiating the coal can produce various other products that can be captured and used in later processing steps, that can be valuable in and of themselves, or that can be subjected to further processing or use in the method 900. That is, in some embodiments, beneficiating the coal can produce or separate gases or liquids from the raw coal. These gases and/or liquids can be captured or separated during processing. For example, beneficiating the coal at block 920 can produce H₂, CO₂, CO, CH₄, C₂H₄, C₃H₆, and/or other hydrocarbon gases, which can be captured and subsequently utilized in block 930 or in other process steps. In some cases, beneficiating the coal can result in liquids such as toluene or benzene which can be captured for subsequent use or processing. In some cases, the impurities removed from the coal by the beneficiation process at block 920 can be captured for subsequent use. For example, water removed from the coal by the beneficiation process can be capture and utilized in subsequent process steps. In some embodiments, beneficiating the coal can also produce a solid material known as ash or char. In some cases, this char can be subjected to further processing to form activated ca

At block 930 the beneficiated coal, also referred to as upgraded coal, can be processed via the processing facility. In some embodiments, processing the beneficiated coal can include subjecting the upgraded coal to a liquid extraction process, such as a pyrolysis process, a direct liquefaction process, an indirect liquefaction process, or a process including one or more membranes.

In some embodiments, block 930 can comprise pyrolyzing the beneficiated coal via the processing facility. In some embodiments pyrolyzing the beneficiated coal can comprise heating the coal above a desired temperature for a duration. In some cases, the coal can be heated at high pressure and in the presence of a solvent. For example, the upgraded coal can be pyrolyzed in the presence of a CO solvent which can be held in a supercritical state. In some cases, the upgraded coal can be pyrolyzed by the MuSCL System developed by TerraPower as described, for example, in U.S. Patent No. 10,144,874.

In some embodiments, the pyrolysis process can comprise exposing the upgraded coal to electromagnetic radiation at a desired intensity and for a desired duration. For example, block 930 can comprise exposing the upgraded coal to microwave and/or radiofrequency (RF) radiation for a desired duration as part of the pyrolysis process. In some cases, this pyrolysis process can result in the bulk of the upgraded coal remaining below pyrolytic temperatures, while individual particles of coal can be subjected to temperatures greater than about 1200 °F. In some cases, this pyrolysis process can also comprise methane activation and/or methylation of at least some of the carbon comprising the upgraded coal. In some embodiments, the upgraded coal can be pyrolyzed by the Wave Liquefaction process developed by H Quest Vanguard, Inc. as described, for example, in U.S. Patent Publication No. 2017/0080399.

In some embodiments, block 930 can comprise subjecting the upgraded coal of block 920 to a liquefaction process. In some embodiments, the liquefaction process can be a direct liquefaction process. In some other embodiments, the liquefaction process can be an indirect liquefaction process.

In some cases where block 930 comprises a direct liquefaction process, the upgraded coal can be heated above a desired temperature for a duration. In some cases, the upgraded coal can be heated in the presence of one or more catalysts and/or at an elevated pressure. In some cases, the upgraded coal can be heated in an atmosphere comprising H. In some cases, the direct liquefaction process can be a hydrogenation or hydro-cracking process. In some cases, the upgraded coal can be subjected to a direct liquefaction process developed by Axens as described, for example, in U.S. Patent Publication No. 2017/0313886.

In some embodiments, block 930 can comprise an indirect liquefaction process, where the upgraded coal can be converted to a gas or gases, which can then be converted to one or more liquids. For example, an indirect liquefaction process can include heating the upgraded coal to a desired temperature for a desired duration at a desire pressure, such as an elevated pressure. In some cases, this can convert at least some of the upgraded coal to a gas or gases, such as syngas, a mixture of H and CO gas. In some cases, these gases, such as syngas, can then be converted to liquids or other materials. For example, the syngas can be converted to ammonia or methanol, which can in turn be subjected to further processing to produce hydrocarbons. In some cases, the gases can be processed to ultimately produce olefins, such as ethylene and propylene. In some cases, the gases can be processed to produce hydrocarbons, such as aromatic hydrocarbons. In some cases, the gases can be processed to produce hydrocarbons such as toluene, benzene, paraxylene, or other hydrocarbons, including polymers and resins. In some cases, the gases can be converted to olefins by a process developed by Honeywell UOP as described, for example, in U.S. Patent Publication No. 2015/0141726. In some cases, the upgraded coal can be subjected to an indirect liquefaction process.

In some embodiments, block 930 can comprise processing the beneficiated coal in the presence of one or more membranes. In some cases, these membranes can serve to physically and/or chemically separate and/or crack the beneficiated coal to produce products therefrom. In some cases, these products can be substantially similar to the products produced by a liquefaction process, but may not use the amount of heat or pressure that a liquefaction process can require. Thus, in some cases, processing beneficiated coal with one or more membranes can produce substantially similar products to a liquefaction process but can require substantially less energy to do so. In some embodiments, the one or more membranes can comprise various pore sizes, chemical properties, physical properties, or electrical properties to isolate desirable compounds and/or produce desirable compounds from the beneficiated coal.

In some embodiments, one or more additives can be added to the beneficiated coal at block 940. In some embodiments, one or more other gases or liquids can be used during the processes of block 940. For example, hydrogen containing gases can be added to or used during a coal liquefaction process. In some cases, natural gases, CO3, or petroleum products can be used as additives during block 930. In some embodiments, the one or more additives can include materials or compounds that are produced during blocks 920 and/or 930, or that can be produced by or captured during previous iterations of process 900.

At block 940, pitch, char, gases, and/or coal liquid are produced via the processing facility. The skilled artisan will appreciated that block 940 can represent the result of blocks 920 and 940, rather than a separate action or process step. While the blocks 910-950 together define the method 900, the method can include additional steps as described herein.

In some embodiments, pitch can be produced via the processing facility at block 940. As used herein, pitch, also known as coal pitch, coal tar, or coal tar pitch, can refer to a mixture of one or more typically viscoelastic polymers as will be well understood by the skilled artisan. In some embodiments, the pitch produced at block 940 can be a direct result of processing the beneficiated coal at step 930. The pitch produced at block 940 can comprise one or more high molecular weight polymers. In some embodiments, the pitch can have a melting point of greater than about 650 °F. In some embodiments, the pitch can have a melting point that is high enough that the pitch can be used in a carbon fiber spinning process, for example as described herein, without the need for a plasticizer.

In some embodiments, the pitch can comprise aromatic hydrocarbons, for example polycyclic aromatic hydrocarbons. In some cases, the pitch can comprise at least about 50 wt. % polycyclic aromatic hydrocarbons, at least about 60 wt. %, 70 wt. %, 80 wt. %, 90 wt. %, 95 wt. %, or 99 wt. % or greater of polycyclic aromatic hydrocarbons. In some embodiments, the pitch can be relatively free of impurities, such as water, non-carbon atoms including sulfur or nitrogen, or material such as coal ash or char. In some cases, the pitch can comprise less than about 0.2 wt. % water, less than about 0.1 wt. %, less than about 0.05 wt. %, or less than about 0.01 wt. % water or lower. In some cases, the pitch can comprise less than about 0.1 wt. % ash or other solid material, less than about 0.05 wt. % ash or solid material, or less than about 0.01 wt. % ash or solid material. In some cases, the pitch can have a flash point greater than about 230 °F, greater than about 250 °F, or greater than about 300 °F. In some cases, the pitch can have an API gravity of less than about 4, less than about 3, or less than about 2, or less. In some embodiments, the pitch can have a hydrogen to carbon (H:C) ratio of about 1: 1. In some embodiments, the pitch can be an isotropic pitch. In some embodiments, the pitch produced by the method 900 is not coke pitch. That is, in some cases, the pitch produced at block 940 is not produced from coke or a coke based material. In some embodiments, coke is not produced at any point during the method 900.

In some embodiments, char can be produced via the processing facility at block 940. As used herein, char, also known as ash, can refer to any solid material which remains after gases, liquids, and/or pitch have been removed from raw coal. For example, in some embodiments, char can be produced during the beneficiation of raw coal at block 920. In some embodiments, char can be produced by the processing of block 930. In some embodiments, char can be produced as a result of blocks 920 and 930.

In some embodiments, char can comprise a solid high surface area carbonaceous material. In some cases, char can have a relatively low H:C ratio, for example lower than the H:C ratio of pitch produced at block 940. In some cases, char can have an H:C ratio of from about 0.05 to about 0.65. In some cases, char can additionally comprise at least some pitch material, which can be referred to herein as intrinsic binder impregnation. In some cases, any residual pitch or other gaseous or liquid materials can be removed from the char prior to any subsequent processing of the char.

In some embodiments, any char produced at block 940 can be subjected to further processing, for example to produce an advanced carbon material such as activated carbon. In some cases, char can be carbonized or heated, for example in a rotary kiln, as part of this further processing. In some cases the char can then be activated, for example via a physical activation process or a chemical activation process. In some cases, physical activation can comprise heating the char in an atmosphere comprising argon and/or nitrogen, or heating the char in an oxidizing atmosphere. In some cases, chemical activation can comprise impregnating the char with one or more chemicals, such as an acid, a base, or a salt. In some cases, chemical activation can further comprise carbonizing or heating the impregnated char to activate it. In some cases, chemical activation can require lower temperatures and less energy than physical activation. Further, in some cases, chemical byproducts produced by the method 900 can be utilized during the chemical activation process.

In some embodiments, one or more liquids can be produced via the processing facility at block 940. These liquids are referred to collectively as coal liquid extracts herein, and can refer to any material that is extracted or produced from raw coal and is liquid at or near normal temperature and pressure (about 68 °F and 1 atmosphere of pressure). For example, in some embodiments, coal liquid extracts can be produced during the beneficiation of raw coal at block 920. In some embodiments, coal liquid extracts can be produced by the processing of block 930. In some embodiments, coal liquid extracts can be produced as a result of blocks 920 and 930.

In some embodiments, one or more gases can be produced via the processing facility at block 940 and can be captured for later use or reuse. In some embodiments, these gases can be produced during the beneficiation of raw coal at block 920. In some embodiments, gases can be produced by the processing of block 930. In some embodiments, gases can be produced as a result of blocks 920 and 930. In some embodiments, the captured gas or gases can comprise hydrogen and/or carbon. In some cases, the captured gas or gases can comprise sulfur. Such gases can include, for example, H₂, CO₂, CO, CH₄, C₂H₄, C₃H₆, and/or other hydrocarbon gases. As described herein, in some cases, the captured gases produced by the method 900 can themselves be used as precursors to form advanced carbon materials.

In some embodiments, coal liquid extracts can comprise one or more liquid hydrocarbons. For example, coal liquid extracts can comprise one or more of benzene, toluene, alkanes or paraffins, alkenes, or other saturated or unsaturated hydrocarbons. In some embodiments, coal liquid extracts produced at block 940 can be subjected to further processing, for example to refine or isolate specific liquids, or to convert coal liquid extracts to other liquid compounds. For example, the coal liquid extracts can be subjected to a process to convert one or more of the coal liquid extracts to benzene and/or paraxylenes. In some cases, the coal liquid extracts can be subjected to processing to produce advanced carbon material, such as resins or polymers. In some cases, the coal liquid extracts can be processed to form polyurethane resins, cyanate ester resins, epoxy resins, methacrylate resins, polyester resins, and others.

Pitch, char, gases, and coal liquid extracts are all described as being produced at block 940, however in some embodiments, one or more of these products can be produced at separate times or separate processing steps from any other product. In some embodiments, one or more of pitch, char, and coal liquid extracts can be produced together by a process step and can need to be separated before any further processing of each individual product can occur. For example, pitch and coal liquid extracts can be simultaneously produced as a result of block 930 and can need to be separated from one another, by any process now know or which can be developed in the future, before further processing of either pitch or coal liquid extracts occurs. Accordingly, the method 900 can comprise a further step of separating one or more of the pitch, char, and coal liquid extracts from each other prior to block 950.

At block 950, the pitch produced at block 940 can be treated via the processing facility to produce one or more advanced carbon materials described herein. In some embodiments, the pitch of block 940 is not subjected to further processing or refinement to alter the chemical composition of the pitch before being treated to form an advanced carbon material. However, in some other embodiments, the pitch can be subjected to one or more processes which can alter the chemical composition of the pitch prior to block 950. For example, impurities can be removed from the pitch prior to block 950. In some cases, the pitch can be subjected to one or more processes to produce mesophase pitch or otherwise alter the composition or properties of the pitch

In some embodiments, block 950 can comprise processing the pitch to form synthetic graphite. In some cases, the synthetic graphite can be subjected to further processing to form synthetic graphene. For example, in some embodiments block 950 can comprise treating the pitch, for example by exposure to heat, elevated pressure, and/or one or more catalysts to form synthetic graphite. As used herein, the term synthetic graphite is used to refer to any graphite material produced from a precursor material, for example any graphite material that does not occur naturally in the earth. In some embodiments, block 950 can further comprise treating or processing the synthetic graphite to form synthetic graphene. As used herein, synthetic graphene refers to any graphene material produced or derived from synthetically formed graphite. For example, block 950 can comprise subjecting the synthetic graphite to mechanical exfoliation to produce synthetic graphene.

In some embodiments, the method 900 can further comprise capturing at least some of the gases, liquids, or other volatile compounds which can be produced as a result of the method, 900, including blocks 910-950. In some embodiments at least 50%, at least 75%, at least 90%, 95%, or 99% of any gaseous or volatile byproducts of the method 900 can be captured. In some cases, some or all of these captured or retained gaseous or volatile byproducts can then be used in the steps of the method 900. For example, CO₂ gas can be produced by one or more of the steps of method 900, which can be captured and subsequently used in any of the steps of method 900. In some cases, the capture and reuse of byproducts can improve the efficiency and/or lower the cost of the method 900.

According to some embodiments, an advanced carbon material can be produced from coal by a method or process including:
providing coal to a processing facility;
beneficiating the coal via the processing facility to remove a desired amount of water, metals, volatile compounds, and other impurities from the coal;
processing at least some of the beneficiated coal via the processing facility;
producing pitch, char, gases, and/or coal liquid extract; and
treating one or more of the pitch, char, gases, and/or coal liquid extract via the processing facility to produce one or more advanced carbon materials.

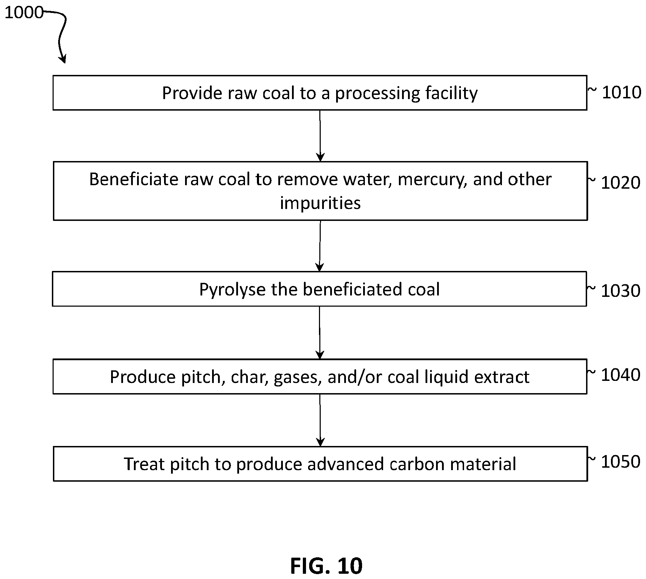

According to some embodiments, and as illustrated in FIG. 10, an advanced carbon material can be produced from coal by a method or process 1000 including:
providing coal to a processing facility at block 1010;
beneficiating the coal via the processing facility at block 1020 to remove a desired amount of water, metals, and other impurities from the coal;
pyrolyzing at least some of the beneficiated coal via the processing facility at block 1030;
producing pitch, char, gases, and/or coal liquid extract at block 1040; and
treating at least one of the pitch, char, gases, and coal liquid extract via the processing facility at block 1050 to produce the advanced carbon material. Although the method 1000 describes a process flow for producing a single type of advanced carbon material via a processing facility, the method 1000 can be used to produce more than one type of advanced carbon material via the processing facility. For example, the method 1000 can be utilized to produce an amount of a first advanced carbon material and subsequently used to produce an amount of a second, different advanced carbon material. In some cases however, the processing facility can be able to produce two or more different types of advanced carbon materials via parallel processing utilizing the method 1000. Further, while producing pitch, char, gases, and/or coal liquid extract is described as a separate block 1040, the pitch, char, gases, and/or coal liquid extract can be produced as a result of blocks 1020 and/or 1030 and may not be a separate process step in and of itself.

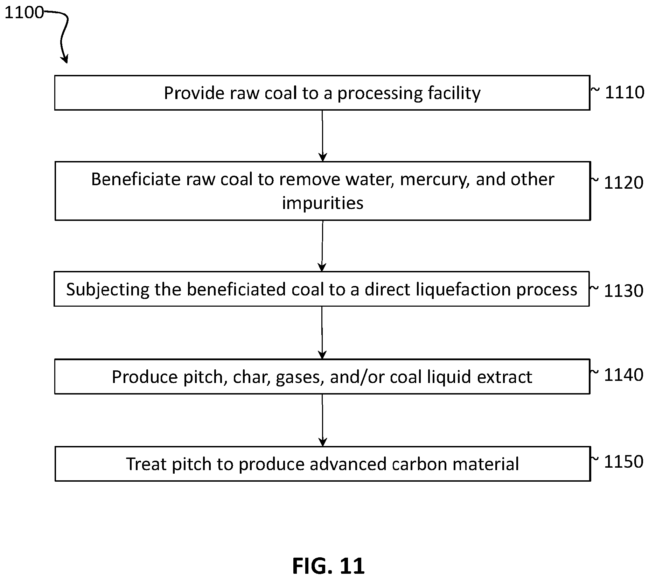

According to some embodiments, and as illustrated in FIG. 11, an advanced carbon material can be produced from coal by a method or process 1100 including:
providing coal to a processing facility at block 1110;
beneficiating the coal via the processing facility at block 1120 to remove a desired amount of water, metals, and other impurities from the coal;
subjecting at least some of the beneficiated coal to a direct liquefaction process via the processing facility at block 1130;
producing pitch, char, gases, and/or coal liquid extract at block 1140; and
treating at least one of the pitch, char, gases, and coal liquid extract via the processing facility at block 1150 to produce the advanced carbon material.

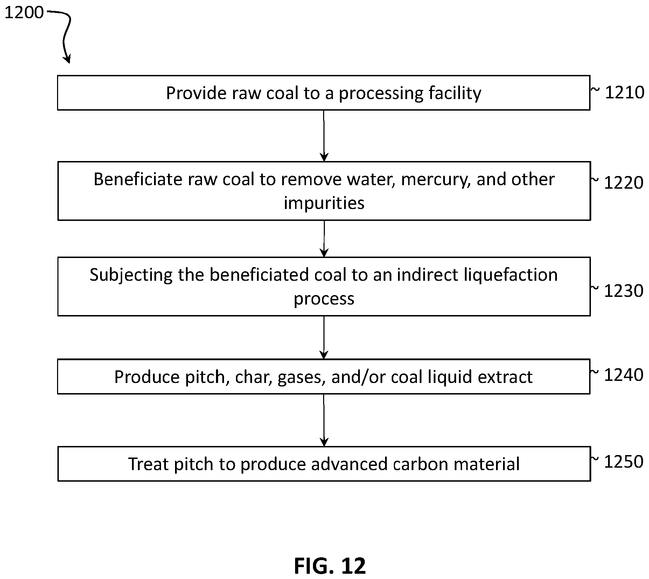

Although the method 1100 describes a process flow for producing a single type of advanced carbon material via a processing facility, the method 1100 can be used to produce more than one type of advanced carbon material via the processing facility. For example, the method 1100 can be utilized to produce an amount of a first advanced carbon material and subsequently used to produce an amount of a second, different advanced carbon material. In some cases however, the processing facility can be able to produce two or more different types of advanced carbon materials via parallel processing utilizing the method 1100. Further, while producing pitch, char, gases, and/or coal liquid extract is described as a separate block 1140, the pitch, char, gases, and/or coal liquid extract can be produced as a result of blocks 1120 and/or 1130 and may not be a separate process step in and of itself. According to some embodiments, and as illustrated in FIG. 12, an advanced carbon material can be produced from coal by a method or process 1200 including:
providing coal to a processing facility at block 1210;
beneficiating the coal via the processing facility at block 1220 to remove a desired amount of water, metals, and other impurities from the coal;
subjecting at least some of the beneficiated coal to an indirect liquefaction process via the processing facility at block 1230;
producing pitch, char, gases, and/or coal liquid extract at block 1240; and
treating at least one of the pitch, char, gases, and coal liquid extract via the processing facility at block 1250 to produce the advanced carbon material.

Although the method 1200 describes a process flow for producing a single type of advanced carbon material via a processing facility, the method 1200 can be used to produce more than one type of advanced carbon material via the processing facility. For example, the method 1200 can be utilized to produce an amount of a first advanced carbon material and subsequently used to produce an amount of a second, different advanced carbon material. In some cases however, the processing facility can be able to produce two or more different types of advanced carbon materials via parallel processing utilizing the method 1200. Further, while producing pitch, char, gases, and/or coal liquid extract is described as a separate block 1240, the pitch, char, gases, and/or coal liquid extract can be produced as a result of blocks 1220 and/or 1230 and may not be a separate process step in and of itself.

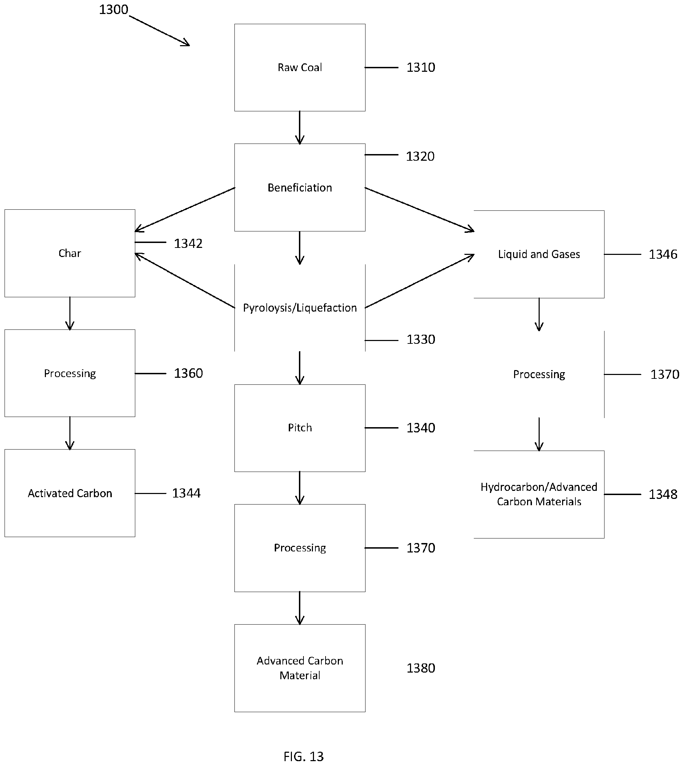

FIG. 13 illustrates a material flow diagram of an example of a method 1300 of producing carbon fiber and, optionally, one or more advanced carbon materials from coal in accordance with the present disclosure, according to an embodiment. Except as otherwise disclosed herein, the method 1300 is the same as or substantially similar to any of the methods 100, 200, 300, 400, 500, 600, 700, 800, 900, 1000, 1100, or 1200 of FIGS. 1-12. For example, the method 1300 can be a combination of two or more of the methods 100, 200, 300, 400, 500, 600, 700, 800, 900, 1000, 1100, or 1200 of FIGS. 1-12. At block 1310, raw coal is provided to a processing facility, for example by a mining process, such as high wall mining. The raw coal can include any of the raw coal disclosed herein. The raw coal can then be subjected to a beneficiation process to remove a desired amount of water, metals, volatile matter, and other impurities as described herein at block 1320. The beneficiation process can include any of the beneficiation processes disclosed herein. In some cases, the beneficiation process can produce byproducts, such as char (block 1342) and gases and/or coal liquid extracts (1346), in addition to the beneficiated coal. The beneficiated coal can be subjected to a liquid extraction process (*e*.*g*., pyrolysis, direct liquefaction, or indirect liquefaction process) at block 1330 and as described herein to produce pitch (block 1340). Again, in some cases, the pyrolysis or liquefaction process of block 1340 can produce byproducts, such as char (block 1342) and coal liquid extracts and gases (block 1346). In some cases, the char 1342 can be processed or treated at block 1360 to produce an additional advanced carbon material, for example activated carbon (block 1344), as described herein. In some cases, the coal liquid extract 1346 can be processed or treated at block 1370 to produce at least one of hydrocarbons (*e*.*g*., benzene and paraxylenes), carbon fibers, or other additional advanced carbon materials (block 1348), as described herein. At block 1350, the pitch can be processed or treated to produce carbon fibers (block 1380), as described herein. In an embodiment, the block 1380 can also include processing or treating the pitch to produce one or more additional advanced carbon materials in addition to the carbon fiber, as described herein.

In some embodiments, the method 1300 can be entirely carried out at a single processing facility. However, in some other cases, one or more blocks can be carried out at different processing facilities and/or different locations. For example, char 1342 or coal liquid extract 1346 can be transported to a second location where blocks 1360 and 1370 can be carried out.

Although the processes described herein relate to the production of carbon-based carbon fibers and additional advanced carbon materials from coal, in some embodiments these processes can be utilized to produce silicon products, such as silicone carbon fibers. For example, in some embodiments, sand or other raw materials comprising silicon can be used in the processes and methods described herein to produce one or more silicone carbon fibers.

Although the processes described herein relate to the production of advanced carbon materials from coal, in some embodiments these processes can be utilized to produce silicon products, such as silicone resins. For example, in some embodiments, sand or other raw materials comprising silicon can be used in the processes and methods described herein to produce one or more silicone resins.

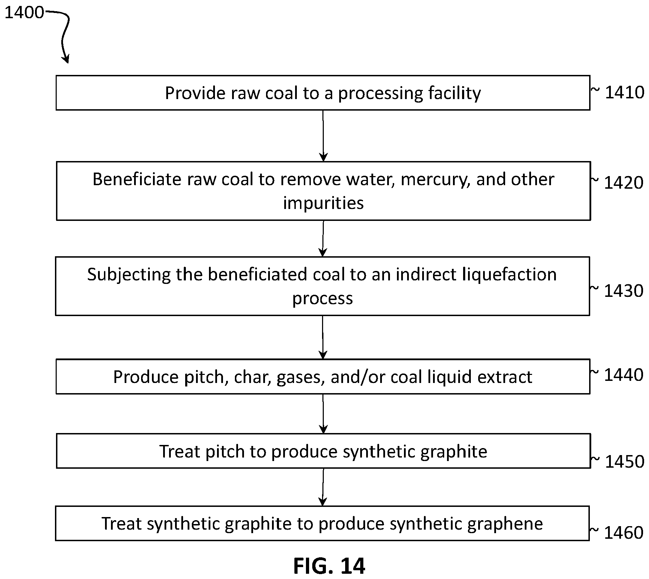

According to some embodiments, and as illustrated in FIG. 14, synthetic graphene can be produced from coal by a method or process 1400 including:
providing coal to a processing facility at block 1410;
beneficiating the coal via the processing facility at block 1420 to remove a desired amount of water, mercury, cadmium, selenium, other heavy metals, and/or other impurities from the coal;
processing at least some of the beneficiated coal via the processing facility at block 1430;
producing pitch, char, gases, and/or coal liquid extract at block 1440; and
treating at least one of the pitch, char, gases, and coal liquid extract via the processing facility at block 1450 to produce synthetic graphite.
treating the synthetic graphite via the processing facility at block 1460 to produce the synthetic graphene.

As described herein, a desired amount of one or more impurities can remain in the beneficiated coal at block 1420 and can thereby be incorporated into the synthetic graphene produced at block 1460 in order to adjust the chemical, electrical, and/or physical properties of the synthetic graphene.

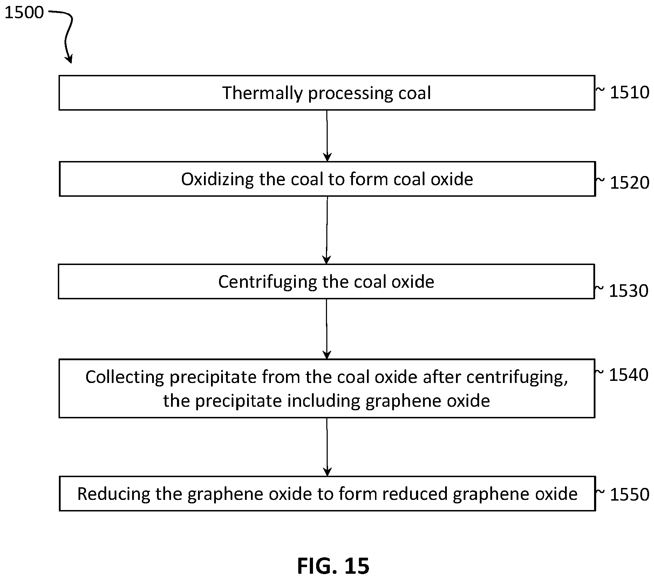

According to some embodiments, and as illustrated in FIG. 15, graphene or another advanced carbon material can be produced from coal by a method or process 1500 including thermally processing coal at block 1510 and forming reduced graphene oxide from the coal after the coal has been at least partially cooled from thermal processing. Forming reduced graphene oxide from the coal can include: oxidizing the coal at block 1520 to form coal oxide; centrifuging the coal oxide at block 1530; collecting precipitate from the coal oxide after centrifuging at block 1540, the precipitate including graphene oxide; and reducing the graphene oxide at block 1550 to produce reduced graphene oxide. In other embodiments, other activities can be used to form graphene from the thermally-treated coal, such as chemical vapor deposition or formation of carbon dots.

Although the method 1500 describes a process flow for producing graphene, the method 1500 can be used to produce more than one type of advanced carbon material. For example, the method 1500 can be utilized to produce an amount of a first advanced carbon material and subsequently used to produce an amount of a second, different advanced carbon material. In some cases however, two or more different types of advanced carbon materials can be produced via parallel processing utilizing the method 1500. Further, various steps of method 1500 can each include multiple steps, and various steps of method 1500 may not be a separate process steps in and of themselves.

The coal that is thermally processed at block 1510 can include raw coal, as described in greater detail above, or coal that is at least partially processed. The raw or at least partially processed coal can include coal crushed to a suitable or predetermined size. Thermally processing the coal can include beneficiating the coal to remove contaminants or impurities, as described above in greater detail. Thermally processing the coal according to this disclosure also can include any of the activities described in U.S. Patent No. 5,403,365.

In many embodiments, thermally processing coal can include thermally processing coal at a temperature of at least about 300° F. More particularly, in some embodiments, thermally processing coal can include heating the coal to a first temperature not to exceed 350°F, transferring the coal to a mercury removal reactor, heating the coal in the mercury removal reactor to a second temperature of at least 500°F, and contacting the coal with an inert gas to remove at least a portion of mercury present in the coal.

In some embodiments, heating the coal to a first temperature not to exceed 350°F includes heating the coal to a first temperature not to exceed between about 250°F and about 350 °F, between about 275°F and about 325°F, between about 285°F and about 315°F, between about 295°F and about 305°F, between about 295°F and about 300°F, between about 300°F and about 305°F, about 290°F, about 295°F, about 300°F, about 301°F, about 302°F, about 303°F, about 304°F, about 305°F, about 310°F, about 315°F, about 320°F, about 325°F, about 330°F, about 335°F, about 340°F, about 345°F, or about 350°F. When the coal is heated to the first temperature, free water and at least a portion of bound water in the coal is vaporized and removed in a sweep gas. In some embodiments, the coal can be heated in a moisture removal reactor and/or a vibrating fluidized-bed system. Dryer auxiliaries in the moisture removal reactor can include a hot gas generator, a coal feeder, and valves.

After heating the coal to the first temperature, the coal can be transferred to a mercury removal reactor, where the coal can be heated to a second temperature of at least 500°F. In some embodiments, the coal can be heated in the mercury removal reactor to a temperature of between about 400°F and about 700°F, between about 450°F and about 650°F, between about 500°F and about 600°F, between about 525°F and about 575°F, between about 535°F and about 570°F, between about 540°F and about 565°F, between about 545°F and about 560°F, between about 550°F and about 555°F, at least about 500°F, at least about 510°F, at least about 520°F, at least about 530°F, at least about 540°F, at least about 545°F, at least about 550°F, at least about 555°F, about 550°F, about 551°F, about 552°F, about 553°F, about 554°F, about 555°F, about 556°F, about 557°F, about 558°F, about 559°F, or about 560°F.

A down-flow reactor can be used to expose the coal to a hot inert gas, which volatizes and removes at least a portion of the mercury and/or at least a portion of one or more dopants. For example, between about 70% and about 80% of the mercury in the coal can be volatized and removed from the coal. In some cases, other dopants or impurities can be removed from the coal, for example, cadmium, selenium, and/or any other element except carbon which may be present in the coal. The coal also can be cooled to a third temperature in the mercury removal reactor, the third temperature being below about 400°F, below about 375°F, below about 350°F, below about 325°F, below about 300°F, below about 275°F, or below about 250°F. Upon cooling to the third temperature, the coal can be reduced in size before forming reduced graphene oxide from the coal.

As noted above, forming reduced graphene oxide from the coal can include: oxidizing the coal at block 1520 to form coal oxide; centrifuging the coal oxide at block 1530; collecting precipitate from the coal oxide after centrifuging at block 940, the precipitate including graphene oxide; and reducing the graphene oxide at block 950 to produce reduced graphene oxide. At block 1520, oxidizing the coal to form a coal oxide can include mixing the coal with at least one of sulfuric acid, nitric acid, or potassium permanganate, or hydrogen peroxide to form the coal oxide. In some embodiments, mixing the coal with at least one of sulfuric acid, nitric acid, potassium permanganate, or hydrogen peroxide to form the coal oxide can include: mixing the coal with sulfuric acid and then mixing nitric acid with the mixture of coal and sulfuric acid.

In some embodiments, mixing the coal with at least one of sulfuric acid, nitric acid, potassium permanganate, or hydrogen peroxide to form the coal oxide can include stirring the mixture of coal, sulfuric acid, and nitric acid for a predetermined period of time. For example, in some embodiments, the mixture of coal, sulfuric acid, and nitric acid can be stirred for at least about 1 hour, at least about 2 hours, at least about 3 hours, at least about 4 hours, at least about 5 hours, between about 1 hours and about 5 hours, between about 2 hours and about 4 hours, between about 2.5 hours and about 3.5 hours, about 1 hour, about 2 hours, about 3 hours, about 4 hours, about 5 hours, less than about 1 hour, less than about 2 hours, less than about 3 hours, less than about 4 hours, or less than about 5 hours.

In some embodiments, mixing the coal with at least one of sulfuric acid, nitric acid, potassium permanganate, or hydrogen peroxide to form the coal oxide can include, after stirring the mixture of coal, sulfuric acid, and nitric acid, mixing potassium permanganate to the mixture of coal, sulfuric acid, and nitric acid and then stirring the mixture of coal, sulfuric acid, nitric acid, and the potassium permanganate for a predetermined period of time. In some embodiments, the mixture of coal, sulfuric acid, nitric acid, and the potassium permanganate can be stirred on a hot plate heated to between about 25°C and about 45°C, between about 30°C and about 40°C, between about 33°C and about 37°C, at least about 25°C, at least about 30°C, at least about 35°C, at least about 40°C, at least about 45°C. about 25°C, about 30°C, about 35°C, about 40°C, or about 45°C. In some embodiments, the mixture of coal, sulfuric acid, nitric acid, and the potassium permanganate can be stirred for at least about 1 hour, at least about 2 hours, at least about 3 hours, at least about 4 hours, at least about 5 hours, at least about 6 hours between about 1 hours and about 6 hours, between about 3 hours and about 5 hours, between about 3.5 hours and about 4.5 hours, about 1 hour, about 2 hours, about 3 hours, about 4 hours, about 5 hours, about 6 hours, less than about 1 hour, less than about 2 hours, less than about 3 hours, less than about 4 hours, less than about 5 hours, or less than about 6 hours.

In some embodiments, mixing the coal with at least one of sulfuric acid, nitric acid, potassium permanganate, or hydrogen peroxide to form the coal oxide can include diluting the mixture of coal, sulfuric acid, nitric acid, and the potassium permanganate with water to form a solution. The mixture of coal, sulfuric acid, nitric acid, and the potassium permanganate with water at a predetermined mixture:water dilution ratio, such as such as about 1:1, about 1:2, about 1:3, about 1:4, about 1:5, about 1:6, about 1:7, or about 1:8.

In some embodiments, mixing the coal with at least one of sulfuric acid, nitric acid, potassium permanganate, or hydrogen peroxide to form the coal oxide can include mixing the solution, as diluted, with hydrogen peroxide. The hydrogen peroxide can include 10% hydrogen peroxide. Upon mixing the hydrogen peroxide with the solution, the solution turns to a yellow or yellow-green color.

In some embodiments, mixing the coal with at least one of sulfuric acid, nitric acid, potassium permanganate, or hydrogen peroxide to form the coal oxide can include performing a first centrifugation of the solution mixed with the hydrogen peroxide. The solution mixed with hydrogen peroxide can be centrifuged for a predetermined period of time, such as at least about 15 minutes, at least about 30 minutes, at least about 45 minutes, at least about 60 minutes, between about 15 minutes and about 45 minutes, about 15 minutes, about 30 minutes, about 45 minutes, or about 60 minutes. The solution mixed with hydrogen peroxide can be centrifuged at a predetermined revolutions per minute (RPM) that can be dependent on the centrifuge machine. In some embodiments, the solution mixed with hydrogen peroxide can be centrifuged at about 500 RPM.

In some embodiments, mixing the coal with at least one of sulfuric acid, nitric acid, potassium permanganate, or hydrogen peroxide to form the coal oxide can include after performing the first centrifugation, separating supernatant of the solution mixed with the hydrogen peroxide from precipitate of the solution mixed with the hydrogen peroxide. The supernatant includes the coal oxide, and the precipitate can be waste. Once the supernatant has been separated from the precipitate, the supernatant can be diluted with water at a predetermined supematant:water dilution ratio. For example, the supernatant can be diluted with water at a dilution ratio of 1:0.5, 1:1, 1:1.5, 1:2, 0.5:1, 1.5:1, or 2:1.

At block 1530, the coal oxide can be centrifuged during a second centrifugation. The coal oxide that is centrifuged can include the dilution of water and coal oxide described above. The coal oxide and water can be centrifuged for a predetermined period of time, such as at least about 10 minutes, at least about 15 minutes, at least about 20 minutes, at least about 25 minutes, at least about 30 minutes, at least about 35 minutes, at least about 40 minutes, between about 10 minutes and about 40 minutes, between about 15 minutes and about 25 minutes, about 15 minutes, about 20 minutes, about 25 minutes, or about 30 minutes. The coal oxide and water can be centrifuged at a predetermined RPM that can be dependent on the centrifuge machine. In some embodiments, the coal oxide and water can be centrifuged at an RPM that is greater than the RPM of centrifuging of the solution mixed with hydrogen peroxide. For example, the coal oxide and water can be centrifuged at greater than 10,000 RPM, such as about 12,800 RPM.

At block 1540, graphene oxide can be collected as precipitate after centrifuging the coal oxide and water, and the supernatant of the centrifugation can be waste. In some cases, block 1540 can include sonicating the coal oxide while the coal oxide is in water. In some cases, block 1540 can include sonicating graphene oxide that has already been collected as a precipitate.

At block 1550, the graphene oxide can be reduced to form reduced graphene oxide. Reducing the graphene oxide removes oxygen containing groups from the graphene oxide and at least partially recovers the electrical conductivity of the graphene. In some embodiments, reducing the graphene oxide to form reduced graphene oxide can include at least sonicating the graphene oxide. The interaction between water and a functional group of the graphene oxide promotes exfoliation of the coal oxide layer. The graphene oxide can be sonicated for a predetermined amount of time, such as at least such as at least about 5 minutes, at least about 10 minutes, at least about 15 minutes, at least about 20 minutes, between about 5 minutes and about 15 minutes, about 5 minutes, about 10 minutes, about 15 minutes, or about 20 minutes. After sonication, the graphene oxide and/or reduced graphene oxide can be analyzed to determine the quality of the graphene oxide and/or reduced graphene oxide using a Raman spectroscopy analysis.

In some embodiments, reducing the graphene oxide to form reduced graphene oxide can include hydrothermally treating the graphene oxide in a par reactor after sonicating the graphene oxide. The graphene oxide can be hydrothermally treated in the par reactor for a predetermined amount of time, such as at least about 1 hour, at least about 2 hours, at least about 3 hours, at least about 4 hours, at least about 5 hours, at least about 6 hours between about 1 hours and about 6 hours, between about 3 hours and about 5 hours, between about 3.5 hours and about 4.5 hours, about 1 hour, about 2 hours, about 3 hours, about 4 hours, about 5 hours, about 6 hours, less than about 1 hour, less than about 2 hours, less than about 3 hours, less than about 4 hours, less than about 5 hours, or less than about 6 hours. The graphene oxide can be hydrothermally treated in the par reactor at a predetermined temperature, such as between about 100°C and about 300°C, between about 130°C and about 230°C, between about 170°C and about 190°C, between about 175°C and about 185°C, at least about 100°C, at least about 140°C, at least about 170°C, at least about 180°C, at least about 190°C, about 170°C, about 175°C, about 180°C, about 185C, or about 190°C. After the graphene oxide is reduced in the par reactor, the resultant solution is clear with jet black flakes and chunks. The jet black flakes and chunks are reduced graphene oxide, which can be confirmed via Raman spectroscopy.

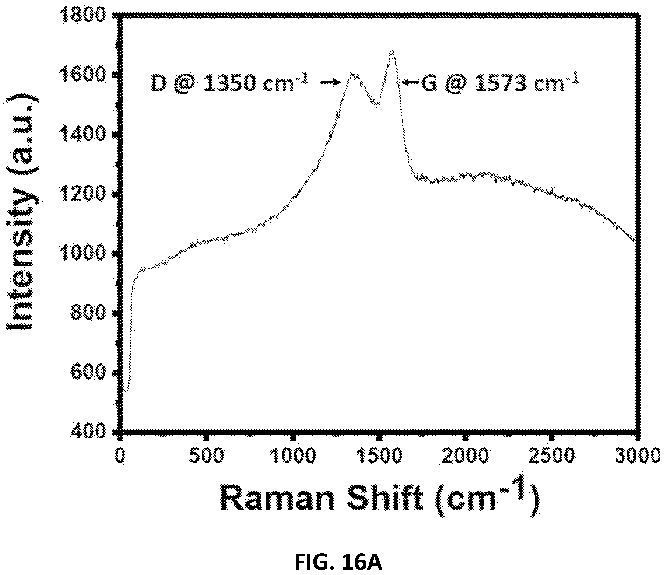

The steps of one or more embodiments of the method 1500 surprisingly produced improved graphene, as demonstrated in the Raman spectroscopy graphs shown in FIGS. 16A-16D. Specifically, FIG. 16A is a Raman spectroscopy graph of a sample of raw (non-thermally-treated) Monarch seam coal. The Monarch seam coal has two characteristic Raman vibrational modes: D-band and G-band. The D-band (1350 cm⁻¹) is the defect-induced Raman band. The D-band represents the ring breathing mode of sp² hybridized carbon. In order to be active, the ring must be adjacent to a graphene edge or a defect. The G-band (1573 cm⁻¹) is the first order Raman spectrum, and corresponds to the in-plane vibration of two neighboring carbon atoms on a sp²-hybridized graphene layer. The G peak corresponds to the high-frequency E_{2g} phonon at Γ-point.

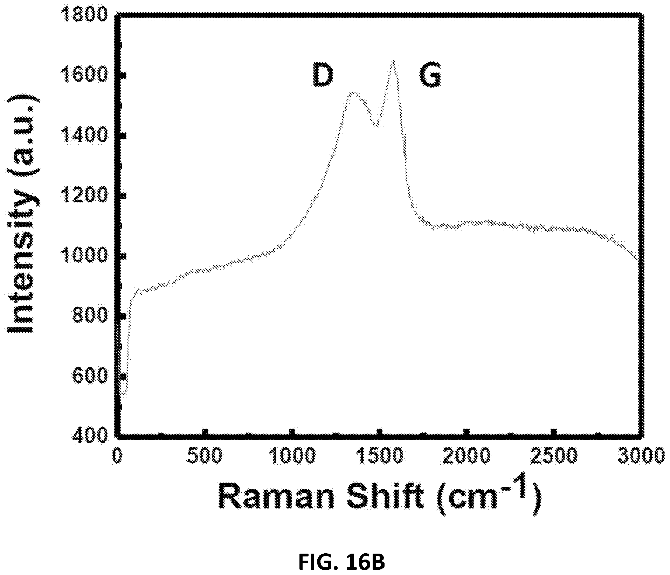

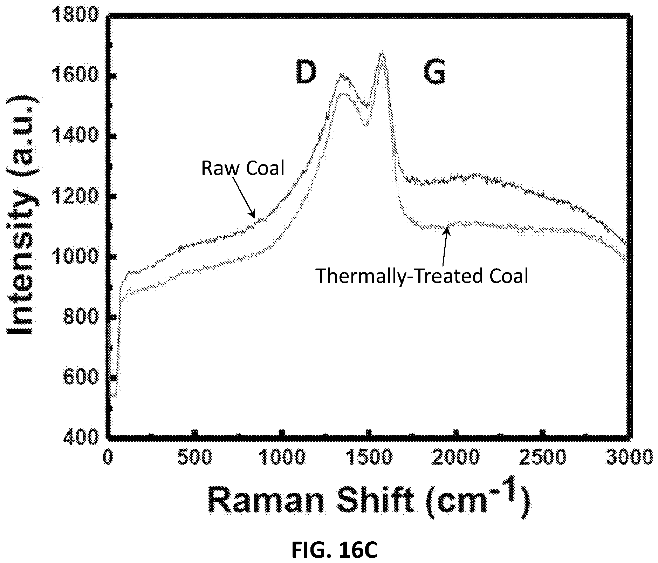

FIG. 16B is a Raman spectroscopy graph of a sample of thermally-treated Monarch seam coal. As shown in FIG. 16B, the thermally-treated Monarch seam coal also has two characteristic Raman vibrational modes: D-band and G-band. FIG. 16C is a Raman spectroscopy graph showing a comparison of the sample of Monarch seam coal of FIG. 16A to the sample of thermally-treated Monarch seam coal of FIG. 16B. The comparison indicates that the thermally-treated coal has less defects than the raw Monarch seam coal, as the intensity of the D-band (defect-induced Raman mode) is less. This difference in intensity is shown as (I_{G}/I_{D})_{Thermally-Treat}>(I_{G}/I_{D})_{Raw}. The comparison also indicates that graphitization and graphene formation is better in the thermally-treated coal sample, as the G-Band (graphitic-mode) is more sharp and symmetrical in the thermally-treated coal sample than the raw coal sample. Accordingly, thermal-treatment of coal according to this disclosure improves the graphene formation and reduces the defects in the coal.

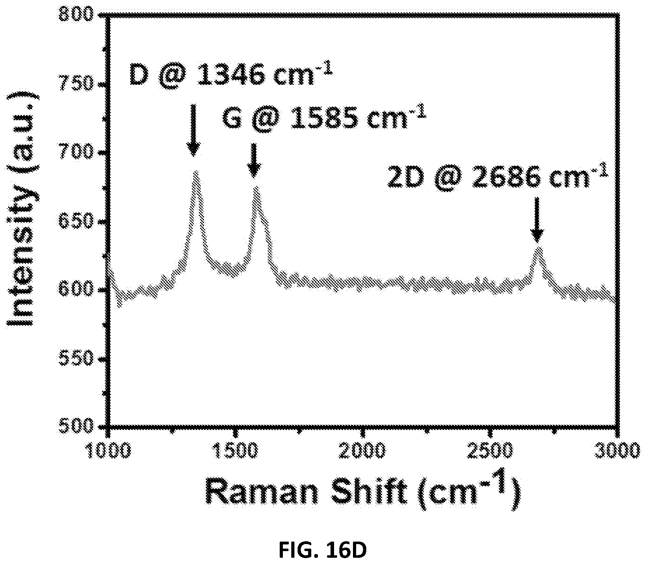

FIG. 16D is a Raman spectroscopy graph of a sample of graphene from thermally-treated Monarch seam coal. As can be seen in the figure, the intensity includes three sharp peaks at the D-band, G-band, and 2D-band. The sharpness of these peaks, for example as compared to the relatively broad peaks and overall shape of the graphs of FIGS. 16A-16C indicates the presence of substantially pure graphene. The resultant graph of FIG. 16D is indicative of substantially pure graphene nanoplatelets successfully derived from coal.

The steps of one or more embodiments of the method 1500 also surprisingly produced graphene oxide at a high yield rate relative to conventional processes for forming graphene from coal. For example, it was surprisingly observed that the thermally processing coal at a temperature of at least about 300° F and then forming reduced graphene oxide from the coal yielded reduced graphene oxide at a yield rate of between approximately 10 weight percent and approximately 20 weight percent of the coal. The steps of one or more embodiments of method 1500 can yield reduced graphene oxide at a yield rate of at least about 5 weight percent of the coal, at least about 10 weight percent of the coal, at least about 15 weight percent of the coal, at least about 20 weight percent of the coal, at least about 25 weight percent of the coal, at least about 30 weight percent of the coal, at least about 35 weight percent of the coal, at least about 40 weight percent of the coal, at least about 45 weight percent of the coal, at least about 50 weight percent of the coal, between about 2 weight percent and about 50 weight percent of the coal, between about 4 weight percent and about 40 weight percent of the coal, between about 8 weight percent and about 30 weight percent of the coal; between about 10 weight percent and about 20 weight percent of the coal, between about 12 weight percent and about 17 weight percent of the coal, between about 5 weight percent and about 10 weight percent of the coal, between about 10 weight percent and about 15 weight percent of the coal, between about 15 weight percent and about 20 weight percent of the coal, between about 20 weight percent and about 25 weight percent of the coal between about 25 weight percent and about 30 weight percent of the coal, between about 30 weight percent and about 35 weight percent of the coal, between about 35 weight percent and about 40 weight percent of the coal, between about 40 weight percent and about 45 weight percent of the coal, between about 45 weight percent and about 50 weight percent of the coal, about 5 weight percent of the coal, about 10 weight percent of the coal, about 12 weight percent of the coal, about 14 weight percent of the coal, about 16 weight percent of the coal, about 18 weight percent of the coal, about 20 weight percent of the coal, about 22 weight percent of the coal, about 24 weight percent of the coal, about 25 weight percent of the coal, about 30 weight percent of the coal, about 35 weight percent of the coal, about 40 weight percent of the coal, about 45 weight percent of the coal, or about 50 weight percent of the coal.

The steps of one or more embodiments of the method 1500 also produced graphene oxide having dopants surprising and useful concentrations. The dopants in the reduced graphene oxide can include one or more of antimony, arsenic, barium, beryllium, boron, bromine, cadmium, chlorine, chromium, cobalt, copper, fluorine, lead, lithium, manganese, mercury, molybdenum, nickel, selenium, silver, strontium, thallium, tin, vanadium, zinc, and/or zirconium. In some cases, the one or more dopants present in the reduced graphene oxide can produce one or more types of defects in the final formed synthetic graphene, such as point defects. In some cases, these defects can enhance or otherwise modify the properties of the resultant graphene, such as the electrical properties. Accordingly, in some cases, a desired amount of one or more dopants in the graphene can result in graphene including a desired amount and/or distribution of defects, such as point defects. The one of more dopants or impurity atoms present in the graphene oxide can be present in concentrations that include up to about 0.1 atomic %, up to about 0.5 atomic %, up to about 1 atomic %, up to about 2 atomic %, up to about 5 atomic %, up to about 10 atomic %, or even up to about 15 atomic % or higher. In some cases the concentration of any of the one or more dopant or impurity atoms in the graphene oxide can include between about 0.1 atomic % and about 15 atomic %, between about 1 atomic % and about 10 atomic %, or between about 2 atomic % and about 5 atomic %, for example.

In some embodiments, the concentrations of one or more dopants in the reduced graphene oxide is controlled during the step(s) of thermally processing the coal, as described in greater detail in relation to block 1510 of the method 1500. In some embodiments, one or more dopants can be collected in vapor removed during the thermal processing of the coal and at least partially returned at a predetermined concentration to the coal or graphene oxide during one or more steps of the method 1500.

Also disclosed herein are various embodiments of a graphene composition including graphene and one or more dopants. The graphene the graphene composition can include one or more of graphene, graphene oxide, and/or reduced graphene oxide in accordance with the method 1500. The one or more dopants in the graphene composition can include at least one of antimony, arsenic, barium, beryllium, boron, bromine, cadmium, chlorine, chromium, cobalt, copper, fluorine, lead, lithium, manganese, mercury, molybdenum, nickel, selenium, silver, strontium, thallium, tin, vanadium, zinc, and/or zirconium.

The one of more dopants or impurity atoms present in the graphene can be present in concentrations that include up to about 0.1 atomic %, up to about 0.5 atomic %, up to about 1 atomic %, up to about 2 atomic %, up to about 5 atomic %, up to about 10 atomic %, or even up to about 15 atomic % or higher. In some cases the concentration of any of the one or more dopant or impurity atoms in the finally formed synthetic graphene include between about 0.1 atomic % and about 15 atomic %, between about 1 atomic % and about 10 atomic %, or between about 2 atomic % and about 5 atomic %, for example.

FIG. 17 is a diagram illustrating the flow of energy and coal in a processing facility for the production of one or more advanced carbon materials as described herein and according to some embodiments. As can be seen in FIG. 17, and as described herein, raw coal from a mine, such as the Brook Mine in Wyoming, can be processed to form carbon fiber and, optionally, one or more additional advanced carbon materials, such as construction materials, and/or activated carbon.

FIG. 18 is a diagram illustrating the process flow of raw coal, for example from a high wall coal mine, as it is processed according to the embodiments described herein to form various advanced carbon materials, such as activated carbon, graphene, materials for use in batteries, and building and construction materials, according to an embodiment. As illustrated, and according to some embodiments, processing of the coal can produce advanced carbon materials which can themselves be subjected to further processing to form other advanced carbon materials. Further, in some embodiments, the byproducts from the production of advanced carbon materials can themselves be subjected to further processing to produce other advanced carbon materials as described herein.

FIG. 19 is a diagram illustrating the process flow of raw coal to various advanced carbon materials according to the processes described herein, according to an embodiment. FIG. 8 further illustrates how, according to some embodiments, the advanced carbon material produced according to the processes described herein can be utilized as one or more components in high-value finished products, such as automotive grade CFRP, or graphene based biosensors.

### APPLICATIONS OF THE CARBON FIBERS

The carbon fibers produced during at least one of the methods 100, 200, 300, 400, or 1300 can be configured to be used in a variety of applications and, optionally, these methods can include using the carbon fibers in one or more of these application. In an embodiment, the carbon fibers can be configured to be used in a carbon fiber reinforced composite. For example, the carbon fibers can be combined with at least one matrix material to form the carbon fiber reinforced composite by any process known in the art or that can be developed in the future. The matrix material can comprise at least one of a polymer (*e.g.*, a resin made from coal), a metal, or a ceramic material. In some cases, the matrix can include any one of the additional advanced carbon materials disclosed herein. In some embodiments, the carbon fiber reinforced polymer can be formed into a desired structure. In some embodiments, the additional advanced carbon materials can be produced via the processes described herein and can be combined with one or more other materials via the processing facility to produce a composite material having a desired form.

In some embodiments, the carbon fiber reinforced composite can include metals or concrete including carbon fibers. In some examples, the carbon fiber reinforced composite can be 3D printed. In some examples, carbon fiber or the carbon fiber reinforced composites produced from coal as described herein can be used as rebar in concrete or can be used as other construction materials.

In an embodiment, the carbon fibers disclosed herein can be modified on site by a user in order to achieve the desired chemical or mechanical properties of the carbon fiber reinforced composite. In some cases, a first carbon fiber produced from coal by the processes described herein can be mixed with predetermined amount of a second carbon fiber that is different than the first carbon fibers. In an example, the first carbon fiber and the second carbon fibers are both formed from coal but exhibit different properties (*e.g.*, the first and second carbon fibers include different impurities). In an example, the first carbon fiber is formed from coal while the second carbon fibers are formed from oil. The amount of the first and second carbon fibers can be selected based on the desired properties of the carbon fiber reinforced composite formed from the first and second carbon fibers. For example, where a user desires a higher young's modulus, they can be directed to add a predetermined amount of a first carbon fiber produced from coal in order to raise the young's modulus of the second carbon fiber formed from oil.

In some cases, an advanced carbon material such as carbon fibers, include activated carbon and/or can be functionalized and tuned as desired. In some cases, any of the advanced carbon materials or carbon fibers described herein can be functionalized or used to form functionalized products. For example, in some cases an advanced carbon material produced according to the methods described herein can be functionalized to adsorb one or more predetermined materials, elements, and/or substances from water, the atmosphere, or other mediums as desired. In some cases, advanced carbon material produced according to the methods described herein can adsorb one or more types of rare earth elements produced by coal processing plants, such as the processing facilities described herein. In some cases, advanced carbon material produced according to the methods described herein can adsorb one or more valuable predetermined elements or compounds from sea water. In some cases, advanced carbon material produced according to the methods described herein can adsorb CO₂ from the ambient atmosphere.

### APPLICATIONS OF THE RESINS

The resins produced during at least one of the methods 500, 600, 700, 800, or 1300 include a plurality of monomer units that each include carbon and hydrogen and a selected amount of a remainder of one or more impurities that were initially present in the raw coal. The resins disclosed herein can be configured to be used in a variety of applications and, optionally, these methods can include using the resins in one or more of these application. In an embodiment, the resins can be configured to produce a polymer part or product. In such an embodiment, the resin can be subjected to further processing via the processing facility to produce the polymer part or product. For example, the resin can be used to produce sheets, extrusion, three dimensional structures using a molding process, or another other suitable process.

In an embodiment, the resins can be configured to produce carbon three dimensional ("3D") devices, such as via a 3D printing process. The 3D printing process can include forming the resin into meshes, hollow objects, solid objects, or other products. In some embodiments, a resin produced by the 3D printing processes described herein can be used in a continuous liquid interface production (CLIP) process as developed by Carbon3D, Inc., to produce a wide variety of polymer objects via the processing facility. Carbon3D, Inc. can use the resins disclosed herein to provide end products with selectable properties via the CLIP process. Accordingly, the processing facility can comprise one or more CLIP printers, such as the M2 Printer developed by Carbon3D, Inc., which can print polymer parts derived directly from coal as described herein. For example, resin produced from coal by the processes described herein can be used in the CLIP process via the processing facility to print dental products which are customized to an individual patient's anatomy. While custom dental products are provided as an example, almost any form of 3D object can be produced via the processes described herein. In an embodiment, the resins used in the 3D printing process can include one or more additives mixed therein. For example, the resins can include graphene mixed therein.

The 3D printed products including the resin can be specific to the needs of each customer. For example, such 3D printed products can have dimensions corresponding to the custom measurements of each customer. In an example, products 3D printed using resins produced via the processes described herein can include custom helmets, pads, or other protective clothing for use in sporting activities and/or combat. In some embodiments, one or more body parts of a user or customer can be scanned and the dimensions thereof can be incorporated into the custom design of the 3D printed product. In some cases, custom 3D printed products using resins produced via the processes described herein can include custom fitted horse shoes and/or saddles.

In an embodiment, the resins disclosed herein can be modified on site by a user in order to achieve the desired chemical or mechanical properties of the final 3D printed product. For example, a first resin produced from coal by the processes described herein can have a first physical property in a final cured state, such as a first young's modulus. Where a user desires to adjust this property, the user can be direct to add a predetermined amount of a second resin or other additional advanced carbon materials produced from coal to the first resin, where the second resin and amount thereof can be selected based on the nature of the adjustment to the first physical property of the first resin produced from coal. For example, where a user desires a higher young's modulus, they can be directed to add a predetermined amount of a second resin produced from coal in order to raise the young's modulus of the first resin. In some cases, this addition can be carried out automatically based on the desired cured material properties of the 3D printed object.

In an embodiments, the material properties of a 3D printed objected can be varied throughout the volume of the object by utilizing two or more resins produced form coal according to the processes described herein that are UV activatable, where each of the two or more resins has different material properties when cured and each is activated by a different wavelength of UV light. For example, in some embodiments a first resin produced from coal having a first material property, such as a first stiffness, can be activated by a first wavelength of UV light. A second resin produced from coal having a second, different material property, such as a second stiffness, can be activated by a second, different wavelength of UV light. The resins can be activated by UV light to form the product as it is being 3D printed, and the wavelength of the UV light can be adjusted to vary the material properties of the product as it is being printed. For example, UV light having a wavelength corresponding to the activation wavelength of the first resign can be used for those parts of the product where the material properties of the first resin are desired. The wavelength can then be varied or changed to the activation wavelength of the second resin so that the material properties of the printed object vary from the properties of the first resin to the properties of the second.

In an embodiment, the resins can be configured to be used in an additive manufacturing process. In an embodiment, the resins produced during block 160 can be configured to form at least a portion of a matrix of a composite, such as a matrix of a carbon fiber reinforced composite.

### APPLICATIONS OF GRAPHENE

The advanced carbon material or materials produced via the methods and processes described herein can be used in a wide variety of applications. In some cases, the advanced carbon materials produced via the processing facility described herein can be subjected to further processing to produce objects, devices, and other products from the advanced carbon materials. In other embodiments, the advanced carbon materials can be distributed to other production facilities for use. Importantly, in some embodiments, the processes described herein can produce two or more types of advanced carbon materials which can be combined at the processing facility into further products.

In some cases, an advanced carbon material, such as graphene, can be functionalized and tuned as desired. In some cases, any of the advanced carbon materials or forms of graphene described herein can be used to adsorb desired elements or compounds or to form functionalized products. For example, in some cases an advanced carbon material produced according to the methods described herein can be functionalized to adsorb one or more predetermined materials, elements, and/or substances from water, the atmosphere, or other mediums as desired. In some cases, advanced carbon material produced according to the methods described herein can adsorb one or more types of rare earth elements produced by coal processing plants, such as the processing facilities described herein. In some cases, advanced carbon material produced according to the methods described herein can adsorb one or more valuable predetermined elements or compounds from sea water. In some cases, advanced carbon material produced according to the methods described herein can adsorb CO₂ from the ambient atmosphere.

In some cases, graphene formed according to the methods described herein can be used as a support or substrate for one or more elements or compounds. In some cases, these elements or compounds can be utilized to perform desired applications or to have predetermined electrical, chemical, and/or physical properties. For example, in some cases graphene formed according to the methods described herein can be arranged in layers to serve as a support, scaffold, or substrate for one or more enzymes that can serve any number of desired functions. In some cases, enzymes supported by a graphene support structure can adsorb CO₂, for example from the ambient environment, to be converted into a secondary product, such as methane.

In some cases, and as described herein, a first amount of pitch can be treated to produce a first advanced carbon material, and a second amount of pitch can be treated to produce a second, different advanced carbon material. In some cases, the first and second advanced carbon materials can be combined to form a new material and/or produce. For example, the first advanced carbon material can comprise carbon fibers and the second advanced carbon can comprise a polymer or resin. The first and second advanced carbon materials can then be combined via the processing facility to produce carbon fiber reinforced polymer by any process known in the art or that can be developed in the future. In some cases, the carbon fiber reinforced polymer can be formed into a desired structure, for example a part or product as specified by a customer. In some embodiments, an advanced carbon material can be produced via the processes described herein and can be combined with one or more other materials via the processing facility to produce a composite material having a desired form. For example, the advanced carbon material can comprise carbon nanotubes. These carbon nanotubes can then be metalized via the processing facility to produce a carbon nanotube metal matrix composite. In some cases, the carbon nanotube metal matrix composite can comprise a bulk material, however in some other cases the carbon nanotube metal matrix composite can be formed in a desired shape. In some cases, a carbon nanotube metal matrix composite can be formed by any processes known in the art or that can be developed in the future, such as via powder metallurgy processes, electrochemical processes, melt processes, and others.

In some embodiments where an advanced carbon material can comprise a resin, the resin can be subjected to further processing via the processing facility to produce a polymer part or product. In some cases, the resin can be used in a three dimensional printing process to form polymer structures such as meshes, hollow objects, solid objects, or other products. In some embodiments, a resin produced by the processes described herein can be used in a continuous liquid interface production (CLIP) process as developed by Carbon3D, Inc., to produce a wide variety of polymer objects via the processing facility. Carbon3D, Inc. uses a number of different resins to provide end products with selectable properties via the CLIP process. In some embodiments, the methods and processes described herein can be used to produce any of the resins used in the CLIP process, for example, polyurethane resins, ester resins, epoxy resins, and others. Accordingly, the processing facility can comprise one or more CLIP printers, such as the M2 Printer developed by Carbon3D, Inc., which can print polymer parts derived directly from coal as described herein. For example, in some embodiments, advanced carbon materials produced from coal by the processes described herein can be used in the CLIP process via the processing facility to print dental products which are customized to an individual patient's anatomy. While custom dental products are provided as an example, almost any form of three-dimensional object can be produced via the processes described herein.

For example, in some embodiments where an advanced carbon material comprises one or more resins for use in 3D printing, such resins can be used to 3D print products specific to the needs of each customer. For example, such 3D printed products can have dimensions corresponding to the custom measurements of each customer. In some examples, products 3D printed using resins produced via the processes described herein can include custom helmets, pads, or other protective clothing for use in sporting activities and/or combat. In some embodiments, one or more body parts of a user or customer can be scanned and the dimensions thereof can be incorporated into the custom design of the 3D printed product. In some cases, custom 3D printed products using resins produced via the processes described herein can include custom fitted horse shoes and/or saddles.

In some embodiments, the advanced carbon material resins used in a 3D printing process can be modified on site by a user in order to achieve the desired chemical or mechanical properties of the final 3D printed product. For example, a first resin produced from coal by the processes described herein can have a first physical property in a final cured state, such as a first young's modulus. Where a user desires to adjust this property, they can be direct to add a predetermined amount of a second resin or other advanced carbon material produced from coal to the first resin, where the second resin and amount thereof can be selected based on the nature of the adjustment to the first physical property of the first resin produced from coal. For example, where a user desires a higher young's modulus, they can be directed to add a predetermined amount of a second resin produced from coal in order to raise the young's modulus of the first resin. In some cases, this addition can be carried out automatically based on the desired cured material properties of the 3D printed object.

In some embodiments, the material properties of a 3D printed objected can be varied throughout the volume of the object by utilizing two or more resins produced form coal according to the processes described herein that are UV activatable, where each of the two or more resins has different material properties when cured and each is activated by a different wavelength of UV light. For example, in some embodiments a first resin produced from coal having a first material property, such as a first stiffness, can be activated by a first wavelength of UV light. A second resin produced from coal having a second, different material property, such as a second stiffness, can be activated by a second, different wavelength of UV light. The resins can be activated by UV light to form the product as it is being 3D printed, and the wavelength of the UV light can be adjusted to vary the material properties of the product as it is being printed. For example, UV light having a wavelength corresponding to the activation wavelength of the first resign can be used for those parts of the product where the material properties of the first resin are desired. The wavelength can then be varied or changed to the activation wavelength of the second resin so that the material properties of the printed object vary from the properties of the first resin to the properties of the second.

In some embodiments, graphene or other advanced carbon materials can be used in the production of graphene or functionalized graphene ink. Graphene inks can be generated from graphene in accordance with the present disclosure and printed onto a source or poured into channel to produce a graphene-based array.

In some embodiments, a first advanced carbon material produced by the processes described herein can be used in a subsequent such process to produce a second, different advanced carbon material. For example, the first advanced carbon material can comprise molecular graphene membranes. The molecular graphene membranes can then be used in the processes described herein to chemically separate products of pyrolysis or liquefaction processes to produce resins. In some cases, this form of chemical separation via graphene membranes can be more thermally efficient than other separation processes that are typically employed. These resins in turn can be used in the CLIP process, for example to print a mesh.

In some embodiments where an advanced carbon material comprises graphene, the graphene can be subjected to further treatment via the processing facility to form, for example, a graphene sensor. In some embodiments, a graphene sensor can include graphene ink. For example, a graphene sensor can include undoped graphene ink as the circuit to an area or point that contains doped graphene. In such an embodiment, the doped graphene area or point can detect, and the undoped graphene ink can carry a signal to a computing device or user interface. In some embodiments, a graphene sensor can include a flake-based graphene sensor, such as a flake-based graphene biosensor. In such embodiments, some graphene flakes can be doped, and some graphene flakes and/or graphene ink acting as a circuit will not be doped. These graphene sensors can be used as disposable chips for detecting diseases via a handheld device. The graphene sensor can be able to immediately detect diseases, such as Lyme disease or the zika virus from a patient's blood, urine, saliva, or other bodily fluids or biological material, thereby eliminating any need to store blood samples for transportation to a lab. Further, the processes described herein can also be used to print the body of the hand-held device, and/or a consumable or attachment, such as a microfluidic chamber, for example via the CLIP process.

In some embodiments, advanced carbon materials produced by the processes described herein can be used in a wide variety of other applications. For example, the advanced carbon material can comprise a carbon foam which can be used as an electrode in a lithium ion battery. More specifically, graphene or reduced graphene oxide produced by the processes described herein can be used in an electrode in a battery. The graphene or reduced graphene oxide produced by the processes described herein used in an electrode in a battery can include one or more of the dopants described herein at any of the amounts described herein. In some cases, the advanced carbon material can comprise activated carbon that can be used in an atmospheric CO₂ recapture process. In some cases, the atmospheric CO₂ recapture process can be carried out via the processing facility and captured CO₂ can be used in the processes described herein.

In some embodiments, advanced carbon materials, such as graphene, formed according to the processes described herein can be used to produce solar panels. In some cases these solar panels can have greater efficiencies than other conventionally produced solar panels. In some embodiments, advanced carbon materials formed according to the processes described herein can be used as precursors in electrospinning processes. For example, advanced carbon materials can be used to electrospin scaffolds or other structures having micron level resolution. In some cases the advanced carbon materials used in electrospinning can be biomaterials produced from coal according to the processes described herein. In some embodiments advanced carbon materials can be used to produce gels, for example medical grade gels such as hydrogels or silicone gels.

In some embodiments, one or more advanced carbon materials produced from coal according to the processes described herein can be used as automotive grade materials in the production of cars, trucks, or other automobiles. For example, carbon fibers, resins, and/or CFRPs can be used as automotive frames, structural components, body panels, engine blocks, and/or other components. In some cases, the components can be 3D printed and can be custom designed according to a user's preferences.

In some embodiments, one or more advanced carbon materials produced from coal according to the processes described herein can be used to form products for use in chemical or biological processes, such chromatography columns, membranes, and filters. In some cases, chromatography columns, membranes, and/or filters can be 3D printed from one or more advanced carbon materials produced from coal according to the processes described herein. In some cases, the chromatography columns, membranes, and/or filters can be used to isolate or remove antibodies, bacteria, parasites, and/or heavy metals from various solutions.

In some embodiments, reinforced graphene can be produced in accordance with the disclosures of the present application. The reinforced graphene includes graphene and nanotubes. Advantageously, graphene having one or more dopants described herein can chemically bond with nanotubes having another dopant. This chemical bond can form between the carbon nanotube and the graphene sheet, and can allow the repetitive layers of graphene sheets to form a graphene filter. Graphene filters in accordance with the present disclosure also can be formed using chemical linkers. For example, different dopant metals on each end of chemical linkers allow the chemical linkers to be directional and control the space between the graphene sheets, the graphene sheets being pre-doped with reactive dopants that are different to the chemical linkers.

In some embodiments, one or more advanced carbon materials produced from coal according to the processes described herein can be used to form circuit boards. For example, a carbon foam produced from coal as described herein can be 3D printed to form a circuit board. In some cases a carbon foam circuit board can have superior electrical and thermal properties to typical printed circuit boards. In some embodiments, one or more advanced carbon materials produced from coal according to the processes described herein can be synthetic graphene and can be used in a variety of electronic applications, for example in forming quantum dots and in computer chips. In some cases, graphene can be used to produce biosensors that can be capable of isolating and/or identifying any number of biologically active molecules or substances, such as disease biomarkers or viruses.

In some embodiments, one or more advanced carbon materials produced from coal according to the processes described herein can include composite materials, such as metals or concrete including carbon fibers, graphene, or other advanced carbon materials. In some examples, metal including one or more advanced carbon materials such as graphene, carbon fibers, or carbon nanotubes can be 3D printed. In some examples, carbon fiber or CFRPs produced from coal as described herein can be used as rebar in concrete or can be used as other construction or building materials.

### ADDITIONAL ADVANCED CARBON MATERIALS

As previously discussed, the methods and processes described herein can be used to produce carbon fiber and, optionally, one or more additional advanced carbon materials from coal. As used herein, the term additional advanced carbon materials can refer to one or more non-carbon fiber materials comprising carbon. The additional advanced carbon materials can be formed from at least one of the pitch (*e.g.*, the portions of the pitch not used to form the carbon fiber) or one or more of the byproducts produced during the methods disclosed herein. In an example, the methods disclosed herein can be utilized to produce an amount of a carbon fiber and, subsequently, an amount of at least one additional advanced carbon material. In an example, the method 100 can be utilized to produce an amount of the carbon fiber and an amount of the at least one second advanced carbon material simultaneously, for instance, via parallel processing utilizing the methods disclosed herein. In some embodiments, the additional advanced carbon materials can be a resin, polymer, or other hydrocarbon material.

In an embodiment, the methods disclosed herein can include forming resin from the pitch or byproducts produced during the methods disclosed herein. For example, the resins formed from at least the pitch can include polyacrylonitrile, polyurethane resins, cyanate ester resins, epoxy resins, methacrylate resins, polyester resins, fused aromatic ring structures (*e*.*g*., polycyclic aromatic hydrocarbons), and other suitable resins. In an example, the resins formed according to the processes disclosed herein can include resins formed from resin precursors. Examples of resin precursors include C2 compounds (*e*.*g*., ethane, ethylene, acetylene, etc.), C3 compounds (*e.g.*, propylene, cyclopropane, propene, etc.), C4 compounds (*e.g.*, butadiene, butane, t-butanol, etc.), B compounds, T compounds, halogen compounds, phenols, or any other suitable resin precursor.

In an embodiment, the pitch can be treated to product at least one of the resins disclosed, for example, by forming the pitch into one or more organic compounds (*e.g.*, olefins, other resin precursors, other polymers) and polymerizing the one or more organic compounds. In an embodiment, the coal liquid extracts can be treated at the processing facility to form at least one of the resins disclosed herein. In an embodiment, forming the resins disclosed herein can include adding one or more additives to the pitch, the byproducts, or the resin if the resin has already been formed. The additives added to the pitch, byproducts, or the resin can include one or more photo activators (*e*.*g*., one or more ultraviolet light activates) or one or more thermal activators.

In such an embodiment, the resin can be subjected to further processing via the processing facility to produce a polymer part or product. For example, the resin can be used to produce sheets, extrusion, three dimensional structures using a molding process, or another other suitable process. In an embodiment, the resins can be configured to produce carbon three dimensional ("3D") devices, such as via a 3D printing process. The 3D printing process can include forming the resin into meshes, hollow objects, solid objects, or other products. In some embodiments, a resin produced by the 3D printing processes described herein can be used in a continuous liquid interface production (CLIP) process as developed by Carbon3D, Inc. In an embodiments, the material properties of a 3D printed objected can be varied throughout the volume of the object by utilizing two or more resins produced form coal according to the processes described herein, where each of the two or more resins has different material properties when cured and/or each is activated by a different stimuluses. In an embodiment, the resins can be configured to be used in an additive manufacturing process.

In an embodiment, the methods disclosed herein can include processing at least one of the pitch or one or more of the byproducts to form synthetic graphite. In some cases, the synthetic graphite can be subjected to further processing to form synthetic graphene. For example, at least one of the pitch or one or more of the byproducts can be treated by exposure to heat, elevated pressure, and/or one or more catalysts to form synthetic graphite. As used herein, the term synthetic graphite is used to refer to any graphite material produced from a precursor material (*e.g.*, any graphite material that does not occur naturally in the earth). In an embodiment, the methods disclosed herein can further comprise treating or processing the synthetic graphite, via the processing facility, to form synthetic graphene. As used herein, synthetic graphene refers to any graphene material produced or derived from synthetically formed graphite. For example, the synthetic graphite can be subjected to mechanical exfoliation to produce synthetic graphene. As described herein, a desired amount of one or more impurities can remain in the beneficiated coal and can thereby be incorporated into the synthetic graphene produced in order to adjust the chemical, electrical, and/or physical properties of the synthetic graphene.

In an embodiment, the methods disclosed herein can include subjecting any char produced during the methods to further processing to produce one or more additional advanced carbon material, such as activated carbon. In an example, processing the char can include carbonizing or heating the char (*e.g.*, in a kiln). The char can then be activated via a physical activation process or a chemical activation process. Physical activation can comprise heating the char in an atmosphere comprising argon and/or nitrogen, or heating the char in an oxidizing atmosphere. Chemical activation can comprise impregnating the char with one or more chemicals, such as an acid, a base, or a salt. In some cases, chemical activation can further comprise carbonizing or heating the impregnated char to activate it. In some cases, chemical activation can require lower temperatures and less energy than physical activation. Further, in some cases, chemical byproducts produced by the method 100 can be utilized during the chemical activation process.

In some cases, the additional advanced carbon materials can comprise primarily carbon atoms. In some embodiments, the additional advanced carbon materials can comprise at least one carbon foams, or pyrolyzed carbon. In some embodiments, the additional advanced carbon materials can comprise one or more allotropes of carbon, such as any allotropes of carbon that are known in the art or that can be developed in the future. In some cases, the additional advanced carbon materials can comprise single-walled carbon nanotubes, multi-walled carbon nanotubes, carbon megatubes, carbon nanoribbons, carbon nanobuds, graphene, graphite nano-platelets, quantum dots, and fullerenes (*e*.*g*., buckminsterfullerene or multi-cored fullerenes).

In some cases, the additional advanced carbon materials can comprise elements in addition to carbon and can be, for example, a polymer or other hydrocarbon material. For example, the additional advanced carbon materials can comprise thermoset or thermoplastic polymers. In some cases, the additional advanced carbon materials can comprise a polyester, vinyl ester, or nylon polymer.

In some cases, the additional advanced carbon materials can comprise a biologically useful material or biopolymer. In other words, the additional advanced carbon materials can comprise a material including carbon that is at least one of used in biological systems or organisms, biocompatible, or is typically be produced by a biological organism. For example, the additional advanced carbon materials can be a protein, amino acid, nucleic acid, collagen, chitosan, sugar, or other biological material. In some cases, the additional advanced carbon materials can comprise a porous material, such as a membrane, for use in a biological and/or chemical process. For example, the additional advanced carbon materials can comprise perforated graphene.

### APPLICATIONS OF THE ADDITIONAL ADVANCED CARBON MATERIALS

The additional advanced carbon materials produced via the methods and processes described herein can be used in a wide variety of applications. In some cases, the additional advanced carbon materials produced via the processing facility described herein can be subjected to further processing to produce objects, devices, and other products from the additional advanced carbon materials. In other embodiments, the additional advanced carbon materials can be distributed to other production facilities for use. Importantly, in some embodiments, the processes described herein can produce two or more types of additional advanced carbon materials which can be combined at the processing facility into further products.

In such an embodiment, the resin can be subjected to further processing via the processing facility to produce a polymer part or product. For example, the resin can be used to produce sheets, extrusion, three dimensional structures using a molding process, or another other suitable process. In an embodiment, the resins can be configured to produce carbon three dimensional ("3D") devices, such as via a 3D printing process. The 3D printing process can include forming the resin into meshes, hollow objects, solid objects, or other products. In some embodiments, a resin produced by the 3D printing processes described herein can be used in a continuous liquid interface production (CLIP) process as developed by Carbon3D, Inc. In an embodiments, the material properties of a 3D printed objected can be varied throughout the volume of the object by utilizing two or more resins produced form coal according to the processes described herein, where each of the two or more resins has different material properties when cured and/or each is activated by a different stimuluses. In an embodiment, the resins can be configured to be used in an additive manufacturing process.

In some embodiments, a first additional advanced carbon material produced by the processes described herein can be used in a subsequent such process to produce a second, different additional advanced carbon material. For example, the first additional advanced carbon material can comprise molecular graphene membranes. The molecular graphene membranes can then be used in the processes described herein to chemically separate products of pyrolysis or liquefaction processes to produce carbon fibers. In some cases, this form of chemical separation via graphene membranes can be more thermally efficient than other separation processes that are typically employed. These carbon fibers in turn can be used in the CLIP process, for example to print a mesh.

In some embodiments where the additional advanced carbon materials comprise graphene, the graphene can be subjected to further treatment via the processing facility to form, for example, a graphene sensor. These graphene sensors can be used as disposable chips for detecting diseases via a handheld device. The graphene sensor can be able to immediately detect diseases, such as Lyme disease or the zika virus from a patient's blood, urine, saliva, or other bodily fluids or biological material, thereby eliminating any need to store blood samples for transportation to a lab. Further, the processes described herein can also be used to print the body of the hand-held device, and/or a consumable or attachment, such as a microfluidic chamber, for example via the CLIP process.

In some embodiments, additional advanced carbon materials produced by the processes described herein can be used in a wide variety of other applications. For example, the additional advanced carbon materials can comprise a carbon foam which can be used as an electrode in a lithium ion battery. In some cases, the additional advanced carbon materials can comprise activated carbon that can be used in an atmospheric CO₂ recapture process. In some cases, the atmospheric CO₂ recapture process can be carried out via the processing facility and captured CO₂ can be used in the processes described herein.

In some embodiments, additional advanced carbon materials, such as graphene, formed according to the processes described herein can be used to produce solar panels. In some cases these solar panels can have greater efficiencies than other conventionally produced solar panels. In some embodiments, additional advanced carbon materials formed according to the processes described herein can be used as precursors in electrospinning processes. For example, additional advanced carbon materials can be used to electrospin scaffolds or other structures having micron level resolution. In some cases the additional advanced carbon materials used in electrospinning can be biomaterials produced from coal according to the processes described herein. In some embodiments additional advanced carbon materials can be used to produce gels, for example medical grade gels such as hydrogels or silicone gels.

In some embodiments, one or more additional advanced carbon materials produced from coal according to the processes described herein can be used as automotive grade materials in the production of cars, trucks, or other automobiles. For example, carbon fiber reinforced composites including resins formed from coal can be used as automotive frames, structural components, body panels, engine blocks, and/or other components. In some cases, the components can be 3D printed and can be custom designed according to a user's preferences.

In some embodiments, one or more additional advanced carbon materials produced from coal according to the processes described herein can be used to form products for use in chemical or biological processes, such chromatography columns, membranes, and filters. In some cases, chromatography columns, membranes, and/or filters can be 3D printed from one or more additional advanced carbon materials produced from coal according to the processes described herein. In some cases, the chromatography columns, membranes, and/or filters can be used to isolate or remove antibodies, bacteria, parasites, and/or heavy metals from various solutions.

In some embodiments, one or more additional advanced carbon materials produced from coal according to the processes described herein can be used to form circuit boards. For example, a carbon foam produced from coal as described herein can be 3D printed to form a circuit board. In some cases a carbon foam circuit board can have superior electrical and thermal properties to typical printed circuit boards. In some embodiments, one or more additional advanced carbon materials produced from coal according to the processes described herein can be synthetic graphene and can be used in a variety of electronic applications, for example in forming quantum dots and in computer chips. In some cases, graphene can be used to produce biosensors that can be capable of isolating and/or identifying any number of biologically active molecules or substances, such as disease biomarkers or viruses.

Unless otherwise indicated, all numbers or expressions, such as those expressing dimensions, physical characteristics, etc., used in the specification (other than the claims) are understood as modified in all instances by the term "approximately." At the very least, and not as an attempt to limit the application of the doctrine of equivalents to the claims, each numerical parameter recited in the specification or claims which is modified by the term "approximately" should at least be construed in light of the number of recited significant digits and by applying ordinary rounding techniques. Further, the terms "have," "has," "having," "include," "includes," and "including" should be interpreted as being both open ended and closed end terms.

In addition, all ranges disclosed herein are to be understood to encompass and provide support for claims that recite any and all subranges or any and all individual values subsumed therein. For example, a stated range of 1 to 10 should be considered to include and provide support for claims that recite any and all subranges or individual values that are between and/or inclusive of the minimum value of 1 and the maximum value of 10; that is, all subranges beginning with a minimum value of 1 or more and ending with a maximum value of 10 or less (e.g., 5.5 to 10, 2.34 to 3.56, and so forth) or any values from 1 to 10 (e.g., 3, 5.8, 9.9994, and so forth).

## Claims

1. A method of producing reduced graphene oxide from coal, the method comprising:
thermally processing coal at a temperature of at least about 148.89°C [300° F];
oxidizing the thermally processed coal to form coal oxide; and
forming reduced graphene oxide from the coal oxide; and
selectively maintaining a predetermined concentration of one or more impurity atoms in the reduced graphene oxide, wherein the one or more impurity atoms comprise one or more of cadmium, selenium, boron, nitrogen, and silicon.

2. The method of claim 1, wherein forming reduced graphene oxide from the coal oxide comprises:
centrifuging the coal oxide;
collecting precipitate from the coal oxide after centrifuging, the precipitate comprising graphene oxide; and
reducing the graphene oxide to form reduced graphene oxide.

3. The method of claim 2, wherein oxidizing the coal to form a coal oxide comprises mixing the coal with at least one of sulfuric acid, nitric acid, or potassium permanganate, or hydrogen peroxide to form the coal oxide.

4. The method of claim 3, wherein mixing the coal with at least one of sulfuric acid, nitric acid, potassium permanganate, or hydrogen peroxide to form the coal oxide comprises:
mixing the coal with at least one of sulfuric acid and nitric acid;
stirring the coal mixed with at least one of the sulfuric acid and the nitric acid;
mixing potassium permanganate to the coal mixed with at least one of the sulfuric acid and the nitric acid;
stirring the coal mixed with the potassium permanganate and at least one of the sulfuric acid and the nitric acid;
diluting, with water, the coal mixed with the potassium permanganate and at least one of the sulfuric acid and the nitric acid to form a solution;
mixing the solution with hydrogen peroxide;
performing a first centrifugation of the solution mixed with the hydrogen peroxide; and
after performing the first centrifugation, separating a supernatant of the solution mixed with the hydrogen peroxide from precipitate of the solution mixed with the hydrogen peroxide, the supernatant comprising the coal oxide.

5. The method of claim 2, further comprising diluting, with water, the coal oxide before centrifuging the coal oxide.

6. The method of claim 2, wherein reducing the graphene oxide to form reduced graphene oxide comprises:
sonicating the graphene oxide; and
hydrothermally treating the graphene oxide in a par reactor after sonicating the graphene oxide.

7. The method of claim 1, wherein thermally processing coal at a temperature of at least about 148.89°C [300°F] comprises:
heating the coal to a first temperature not to exceed 176.67°C [350°F];
transferring the coal to a mercury removal reactor;
heating the coal in the mercury removal reactor to a second temperature of at least 260.00°C [500°F]; and
contacting the coal with an inert gas to remove at least a portion of mercury present in the coal.

8. The method of claim 1, wherein forming reduced graphene oxide from the coal oxide comprises forming the reduced graphene oxide from the coal oxide at a reduced graphene oxide yield rate of between approximately 10 weight % and approximately 20 weight % of the coal.

9. The method of claim 1, wherein the predetermined concentration of one or more impurity atoms is between 0.1 atomic % and 15 atomic %.

10. The method of claim 1, wherein the predetermined concentration of one or more impurity atoms is between 0.1 atomic % and 10 atomic %.

11. The method of claim 1, wherein the one or more impurity atoms occur naturally in the amount of coal.

12. The method of claim 1, further comprising collecting one or more impurity atoms in vapor removed while thermally processing coal at a temperature of at least about 148.89°C [300°F]; and
incorporating a predetermined amount of the collected one or more impurity atoms into the reduced graphene oxide.

13. A method of producing an electrode of a battery ,_comprising forming the reduced graphene oxide of claim 1 into the electrode of the battery.

## Patentansprüche

1. Verfahren zur Herstellung von reduziertem Graphenoxid aus Kohle, wobei das Verfahren Folgendes umfasst:
thermisches Verarbeiten von Kohle bei einer Temperatur von mindestens etwa 148,89 °C [300 °F];
Oxidieren der thermisch verarbeiteten Kohle, um Kohlenoxid zu bilden; und
Bilden von reduziertem Graphenoxid aus dem Kohlenoxid; und
selektives Aufrechterhalten einer vorbestimmten Konzentration von einem oder mehreren Fremdatomen in dem reduzierten Graphenoxid, wobei das eine oder die mehreren Fremdatome eines oder mehrere von Cadmium, Selen, Bor, Stickstoff und Silicium umfassen.

2. Verfahren nach Anspruch 1, wobei das Bilden von reduziertem Graphenoxid aus dem Kohlenoxid Folgendes umfasst:
Zentrifugieren des Kohlenoxids;
Sammeln von Niederschlag aus dem Kohlenoxid nach dem Zentrifugieren, wobei der Niederschlag Graphenoxid umfasst; und
Reduzieren des Graphenoxids, um reduziertes Graphenoxid zu bilden.

3. Verfahren nach Anspruch 2, wobei das Oxidieren der Kohle, um ein Kohlenoxid zu bilden, das Mischen der Kohle mit mindestens einem von Schwefelsäure, Salpetersäure oder Kaliumpermanganat oder Wasserstoffperoxid umfasst, um das Kohlenoxid zu bilden.

4. Verfahren nach Anspruch 3, wobei das Mischen der Kohle mit mindestens einem von Schwefelsäure, Salpetersäure, Kaliumpermanganat oder Wasserstoffperoxid, um das Kohlenoxid zu bilden, Folgendes umfasst:
Mischen der Kohle mit mindestens einer von Schwefelsäure und Salpetersäure;
Rühren der Kohle, die mit mindestens einer der Schwefelsäure und der Salpetersäure gemischt ist;
Mischen von Kaliumpermanganat in die Kohle, die mit mindestens einer der Schwefelsäure und der Salpetersäure gemischt ist;
Rühren der Kohle, die mit dem Kaliumpermanganat und mindestens einer der Schwefelsäure und der Salpetersäure gemischt ist;
Verdünnen, mit Wasser, der Kohle, die mit dem Kaliumpermanganat und mindestens einer der Schwefelsäure und der Salpetersäure gemischt ist, um eine Lösung zu bilden;
Mischen der Lösung mit Wasserstoffperoxid;
Durchführen einer ersten Zentrifugation der Lösung, die mit dem Wasserstoffperoxid gemischt ist; und
nach dem Durchführen der ersten Zentrifugation Abtrennen eines Überstands der Lösung, die mit dem Wasserstoffperoxid gemischt ist, vom Niederschlag der Lösung, die mit dem Wasserstoffperoxid gemischt ist, wobei der Überstand das Kohlenoxid umfasst.

5. Verfahren nach Anspruch 2, das weiter das Verdünnen, mit Wasser, des Kohlenoxids vor dem Zentrifugieren des Kohlenoxids umfasst.

6. Verfahren nach Anspruch 2, wobei das Reduzieren des Graphenoxids, um reduziertes Graphenoxid zu bilden, Folgendes umfasst:
Ultraschallbehandlung des Graphenoxids; und
hydrothermische Behandlung des Graphenoxids in einem Par-Reaktor nach Ultraschallbehandlung des Graphenoxids.

7. Verfahren nach Anspruch 1, wobei eine thermische Verarbeitung von Kohle bei einer Temperatur von mindestens etwa 148,89 °C [300 °F] Folgendes umfasst:
Erhitzen der Kohle auf eine erste Temperatur, die nicht 176,67 °C [350 °F] zu überschreiten hat;
Überführen der Kohle zu einem Reaktor zur Quecksilberentfernung;
Erhitzen der Kohle in dem Reaktor zur Quecksilberentfernung auf eine zweite Temperatur von mindestens 260,00 °C [500 °F]; und
Inkontaktbringen der Kohle mit einem Inertgas, um zumindest einen Teil an Quecksilber, das in der Kohle vorliegt, zu entfernen.

8. Verfahren nach Anspruch 1, wobei das Bilden von reduziertem Graphenoxid aus dem Kohlenoxid das Bilden des reduzierten Graphenoxids aus dem Kohlenoxid bei einer Ausbeute von reduziertem Graphenoxid von zwischen ungefähr 10 Gewichts-% und ungefähr 20 Gewichts-% der Kohle umfasst.

9. Verfahren nach Anspruch 1, wobei die vorbestimmte Konzentration von einem oder mehreren Fremdatomen zwischen 0,1 Atom-% und 15 Atom-% beträgt.

10. Verfahren nach Anspruch 1, wobei die vorbestimmte Konzentration von einem oder mehreren Fremdatomen zwischen 0,1 Atom-% und 10 Atom-% beträgt.

11. Verfahren nach Anspruch 1, wobei das eine oder die mehreren Fremdatome in der Menge von Kohle natürlich vorkommen.

12. Verfahren nach Anspruch 1, das weiter Folgendes umfasst: Sammeln von einem oder mehreren Fremdatomen im Dampf, der während der thermischen Verarbeitung von Kohle bei einer Temperatur von mindestens etwa 148,89 °C [300 °F] entfernt wird; und
Einbauen einer vorbestimmten Menge des/der gesammelten Fremdatoms/Fremdatome in das reduzierte Graphenoxid.

13. Verfahren zur Herstellung einer Elektrode einer Batterie, das das Bilden des reduzierten Graphenoxids nach Anspruch 1 in der Elektrode der Batterie umfasst.

## Revendications

1. Méthode de production d'oxyde de graphène réduit à partir de charbon, la méthode comprenant :
le traitement thermique de charbon à une température d'au moins environ 148,89°C [300°F] ;
l'oxydation du charbon traité thermiquement afin de former de l'oxyde de charbon ; et
la formation d'oxyde de graphène réduit à partir de l'oxyde de charbon ; et
le maintien de manière sélective d'une concentration prédéterminée d'un ou plusieurs atomes d'impureté dans l'oxyde de graphène réduit, où l'un ou plusieurs atomes d'impureté comprennent un ou plusieurs atomes parmi le cadmium, le sélénium, le bore, l'azote et le silicium.

2. Méthode selon la revendication 1, dans laquelle la formation d'oxyde de graphène réduit à partir de l'oxyde de charbon comprend :
la centrifugation de l'oxyde de charbon ;
la récupération du précipité de l'oxyde de charbon après centrifugation, le précipité comprenant de l'oxyde de graphène ; et
la réduction de l'oxyde de graphène afin de former de l'oxyde de graphène réduit.

3. Méthode selon la revendication 2, dans laquelle l'oxydation du charbon afin de former un oxyde de charbon comprend le mélange du charbon avec au moins l'un parmi l'acide sulfurique, l'acide nitrique, ou le permanganate de potassium, ou le peroxyde d'hydrogène, afin de former l'oxyde de charbon.

4. Méthode selon la revendication 3, dans laquelle le mélange du charbon avec au moins l'un parmi l'acide sulfurique, l'acide nitrique, le permanganate de potassium, ou le peroxyde d'hydrogène, afin de former l'oxyde de charbon, comprend :
le mélange du charbon avec au moins l'un parmi l'acide sulfurique et l'acide nitrique ;
l'agitation du charbon mélangé avec au moins l'un parmi l'acide sulfurique et l'acide nitrique ;
le mélange du permanganate de potassium avec le charbon mélangé avec au moins l'un parmi l'acide sulfurique et l'acide nitrique ;
l'agitation du charbon mélangé avec le permanganate de potassium et au moins l'un parmi l'acide sulfurique et l'acide nitrique ;
la dilution, par de l'eau, du charbon mélangé avec le permanganate de potassium et au moins l'un parmi l'acide sulfurique et l'acide nitrique, afin de former une solution ;
le mélange de la solution avec du peroxyde d'hydrogène ;
la mise en oeuvre d'une première centrifugation de la solution mélangée avec le peroxyde d'hydrogène ; et
après avoir mis en oeuvre la première centrifugation, la séparation d'un surnageant de la solution mélangée avec le peroxyde d'hydrogène et d'un précipité de la solution mélangée avec le peroxyde d'hydrogène, le surnageant comprenant l'oxyde de charbon.

5. Méthode selon la revendication 2, comprenant en outre la dilution, par de l'eau, de l'oxyde de charbon avant la centrifugation de l'oxyde de charbon.

6. Méthode selon la revendication 2, dans laquelle la réduction de l'oxyde de graphène afin de former de l'oxyde de graphène réduit comprend :
le traitement aux ultrasons de l'oxyde de graphène ; et
le traitement hydrothermique de l'oxyde de graphène dans un réacteur de Par après le traitement aux ultrasons de l'oxyde de graphène.

7. Méthode selon la revendication 1, dans laquelle le traitement thermique de charbon à une température d'au moins environ 148,89°C [300°F] comprend :
le chauffage du charbon à une première température ne dépassant pas 176,67°C [350°F] ;
le transfert du charbon dans un réacteur d'élimination du mercure ;
le chauffage du charbon dans le réacteur d'élimination du mercure à une deuxième température d'au moins 260,00°C [500°F] ; et
la mise en contact du charbon avec un gaz inerte afin d'éliminer au moins une portion du mercure présent dans le charbon.

8. Méthode selon la revendication 1, dans laquelle la formation d'oxyde de graphène réduit à partir de l'oxyde de charbon comprend la formation de l'oxyde de graphène réduit à partir de l'oxyde de charbon selon un taux de rendement en oxyde de graphène réduit compris entre environ 10% en poids et environ 20% en poids du charbon.

9. Méthode selon la revendication 1, dans laquelle la concentration prédéterminée des un ou plusieurs atomes d'impureté est comprise entre 0,1% atomique et 15% atomique.

10. Méthode selon la revendication 1, dans laquelle la concentration prédéterminée des un ou plusieurs atomes d'impureté est comprise entre 0,1% atomique et 10% atomique.

11. Méthode selon la revendication 1, dans laquelle les un ou plusieurs atomes d'impureté sont présents naturellement dans la quantité de charbon.

12. Méthode selon la revendication 1, comprenant en outre la collecte d'un ou plusieurs atomes d'impureté dans la vapeur éliminée lors du traitement thermique du charbon à une température d'au moins environ 148,89°C [300°F] ; et
l'incorporation d'une quantité prédéterminée des un ou plusieurs atomes d'impureté collectés dans l'oxyde de graphène réduit.

13. Méthode de production d'une électrode d'une batterie, comprenant la formation de l'oxyde de graphène réduit selon la revendication 1 dans l'électrode de la batterie.
